# EUROPÄISCHE PATENTSCHRIFT

(11) **EP 2 504 359 B1**
(45) Veröffentlichungstag und Bekanntmachung des Hinweises auf die Patenterteilung: **23.03.2016**
(21) Anmeldenummer: 10798744.8
(22) Anmeldetag: 24.11.2010
(51) Int. Cl.: C07K 16/00, C07K 16/18, C07K 16/46

(54) **MONOSPEZIFISCHE POLYPEPTIDREAGENZIEN**
MONOSPECIFIC POLYPEPTIDE REAGENTS
RÉACTIFS POLYPEPTIDIQUES MONOSPÉCIFIQUES

(30) Priorität: 27.11.2009 DE 102009047243
(43) Veröffentlichungstag der Anmeldung: 03.10.2012
(73) Patentinhaber: Orgentec Diagnostika GmbH, 55129 Mainz (DE)
(72) Erfinder: POPPE, Robert, 55128 Mainz (DE)
(74) Vertreter: Weickmann & Weickmann
(86) Internationale Anmeldenummer: PCT/EP2010/068134
(87) Internationale Veröffentlichungsnummer: WO 2011/064257

(56) Entgegenhaltungen:
- EP-A1- 1 757 622
- WO-A1-2007/062466
- WO-A1-2008/131252
- WO-A1-2009/135627
- WO-A2-01/51644
- WO-A2-2007/109321
- HUST MICHAEL ET AL: "Single chain Fab (scFab) fragment", BMC BIOTECHNOLOGY, BIOMED CENTRAL LTD. LONDON, GB, Bd. 7, Nr. 1, 8. März 2007 (2007-03-08), Seite 14, XP021023594, ISSN: 1472-6750, DOI: DOI:10.1186/1472-6750-7-14
- LE GALL F ET AL: "Immunosuppressive properties of anti-CD3 single-chain Fv and diabody", JOURNAL OF IMMUNOLOGICAL METHODS, ELSEVIER SCIENCE PUBLISHERS B.V.,AMSTERDAM, NL, Bd. 285, Nr. 1, 1. Februar 2004 (2004-02-01), Seiten 111-127, XP004489671, ISSN: 0022-1759, DOI: DOI:10.1016/J.JIM.2003.11.007
- MULLER K M ET AL: "The first constant domain (CH1 and CL) of an antibody used as heterodimerization domain for bispecific miniantibodies", FEBS LETTERS, ELSEVIER, AMSTERDAM, NL, Bd. 422, Nr. 2, 30. Januar 1998 (1998-01-30), Seiten 259-264, XP004261818, ISSN: 0014-5793, DOI: DOI:10.1016/S0014-5793(98)00021-0
- SHAO CHANGLI ET AL: "The expression and characterization of a bifunctional protein in E. coli for autologous erythrocyte agglutination test.", CELLULAR & MOLECULAR IMMUNOLOGY AUG 2008 LNKD- PUBMED:18761818, Bd. 5, Nr. 4, August 2008 (2008-08), Seiten 299-306, XP002627304, ISSN: 1672-7681
- LONG M C ET AL: "Construction and characterization of monoclonal antibodies against western equine encephalitis virus.", HYBRIDOMA APR 2000 LNKD- PUBMED:10868791, Bd. 19, Nr. 2, April 2000 (2000-04), Seiten 121-127, XP002627305, ISSN: 0272-457X
- TINCANI A ET AL: "Minimal requirements for antiphospholipid antibodies ELISAs proposed by the European Forum on antiphospholipid antibodies", THROMBOSIS RESEARCH, TARRYTOWN, NY, US, Bd. 114, Nr. 5-6, 1. Januar 2004 (2004-01-01), Seiten 553-558, XP004613201, ISSN: 0049-3848, DOI: DOI:10.1016/J.THROMRES.2004.06.035
- DÜBEL ET AL.: "Rekombinante Antikörper - Werkzeuge gegen Krebs, Infektionen und Autoimmunerkrankungen ?", BIOLOGIE IN UNSERER ZEIT, Bd. 34, Nr. 6, Dezember 2004 (2004-12), Seiten 372-379, XP002640338, Weinheim in der Anmeldung erwähnt
- NIEBA L ET AL: "Disrupting the hydrophobic patches at the antibody variable/constant domain interface: Improved in vivo folding and physical characterization of an engineered scFv fragment", PROTEIN ENGINEERING, OXFORD UNIVERSITY PRESS, SURREY, GB, Bd. 10, Nr. 4, 1. Januar 1997 (1997-01-01) , Seiten 435-444, XP002249462, ISSN: 0269-2139, DOI: DOI:10.1093/PROTEIN/10.4.435
- OLAFSEN TOVE ET AL: "Characterization of engineered anti-p185HER-2 (scFv-CH3)2 antibody fragments (minibodies) for tumor targeting", PROTEIN ENGINEERING, DESIGN AND SELECTION, OXFORD JOURNAL, LONDON, GB, Bd. 17, Nr. 4, 1. April 2004 (2004-04-01), Seiten 315-323, XP002484237, ISSN: 1741-0126, DOI: DOI:10.1093/PROTEIN/GZH040
- ICHIKAWA K ET AL: "A chimeric antibody with the human gamma1 constant region as a putative standard for assays to detect IgG beta2-glycoprotein I-dependent anticardiolipin and anti-beta2-glycoprotein I antibodies.", ARTHRITIS AND RHEUMATISM NOV 1999 LNKD- PUBMED:10555042, Bd. 42, Nr. 11, November 1999 (1999-11), Seiten 2461-2470, XP002640339, ISSN: 0004-3591
- SAZINSKY STEPHEN L ET AL: "Aglycosylated immunoglobulin G1 variants productively engage activating Fc receptors", PROCEEDINGS OF THE NATIONAL ACADEMY OF SCIENCES, NATIONAL ACADEMY OF SCIENCES, Bd. 105, Nr. 51, 23. Dezember 2008 (2008-12-23), Seiten 20167-20172, XP009143223, ISSN: 0027-8424, DOI: DOI:10.1073/PNAS.0809257105
- WU A M ET AL: "Multimerization of a chimeric anti-CD20 single-chain Fv-Fc fusion protein is mediated through variable domain exchange", PROTEIN ENGINEERING, OXFORD UNIVERSITY PRESS, SURREY, GB, Bd. 14, Nr. 12, 1. Januar 2001 (2001-01-01), Seiten 1025-1033, XP002982160, ISSN: 0269-2139, DOI: DOI:10.1093/PROTEIN/14.12.1025
- LI ERQIU ET AL: "Mammalian cell expression of dimeric small immune proteins (SIP)", PROTEIN ENGINEERING, OXFORD UNIVERSITY PRESS, SURREY, GB, Bd. 10, Nr. 6, 1. Januar 1997 (1997-01-01) , Seiten 731-736, XP002252049, ISSN: 0269-2139, DOI: DOI:10.1093/PROTEIN/10.6.731
- A. Schouten: "Plantibodies : requirements for expression and subcellular targeting", , 1998, XP002640340, Gefunden im Internet: URL:http://library.wur.nl/WebQuery/wda/lan g?dissertatie/nummer=2513 [gefunden am 2011-06-07]

## Beschreibung

Die vorliegende Erfindung bezieht sich auf ein neues antigen-bindendes Proteinkonstrukt oder "Modubody", welches mindestens drei funktionale Einzeldomänenmodule eines Antikörpers enthält. Die Modubodies enthalten eine Domäne aus der variablen Schwerkettenregion eines Antikörpers (VH), eine Domäne aus der variablen Leichtkettenregion eines Antikörpers (VL) und binden monospezifisch an ein Antigen. Ferner enthalten die Modubodies eine Domäne aus der konstanten Region von Antikörpern. Die Modubodies können für diagnostische oder therapeutische Zwecke eingesetzt werden.

### Hintergrund der Erfindung

### Antikörperstruktur

Antikörper sind Plasmaglycoproteine, die aus mehreren Polypeptidketten bestehen, welche durch Disulfidbrücken verbunden sind. Ein Standardantikörper besteht aus zwei identischen schweren Immunglobulin (Ig)-Ketten und zwei identischen leichten Ketten. Beide Antikörperketten bestehen aus verschiedenen ca. 110 Aminosäurereste langen Proteindomänen, die in Form einer charakteristischen Immunglobulinfaltung aus β-Faltblättern aufgebaut sind. Die schwere Kette besteht aus einer variablen (VH) Domäne und drei oder vier konstanten Domänen (CH1, CH2, CH3, CH4). Die leichte Kette besteht aus einer variablen (VL) und einer konstanten (CL) Domäne. Die variablen Anteile der schweren und leichten Kette, insbesondere die hypervariablen komplementaritätsbestimmenden Regionen (CDR) tragen zur Antigenspezifität bei. Das Immunsystem stellt eine hohe Diversität von Antikörpern gegen unterschiedlichste Antigene bereit. Antikörper können verschiedenen Klassen, z.B. IgM, IgA, IgG, IgE, IgD, zugeordnet werden.

### Antikörper-Fragmente und Substrukturen

Antikörperfragmente können durch enzymatische Spaltung oder durch rekombinante Verfahren erhalten werden. Ein mit der Antigenerkennungsfunktion ausgestattetes Fab-Fragment enthält, über Disulfidbrücken miteinander verbunden, die VH-CH1-Domänen der schweren Kette und die VL-CL-Domänen der leichten Kette, während das Fv-Fragment nur die variablen Regionen von schwerer und leichter Kette umfasst. Diese aus mehreren Proteinuntereinheiten bestehenden Fragmente können jedoch nur aufwendig in biologisch aktiver Form mit akzeptabler Ausbeute hergestellt werden (Read et al. (2007), Appl. Environ. Microbiol. 73: 5088-96).

Ein einzelkettiges Fv-Fragment (scFv) stellt eine kleine ca. 28 kDa schwere antigenbindende Substruktur in Form einer kovalenten Kopplung von VH-und VL-Domänen über einen Peptidlinker dar (Hu et al. (1996), Cancer Res 56: 3055-61). Die Faltungseffizienz, die mitunter mangelnde Stabilität und die Toxizität dieser Strukturen limitieren jedoch häufig die Ausbeute bei der Herstellung von biologisch aktiven scFv in bakteriellen Expressionssystemen (Nieba et al. (1997), Protein Eng 10: 435-44).

Als Minibody (Mini-Antikörper) wird ein ca. 75 kDa schweres chimäres Molekül aus einem scFv und einer Hinge-Region aus der schweren Kette fusioniert mit der CH3-Domäne bezeichnet, welches sich unterstützt von der CH3-Domäne zu einem über Disulfidbrücken der Hinge-Region kovalent verbundenen bivalenten Molekül zusammenlagert (Wu, EP0627932B1). Die CH3-Domäne dient dabei als Dimerisierungsdomäne zur Herstellung von Homodimeren (vgl. Dübel et al., Biol. Unserer Zeit, 34. Jg., Nr. 6, S. 372-379, 2004). Diese Moleküle zeigen bei der Expression in *E. coli* jedoch geringe Expressionsraten und proteolytischen Abbau in der Hinge-Region. (Hu. et al. (1996), Cancer Research 56: 3055-61).

Wie der Minibody besitzt der Diabody zwei Antigenbindungsstellen. Im Diabody sind in Form einer einzelnen Polypeptidkette VL- und VH-Domänen zu einem divalenten und bispezifischen Molekül verbunden (Hollinger et al. (1993), PNAS 90: 6444-48).

Monobodies stellen chimäre antigenbindende Polypeptide dar, welche innerhalb eines Fibronektin Typ III-Gerüstes hypervariabele CDR-Schleifen präsentieren (Koide, EP0985039B1). Diese Moleküle stellen eine wertvolle Klasse an neuartigen Affinitätsreagenzien zur Verfügung. Durch die Transplantation von CDR-Schleifen in ein heterologes Fibronektingerüst gehen jedoch Effektor- und Detektionsfunktionen nativer Antikörper verloren.

Nanobodies bestehen aus einzelkettigen antigenbindenden V_{H}H-Domänen (variable Domäne eines Schwerketten-Antikörpers) und beruhen auf der Beobachtung, dass bei Kamelen und Lamas natürliche und funktionale Antikörper, welche nur aus schweren Ketten bestehen, vorkommen (Caserman und Harmers, EP19930919098). Die Löslichkeit von humanen VH-Domänen (variablen Schwerketten-Domänen) ist jedoch aufgrund hydrophober Regionen, welche im intakten Antiköper mit Regionen der leichten Kette interagieren, häufig eingeschränkt (Barthelemy et al. (2007) J. Biol. Chem. 283: 3639-54).

EP 1 575 622 A1 betrifft bivalente Antikörperkonstrukte, die ein heterotetrameres oder homodimeres Format aufweisen, das von Disulfidbrücken stabilisiert wird.

Shao & Zhang (Cellular and Molecular Immunology, Band 5, Nr. 4, Seiten 299-306, 2008) beschreiben die Expression und Charakterisierung eines bifunktionalen Proteins in *E. coli* für einen autologen Erythrozyten-Agglutinierungstest. Bei dem bifunktionalen Protein handelt es sich um ein rekombinantes Fusionsprotein aus einem ScFv (V_{H}-Linker-V_{L}), das für das anti-H-Antigen des monoklonalen Antikörpers 2E8 kodiert, und einem HIV-1 gp41-Antigenpeptid.

Li et al. (Protein Engineering, Band 10, Nr. 6, Seiten 731-736, 1997) beschreiben die Expression dimerer kleiner Immunproteine (small immune proteins; SIP) in Säugerzellen. Bei SIP handelt es sich um bivalente Moleküle, die gegebenenfalls durch Disulfidbrücken stabilisiert sind.

### Antikörper in der Immundiagnose

Es existieren zahlreiche Immunoassayformate, um in einer biologischen Probe die Anwesenheit bzw. Konzentration eines spezifischen Antikörpers, z.B. gegen ein Pathogen, ein Autoantigen oder ein Allergen, zu bestimmen. In der Regel sind diese Assays auf den Nachweis einer spezifischen Antikörperklasse oder einer Kombination bestimmter Antikörperklassen ausgerichtet und verwenden spezifische interne Kontrollen oder Kalibratoren. Immunoassays, die beispielsweise zur Bestimmung von humanen Autoantikörpern geeignet sind, beinhalten in der Regel eine Positivkontrolle, eine Negativkontrolle sowie einen Indexkalibrator oder eine Abstufung unterschiedlicher Kalibratorkonzentrationen (Standardreihe) zur Erstellung einer Eichkurve, mit welcher Antikörperkonzentrationen in einer Probe interpoliert werden können. Üblicherweise werden diese Kontroll- und Kalibrierreagenzien durch Verdünnung seropositiven Plasmas oder Serums in einem geeigneten Verdünnungsmedium hergestellt.

Beispielsweise werden die Kalibratoren und Kontrollen für die isotypspezifische Bestimmung von β2-Glycoprotein-Autoantikörpern aus dem Serum von humanen Spendern präpariert, die hohe Konzentrationen dieser Autoantikörper der Klassen IgG, IgM und/oder IgA enthalten. Die Verwendung von humanem seropositivem Serum oder Plasma zur Herstellung von Kontrollen und Kalibratoren ist jedoch mit zahlreichen Nachteilen verbunden, wie z.B. der Schwierigkeit, solche Reagenzien in großen Mengen und geeigneter Qualität zu beschaffen, Unterschiede in den Bindungscharakteristika bei verschiedenen Chargen, heterogene polyklonale Spezifität, heterogene Isotypenzusammensetzung, Anwesenheit von Pathogenen, Kosten etc.

Da Antikörper mit hoher Spezifität und Affinität an Antigene binden, sind sie in der Immundiagnose von zentraler Bedeutung. Die Größe der natürlichen Antikörpermoleküle und ihr komplexer Aufbau aus mehreren Polypeptidketten mit einer hohen Zahl an Inter- und Intra-Domänen, Vernetzungen durch Disulfidbrücken sowie Glykosilierungspositionen stellen eine erhebliche Hürde bei der Konstruktion und der rekombinanten Expression von spezifischen Antikörpern dar.

Hackett et al. (EP1018019 B1) offenbaren ein Verfahren zur Herstellung von Reagenzien zum Gebrauch als Kalibratoren und Kontrollen, wobei das Reagenz ein chimärer monoklonaler Antikörper ist, der variable Regionen der schweren und leichten Kette aus einer ersten Wirtsspezies umfasst, fusioniert an die konstanten Regionen der schweren und leichten Kette aus einer zweiten Wirtsspezies, die derjenigen des zu bestimmenden Antikörpers entspricht. Die Herstellung dieser spezies-chimären monoklonalen Antikörper erfordert jedoch einen hohen technischen Aufwand, wie er der Produktion monoklonaler Antikörper eigen ist. Hackett benennt zwar die theoretische Möglichkeit, als künstliche Kalibratoren auch Polypeptide zu verwenden, die spezifisch an einen vorbestimmten Liganden binden und an eine Region von Antikörpern der gewünschten Wirtsspezies fusioniert sind. Jedoch wird von Hackett kein Verfahren bereitgestellt, durch das einzelkettige synthetische Polypeptidkalibratoren aufgebaut werden können.

Es besteht somit ein anhaltender Bedarf für kleine, Antikörper-ähnliche Moleküle, welche ein Antigen spezifisch erkennen, mit Antikörpern gegen natürliche Immunglobuline Komplexe bilden und leicht und und in großer Menge in bakteriellen Expressionssystemen hergestellt werden können.

### Zusammenfassung der Erfindung

Ein Gegenstand der Erfindung ist ein Fusionspolypeptid umfassend
(i) eine erste Domäne, umfassend die variable Schwerkettenregion eines Antikörpers (VH) oder zumindest einen die Antigenbindung vermittelnden Abschnitt davon,
(ii) eine zweite Domäne, umfassend die variable Leichtkettenregion eines Antikörpers (VL) oder zumindest einen die Antigenbindung vermittelnden Abschnitt davon und
(iii) eine dritte Domäne, umfassend einen Abschnitt einer konstanten Schwerkettenregion eines Antikörpers (CHX),
wobei die Domäne (iii) eine Länge von 80-130 Aminosäureresten aufweist und Aminosäurepositionen,
deren Seitenketten entsprechend dem Konzept der "knobs-into-holes"-Anordnung Interaktionen aufweisen,
modifiziert sind,
wobei die Domänen (i), (ii) und (iii) über Peptidlinker (L) miteinander verknüpft sind,
wobei die Peptidlinker (L) jeweils unabhängig eine Länge von 25-45

Aminosäureresten aufweisen und vollständig aus Glycin- und/oder Serinresten bestehen, und wobei das Fusionspolypeptid keine intermolekularen Disulfidbrücken ausbildet und frei von Hinge-Regionen aus Antikörpern ist.

Ein weiterer Gegenstand der Erfindung ist eine Nukleinsäure, die für ein Fusionspolypeptid wie oben angegeben kodiert.

Noch ein weiterer Gegenstand der Erfindung ist eine Wirtszelle, die eine erfindungsgemäße Nukleinsäure enthält.

Noch ein weiterer Gegenstand der Erfindung ist ein Verfahren zur Herstellung eines Fusionspolypeptid durch Kultivierung einer erfindungsgemäßen Wirtszelle und Gewinnung des Fusionspolypeptids aus der Zelle oder dem Kulturüberstand.

Noch weitere Aspekte der vorliegenden Erfindung betreffen die Verwendung von Fusionspolypeptiden als Reagenzien in diagnostischen oder biochemischen Tests sowie medizinische Anwendungen der Fusionspolypeptide, Nukleinsäuren und Wirtszellen.

Die oben beschriebenen erfindungsgemäßen Fusionspolypeptide werden als "Modubodies" bezeichnet.

Modubodies sind Konstrukte, welche aus Domänen der variablen schweren (VH) und variablen leichten (VL) Ketten eines Antikörpers und einer oder mehrerer Domänen der konstanten Region der schweren Kette (CH1, CH2, CH3, CH4) von Antikörpern bestehen, wobei diese Domänen eines Antikörpers in Form einer linearen Abfolge von einander unabhängig strukturierter Funktionsmodule über geeignete Linkersequenzen miteinander gekoppelt sind. Die einzelnen Domänen haben eine Länge von 80-130 Aminosäuren. Modubodies bestehen aus einer einzigen Polypeptidkette und besitzen eine einzige Antigenbindungsstelle, die aus den Domänen VH und VL gebildet wird. Modubodies können keine intermolekularen Disulfidbrücken ausbilden, so dass sie als Monomere vorliegen. Modubodies sind frei von Hinge-Regionen aus Antikörpern. Sie sind somit Miniaturversionen von monovalenten Antikörpern, die in Form einer einzelnen Proteinkette leicht und in großen Mengen in geeigneten Expressionssystemen, z.B. bakteriellen Expressionssystemen, hergestellt werden können. Modubodies sind aufgrund ihrer einzelkettigen Struktur, ihrer geringen Größe, der monoklonalen Zusammensetzung, ihrer leichten Herstellbarkeit und ihrer Stabilität ideale Reagenzien für die biochemische Forschung, für die Verwendung in diagnostischen Assays und als Therapeutika.

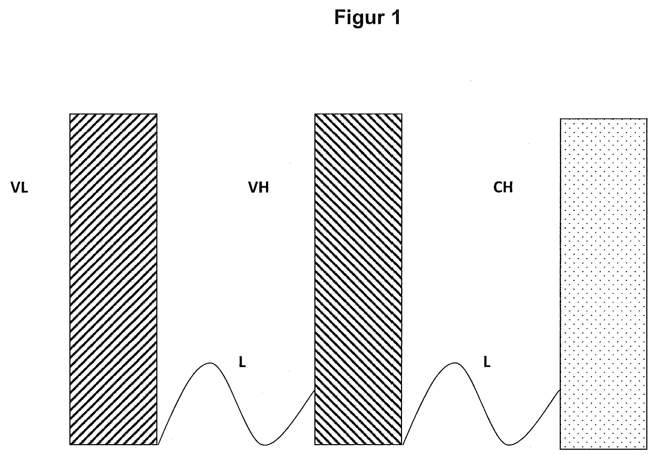

Ein Modubody ist somit ein chimäres Molekül mit einem typischen Aufbau wie in **Figur 1****,** welches aus separaten Funktionsmodulen besteht, die sich aus Domänen der variablen schweren (VH) und variablen leichten (VL) Ketten eines Antikörpers und einer oder mehrerer Domänen der konstanten Region der schweren Kette (CHX, z.B. CH1, CH2, CH3 oder CH4) von Antikörpern ableiten. Die einzelnen voneinander unabhängig strukturierten Funktionsmodule sind in einer linearen Abfolge über geeignete Linkersequenzen (L) miteinander gekoppelt.

### Beschreibung der Abbildungen

**Figur 1****:**
   Schematischer Aufbau einer Ausführungsform eines erfindungsgemäßen Fusionspolypeptids (Modubody), wobei **VL** eine Domäne der variablen leichten Kette, **VH** eine Domäne der variablen schweren Kette und **CHX** eine Domäne der konstanten Region der schweren Kette (CH1, CH2, CH3 oder CH4) von Antikörpern darstellt. Die einzelnen Domänen sind durch heterologe, flexible Peptidlinker **L** untereinander in linearer Abfolge verbunden.
**Figur 2****:**
   Charakterisierung der Bindungsreaktion eines scFv-CAD Reaktionsmoduls an das Ziel β2-Glycoprotein.
   Zur ELISA-Detektion der spezifischen β2-Glycoprotein-Bindeaktivität des scFv-CAD-Reaktionsmoduls wurden Verdünnungsserien von scFv-CAD in der angezeigten Konzentration auf Mikrotiterplatten, die mit β2-Glycoprotein und zur Kontrolle mit Rinderserumalbumin (BSA) beschichtet waren, aufgetragen. Die Bindung wurde mit einem Peroxidase-markierten RGS-6X-His-Antikörper (Qiagen, Hilden) und einer Tetramethylbenzidin (TMB)-Farbreaktion durch Messung der O.D. 450 bestimmt.
**Figur 3****:**
   Charakterisierung der Stabilität eines scFv-CAD-Reaktionsmoduls gegen Temperaturstress.
   A. Um den Einfluss von 50 °C Temperaturstress auf die β2-Glycoprotein-Bindeaktivtät des CAD-scFv zu untersuchen, wurde das gereinigte Proteinmodul in einer Konzentration von 5 µg/ml über einen Zeitraum von 0-180 Minuten einer Temperatur von 50 °C ausgesetzt. Die Bindung des scFv-CAD an β2-Glycoprotein wurde im ELISA mit einem Peroxidase-markierten RGS-6X-His-Antikörper und einer TMB-Farbreaktion durch Messung der O.D. 450 bestimmt.
   B. Um die Inaktivierungstemperatur des scFv-CAD zu bestimmen, wurde das gereinigte Proteinmodul in einer Konzentration von 5 µg/ml in Kalibratorverdünnungsmedium für 10 min einer Serie von Temperaturen von 20 °C bis 90 °C ausgesetzt. Die Bindung des scFv-CAD an β2-Glycoprotein wurde im ELISA mit einem Peroxidase-markierten RGS-6X-His-Antikörper und einer TMB-Farbreaktion durch Messung der O.D. 450 bestimmt.
**Figur 4****:**
   Charakterisierung der Kalibratorfunktion eines CAD-IgG-CH3 Modubodies durch Bindung an das Ziel β2-Glycoprotein und Detektion mit einem Anti-human-IgG-Peroxidase-Sekundärantikörper. CAD-IgG-CH3 wurde in der angezeigten Konzentration auf Mikrotiterplatten, die mit β2-Glycoprotein beschichtet waren, aufgetragen und die Bindung mit einem Peroxidase-markierten Anti-human-IgG-Antikörper (Jackson Immunoresearch) und einer TMB-Farbreaktion durch Messung der O.D. 450 bestimmt.
**Figur 5****:**
   Charakterisierung der Stabilität eines CAD-IgG-CH3 Modubodies gegen Temperaturstress. Die Stabilität gegenüber Hitzestess wurde untersucht, indem Verdünnungen des CAD-IgG-CH3 Modubodies in den angezeigten Konzentrationen für 60 Minuten und 90 Minuten bei 50 °C inkubiert wurden und im Vergleich zu einer bei Raumtemperatur gelagerten Verdünnungsserie im Anti-β2-Glycoproteinimmunoassay untersucht wurden. Hierbei wurde die Bindung des CAD-IgG-CH3 Modubodies an β2-Glycoprotein mit einem Peroxidase-markierten Anti-human-IgG-Antikörper und einer TMB-Farbreaktion durch Messung der O.D. 450 bestimmt.
**Figur 6****:**
   Charakterisierung der Stabilität eines CAD-IgG-CH3 Modubodies gegen Lagerung bei 36 °C. Die Stabilität gegenüber erhöhter Lagertemperatur wurde untersucht, indem Verdünnungen des CAD-IgG-CH3 Modubodies in den angezeigten Konzentrationen für 1, 2, 4, 7, 10 Tage bei 36 °C inkubiert wurden und im Vergleich zu einer bei 4 °C gelagerten Verdünnungsserie im Anti-β2-Glycoproteinimmunoassay untersucht wurden. Hierbei wurde die Bindung des CAD-IgG-CH3 Modubodies an β2-Glycoprotein mit einem Peroxidase-markierten Anti-human-IgG Antikörper und einer TMB-Farbreaktion durch Messung der O.D. 450 bestimmt.
**Figur 7****:**
   Charakterisierung der Stabilität eines CAD-IgG-CH3 Modubodies gegen Eintrocknung. Die Stabilität gegenüber Eintrocknung wurde untersucht, indem Verdünnungen des CAD-IgG-CH3 Modubodies in einer Speedvak-Vorrichtung unter Vakuum bei 22 °C eingetrocknet und anschließend zu den angezeigten Konzentrationen resolubilisiert wurden. Anschließend wurde die Bindung des resolubilisierten CAD-IgG-CH3 Modubodies an β2-Glycoprotein mit einem Peroxidase-markierten Anti-human-IgG-Antikörper und einer TMB-Farbreaktion durch Messung der O.D. 450 bestimmt.
**Figur 8****:**
   Charakterisierung der Stabilität eines CAD-IgG-CH3 Modubodies gegen wiederholte Gefrier-Auftauzyklen. Die Stabilität gegenüber wiederholten Gefrier-Auftauzyklen wurde untersucht, indem Verdünnungen des CAD-IgG-CH3 Modubodies in fünf wiederholten Gefrier-Auftauzyklen bei -70 °C eingefroren und bei 37 °C wieder aufgetaut wurden und anschließend mit den angezeigten Konzentrationen im Anti-β2-Glycoproteinimmunoassay untersucht wurden. Hierbei wurde die Bindung des CAD-IgG-CH3 Modubodies an β2-Glycoprotein mit einem Peroxidase-markierten Anti-human-IgG-Antikörper und einer TMB-Farbreaktion durch Messung der O.D. 450 bestimmt.
**Figur 9****:**
   Charakterisierung der Kalibratorfunktion des CAD-IgG-CH2-Modubodies durch Bindung an das Ziel ß2-Glycoprotein und Detektion mit einem Anti-human-IgG-Peroxidase Sekundärantikörper. CAD-IgG-CH2 wurde in der angezeigten Konzentration auf Mikrotiterplatten, die mit ß2-Glycoprotein im Komplex mit Cardiolipin beschichtet waren, aufgetragen und die Bindung mit einem Peroxidase-markierten Anti-human-IgG-Antikörper (Jackson Immunoresearch) und einer TMB-Farbreaktion durch Messung der O.D. 450 nm bestimmt.
**Figur 10****:**
   Charakterisierung der Stabilität des CAD-IgG-CH2-Modubodies gegen Eintrocknung. Die Stabilität gegenüber Eintrocknung wurde untersucht, indem Verdünnungen des CAD-IgG-CH2-Modubodies in einer Speedvak-Vorrichtung unter Vakuum bei 22 °C eingetrocknet und anschließend zu den angezeigten Konzentrationen resolubilisiert wurden. Anschließend wurde die Bindung des resolubilisierten CAD-IgG-CH2-Modubodies an ß2-Glycoprotein mit einem Peroxidase-markierten Anti-human-IgG-Antikörper und einer TMB-Farbreaktion durch Messung der O.D. 450 nm bestimmt.
**Figur 11****:**
   Charakterisierung der Stabilität des CAD-IgG-CH2-Modubodies gegen wiederholte Gefrier-Auftauzyklen. Die Stabilität gegenüber wiederholten Gefrier-Auftauzyklen wurde untersucht, indem Verdünnungen des CAD-IgG-CH2-Modubodies in fünf wiederholten Gefrier-Auftauzyklen bei -70 °C eingefroren und bei 37 °C wieder aufgetaut wurden und anschließend mit den angezeigten Konzentrationen auf Mikrotiterplatten, die mit β2-Glycoprotein im Komplex mit Cardiolipin beschichtet waren, untersucht wurden. Hierbei wurde die Bindung des CAD-IgG-CH2-Modubodies an β2-Glycoprotein mit einem Peroxidase-markierten Anti-human-IgG-Antikörper und einer TMB-Farbreaktion durch Messung der O.D. 450 nm bestimmt.
**Figur 12****:**
   Charakterisierung der Kalibratorfunktion des multifunktionalen CAD-IgM-IgA-IgG-Modubodies durch Bindung an das Ziel ß2-Glycoprotein und separate Detektion mit den isotypspezifischen Anti-human-IgM-, Anti-human-IgA- und Anti-human-IgG-Peroxidase Sekundärantikörpern. CAD-IgM-IgA-IgG wurde in der angezeigten Konzentration auf Mikrotiterplatten, die mit β2-Glycoprotein im Komplex mit Cardiolipin beschichtet waren, aufgetragen und die Bindung in separaten Bestimmungen mit isotypspezifischen Peroxidase-markierten Anti-human-IgM-, Anti-human-IgA- und Anti-human-IgG-Antikörpern (Jackson Immunoresearch) und einer TMB-Farbreaktion durch Messung der O.D. 450 nm bestimmt.
**Figur 13****:**
   Charakterisierung der Stabilität des CAD-IgM-IgA-IgG-Modubodies gegen Eintrocknung. Die Stabilität gegenüber Eintrocknung wurde untersucht, indem Verdünnungen des CAD-IgM-IgA-IgG-Modubodies in einer Speedvak-Vorrichtung unter Vakuum bei 22 °C eingetrocknet und anschließend zu den angezeigten Konzentrationen resolubilisiert wurden. Anschließend wurde die Bindung des resolubilisierten CAD-IgM-IgA-IgG-Modubodies an β2-Glycoprotein in separaten Bestimmungen mit isotypspezifischen Peroxidase-markierten Anti-human-IgM-, Anti-human-IgA- und Anti-human-IgG-Antikörpern (Jackson Immunoresearch) und einer TMB-Farbreaktion durch Messung der O.D. 450nm bestimmt.
**Figur 14****:**
   Charakterisierung der Stabilität des CAD-IgM-IgA-IgG-Modubodies gegen wiederholte Gefrier-Auftauzyklen. Die Stabilität gegenüber wiederholten Gefrier-Auftauzyklen wurde untersucht, indem Verdünnungen des CAD-IgM-IgA-IgG-Modubodies in fünf wiederholten Gefrier-Auftauzyklen bei -70 °C eingefroren und bei 37 °C wieder aufgetaut wurden und anschließend mit den angezeigten Konzentrationen auf Mikrotiterplatten, die mit β2-Glycoprotein im Komplex mit Cardiolipin beschichtet waren, untersucht wurden. Anschließend wurde die Bindung des CAD-IgM-IgA-IgG-Modubodies an ß2-Glycoprotein in separaten Bestimmungen mit isotypspezifischen Peroxidase-markierten Anti-human-IgM-, Anti-human-IgA-und Anti-human-IgG-Antikörpern (Jackson Immunoresearch) und einer TMB-Farbreaktion durch Messung der O.D. 450nm bestimmt.
**Figur 15****:**
   Charakterisierung der Bindungsreaktion des CAD-IgG-CH3-Knob02-Modubodies an das Ziel ß2-Glycoprotein. Zur ELISA Detektion der spezifischen ß2-Glycoprotein-Bindeaktivität des CAD-IgG-CH3-Knob02-Modubodies wurden Verdünnungsserien von CAD-IgG-CH3-Knob02 in der angezeigten Konzentration auf Mikrotiterplatten, die mit ß2-Glycoprotein und zur Kontrolle mit Rinderserumalbumin (BSA) beschichtet waren, aufgetragen. Die Bindung wurde mit einem Peroxidase-markierten RGS-6X-His-Antikörper (Qiagen, Hilden) und einer Tetramethylbenzidin (TMB)-Farbreaktion durch Messung der O.D. 450nm bestimmt.

### Ausführliche Beschreibung der Erfindung

Bisherige Verfahren zur Herstellung von Antikörpern, Antikörperfragmenten und kleinen antikörperähnlichen Molekülen weisen zahlreiche Einschränkungen bezüglich der Möglichkeit, diese in biologisch aktiver Form kostengünstig herzustellen, auf.

Bisherige Verfahren zur Herstellung von Kalibratoren und Standardmaterialien für diagnostische Assays weisen zahlreiche Nachteile bezüglich der Verfügbarkeit, der einheitlichen Bindungs- und Stabilitätscharakteristika, der Sicherstellung der Abwesenheit von Pathogenen und der Herstellungskosten auf. Diese Einschränkungen und Nachteile werden durch die vorliegende Erfindung behoben. Zudem besteht ein anhaltender Bedarf für kleine Antikörper-ähnliche Moleküle, welche ein Antigen spezifisch erkennen und mit Antikörpern gegen natürliche Immunglobuline Komplexe bilden.

Die vorliegenden Offenbarung ermöglicht die Herstellung monospezifischer Polypeptidreagenzien - "Modubodies" - für die biochemische Forschung, für die Verwendung in diagnostischen Assays oder als Bestandteil von Therapeutika.

Ein Modubody ist ein chimäres einzelkettiges Fusionspolypeptid, welches aus mindestens drei Domänen, nämlich einer ersten Domäne aus der variablen Schwerkettenregion eines Antikörpers (VH), einer zweiten Domäne aus der variablen Leichtkettenregion eines Antikörpers (VL) und einer dritten Domäne umfassend einen Teilabschnitt einer konstanten Schwerkettenregion eines Antikörpers (CHX) besteht. Die einzelnen Domänen sind über geeignete Peptidlinker miteinander verknüpft. Der Modubody ist monovalent, d.h. er weist eine einzige Antigenbindungsstelle auf.

Beispielsweise besitzen die offenbarten Modubodies eine Struktur (beginnend am N-Terminus) wie folgt:
VH-L-VL-L-CHX oder
VL-L-VH-L-CHX,
wobei VH die variable Schwerkettenregion eines Antikörpers oder zumindest einen die Antigenbindung vermittelnden Abschnitt davon, VL die variable Leichtkettenregion eines Antikörpers oder zumindest einen die Antigenbindung vermittelnden Abschnitt davon, L einen Peptidlinker und CHX eine Domäne umfassend einen Teilabschnitt einer konstanten Schwerkettenregion eines Antikörpers bedeutet. CHX kann beispielsweise aus den Abschnitten CH1, CH2, CH3 und CH4 aus der konstanten Schwerkettenregion von Antikörpern, bevorzugt von Antikörpern der Klassen IgG, IgM, IgE und IgA, besonders bevorzugt von humanen Antikörpern der Klassen IgG, IgM, IgE und IgA, ausgewählt werden. Bevorzugte Beispiele der Domäne CHX sind IgG-CH1, IgG-CH2, IgG-CH3, IgA-CH2, IgA-CH3, IgM-CH2, IgM-CH3 und IgM-CH4, insbesondere aus den jeweiligen humanen Antikörpern.

Die erste Domäne (VH) und die zweite Domäne (VL) bilden zusammen die Antigenbindungsstelle (Bindemodul). Sie werden vorzugsweise so ausgewählt, dass sie beide aus einem einzigen Ursprungsantikörper stammen. Dieser Ursprungsantikörper ist ein beliebiger monoklonaler Antikörper, z.B. ein monoklonaler Antikörper aus einer nicht-humanen Säugerspezies (z.B. Ratte, Maus oder Kaninchen), ein humaner Antikörper oder ein humanisierter Antikörper.

Der Modubody kann gegen ein beliebiges Antigen gerichtet sein, beispielsweise ein diagnostisch oder therapeutisch relevantes Antigen. Bevorzugte Beispiele von spezifischen Antigenen sind β2-Glycoprotein, Phosphatidylserin, Vascular Endothelial Growth Factor (VEGF-A), Tumor Necrosis Factor (TNF-alpha), Smoothened Homolog (SMO), Protein Patched Homolog (PTC1), B-Lymphocyte Antigen (CD20), Cytotoxic T-Lymphocyte Protein 4 (CTLA4), Amyloid beta A4 Protein (APP), Presenilin-1 (PS1), CC-Chemokine Receptor (CCR-5), Telomer Repeat-binding Factor 1 (TRF1) und Toll-like Receptors (TLR1-10).

Die erste und die zweite Domäne des erfindungsgemäßen Polypeptids können die vollständigen variablen Schwer- oder Leichtkettenregionen eines Antikörpers oder zumindest einen die Antigenbindung vermittelnden Abschnitt davon enthalten. Vorzugsweise enthalten die Domänen zumindest die Regionen CDR1, CDR2 und CDR3 der betreffenden Schwer- oder Leichtkettenregion vollständig und zumindest Teile der entsprechenden Framework-Regionen FR1, FR2, FR3 und FR4. Die Länge der Domänen VH und VL beträgt mindestens 80, mindestens 90 oder mindestens 100 Aminosäurereste und bis zu 110 oder bis zu 120 Aminosäurereste.

Das erfindungsgemäße Fusionspolypeptid enthält mindestens eine Domäne umfassend einen Abschnitt einer konstanten Schwerkettenregion eines Antikörpers (CHX). Vorzugsweise ist die dritte Domäne bzw. sind die dritten Domänen C-terminal zu den VH- und VL-Domänen angeordnet. Eine dritte Domäne kann einen vollständigen Abschnitt aus einer konstanten Schwerkettenregion oder einen Teilabschnitt davon enthalten, welcher dem Fusionspolypeptid eine ausreichende strukturelle Stabilität vermittelt. Die Länge einer dritten Domäne beträgt mindestens 80, mindestens 90 oder mindestens 100 Aminosäurereste und bis zu 110, bis zu 120 oder bis zu 130 Aminosäurereste. Das Fusionspolypeptid kann gegebenenfalls mehrere dritte Domänen enthalten, gleich oder verschieden sein können, z.B. 2, 3 oder 4. Sollten mehrere dritte Domänen vorhanden sein, sind diese jeweils über Peptidlinker miteinander verknüpft und vorzugsweise am C-Terminus des Fusionspolypeptids angeordnet. Beispiele für den strukturellen Aufbau von erfindungsgemäßen Fusionspolypeptiden mit 2 (oder 3) dritten Domänen sind wie folgt:
VH - L - VL - L - CHX1 - L - CHX2 (-L-CHX3) oder
VL - L - VH - L - CHX1 - L - CHX2 (-L-CHX3),
wobei VH, L und VL wie zuvor definiert sind und CHX1, CHX2 und CHX3 jeweils eine dritte Domäne CHX wie zuvor definiert bedeuten.

Das erfindungsgemäße Fusionspolypeptid enthält erste, zweite und dritte Domänen, die jeweils über Peptidlinker (L) miteinander verknüpft sind. Diese Peptidlinker bestehen aus Sequenzen, welche heterolog zu den Aminosäuresequenzen der ersten, zweiten und dritten Domänen sind, bzw. aus Sequenzen, die nicht in natürlichen Immunglobulinen vorliegen. Die Peptidlinker, welche die einzelnen Domänen miteinander verknüpfen, können jeweils gleich oder verschieden sein. Die Peptidlinker weisen jeweils unabhängig eine Länge von 25-45 und besonders bevorzugt von 30-40 Aminosäureresten auf. Ferner sind die Peptidlinker flexible Linker ohne Sekundärstruktur. Die Peptidlinker bestehen vollständig aus Glycin- und/oder Serinresten. Besonders geeignet sind Peptidlinker, welche mehrere Abfolgen der Sequenz SGGGG enthalten. Ein besonders bevorzugtes Beispiel für einen Peptidlinker ist in SEQ ID NO: 3 gezeigt. Gegebenenfalls können Linkersequenzen auch am N- und/oder C-Terminus des Fusionspolypeptids vorhanden sein.

Neben den Domänen (i), (ii) und (iii) sowie den zwischen den Domänen angeordneten Peptidlinkern kann das Fusionspolypeptid gegebenenfalls noch weitere Sequenzabschnitte enthalten, beispielsweise einen Signalpeptidabschnitt, der am N- und/oder C-Terminus angeordnet ist und die Expression und/oder Sekretion des Polypeptids erleichtert. Ferner kann das Fusionspolypeptid eine oder mehrere zusätzliche Nicht-ImmunglobulinDomänen, z.B. Nachweis- oder Erkennungsdomänen, d.h. zum Nachweis bzw. zur Erkennung des Fusionspolypeptids geeignete Peptidsequenzen (Tags), z.B. ein FLAG-Epitop oder eine poly-His-Sequenz, enthalten. Außerdem kann das Fusionspolypeptid gegebenenfalls auch eine oder mehrere Nicht-Immunglobulin-Effektordomänen enthalten. Die zusätzlichen Domänen sind - sofern vorhanden - vorzugsweise über einen Peptidlinker, z.B. einem Peptidlinker wie zuvor definiert, mit dem restlichen Fusionspolypeptid verbunden.

Besonders bevorzugte Beispiele für erfindungsgemäße Fusionspolypeptide enthalten eine oder mehrere Domänen VH, VL und/oder CHX wie zuvor definiert, die eine Identität auf Aminosäureebene von mindestens 90 %, vorzugsweise von mindestens 95 % zu den entsprechenden Domänen gemäß SEQ ID NO: 1 (VL), SEQ ID NO: 2 (VH), SEQ ID NO: 4 (VH, VL), SEQ ID NO: 8 (IgG-CH1), SEQ ID NO: 10 (IgG-CH2), SEQ ID NO: 12 (IgG-CH3), SEQ ID NO: 14 (IgA-CH2), SEQ ID NO: 16 (IgA-CH3), SEQ ID NO: 18 (IgM-CH2), SEQ ID NO: 20 (IgM-CH3) oder SEQ ID NO: 22 (IgM-CH4) aufweisen.

Gegebenenfalls kann das erfindungsgemäße Fusionspolypeptid eine oder mehrere Modifikationen gegenüber der natürlichen Sequenz der darin enthaltenen VH-, VL- und CHX-Domänen aufweisen. So kann beispielsweise mindestens ein Asparaginrest an einer Glykosilierungsposition, z.B. in einer CHX-Domäne, durch einen anderen Aminosäurerest, vorzugsweise Serin, Alanin oder Glycin, ersetzt sein. Des Weiteren kann gegebenenfalls mindestens ein Cysteinrest, z.B. in einer CHX-Domäne, der nicht mit einem in der betreffenden Domäne vorhandenen zweiten Cysteinrest eine Disulfidbrücke ausbildet und daher potenziell die Ausbildung intermolekularer Disulfidbrücken bewirkt, durch einen anderen Aminosäurerest, vorzugsweise Serin, Alanin oder Glycin, ersetzt sein.

Die erfindungsgemäßen Modubodies sind künstliche monospezifische antikörperähnliche Moleküle, die in Form einer einzelnen Proteinkette leicht und in großen Mengen in geeigneten Expressionssystemen, z.B. bakteriellen Expressionssystemen, hergestellt werden können. Als Reagenzien für biochemische oder diagnostische Assays können Modubodies ein Antigen über ein Bindemodul (VH + VL) spezifisch binden und über ein Reaktionsmodul (CHX) mit spezies- und isotypspezifischen Sekundärantikörpern spezifisch nachgewiesen werden. Eine Abfolge mehrerer Reaktionsmodule (z.B. CHX1 und CHX2 oder CHX und eine Nicht-Immunglobulin-Nachweis- oder Erkennungsdomäne) ermöglicht die Detektion mit unterschiedlichen Reagenzien, z.B. mit unterschiedlichen spezies- oder isotypspezifischen Sekundärantikörpern. Als Reagenzien für therapeutische Funktionen können Modubodies über ein Bindemodul an ein Antigen auf einer Zielstruktur spezifisch binden und über ein Reaktionsmodul (CHX) oder eine Serie von Reaktionsmodulen (z.B. CHX1 und CHX2 oder CHX und eine Nicht-Immunglobulin-Effektordomäne) spezifische Effektorfunktionen ausüben.

CHX-Domänen, die potenziell Dimere ausbilden könnten, werden so modifiziert, dass eine Dimerisierung ausgeschlossen werden kann und die Modubodies monovalent vorliegen.

Aminosäurepositionen, deren Seitenketten entsprechend dem Konzept der "knobs-into-holes"-Anordnung (Crick, F.H.C. (1952), Nature, 170: 882-883) Interaktionen aufweisen, werden modifiziert.

So können Aminosäurepositionen, deren Seitenketten Kontakte an der Grenzfläche von IgG₁-CH3-Dimeren (Ridgway J.B.B et al. (1996), Protein Engineering, 9: 617-621) ausbilden, so modifiziert werden, dass an gegenüber liegenden Kontaktstellen im IgG-CH3-Dimer voluminöse Aminosäureseitenketten vorliegen und durch diese "knob-knob"-Position eine Dimerisierung sterisch blockiert wird. Die Interaktionen zwischen den kontaktierenden Aminosäureseitenketten zweier IgG₁-CH3-Domänen können beispielsweise durch Austausch der Aminosäurepositionen Threonin 366, Threonin 394, Phenylalanin 405 gegen Tyrosin in den Positionen 366, 394 und 405 (Nummerierungsschema entsprechend dem Kabat EU Index (Kabat et al., (1991) Sequences of Proteins of Immunological Interest 5th Edition, NIH Publication 91-3242), blockiert werden.

Die erfindungsgemäßen Modubodies zeichnen sich außerdem durch eine überraschende Stabilität, z.B. Stabilität gegenüber Temperaturstress oder Stabilität gegenüber Trocknungs/Rekonstitutions- oder Einfrier/Auftauzyklen aus. So bleiben, im Vergleich zu einer unbehandelten Probe, auch in verdünnten Lösungen (5 µg/ml) der Modubodies über 90 % der Antigenbindeaktivität nach 10-tägiger Inkubation bei 36 °C oder nach Trocknung und Rekonstitution oder nach 5-fach wiederholten Einfrier/Auftauzyklen erhalten.

Die de novo Konstruktion eines Modubodies umfasst vorzugsweise die Schritte:
a) Auswahl von räumlich abgegrenzten Domänen aus Immunglobulinen mit bekannter oder modellierter Raumstruktur unter Berücksichtigung von Sequenz- und Strukturdatenbankinformationen;
b) Disulfidbrückenoptimierung der gewählten Domänen, indem Cysteinpositionen, die im Kontext des intakten Immunglobulins Disulfidbrücken außerhalb der gewählten Domäne ausbilden, zu einer strukturneutralen Aminosäure, vorzugsweise Serin, Alanin oder Glycin, editiert werden;
c) Optionale Editierung von Asparagin-gekoppelten Glykosilierungspositionen vorzugsweise zu Serin, Alanin oder Glycin;
d) Verknüpfung einer Auswahl der aus den Schritten a) bis c) resultierenden Funktionsmodule mit flexiblen Linkersequenzen, die aus den Aminosäuren Glycin und/oder Serin bestehen und besonders bevorzugt der Linkersequenz (SEQ ID NO: 3) entsprechen, wobei abhängig von der Position des Funktionsmoduls innerhalb des Modubodies und der Klonierungsstrategie eine aus den Schritten a) bis c) resultierende Modulsequenz mit einer flexiblen Linkersequenz verknüpft werden kann und die Linkersequenz den N- oder C-Terminus des Funktionsmoduls bilden kann und wobei optional eine Aminosäuresequenz, die mehrere Funktionsmodule umfasst oder eine den kompletten Modubody aufbauende Aminosäuresequenz, generiert werden kann;
e) Übersetzung der aus den Schritten a) bis d) resultierenden Aminosäuresequenzen in korrespondierende DNA-Sequenzen, vorzugsweise unter Berücksichtigung einer für das beabsichtigte Expressionssystem optimierten Codonhäufigkeitstabelle, wobei die resultierenden DNA-Sequenzen optional mit flankierenden Restriktionsenzymschnittstellen und konservativen Basensubstitutionen zur Vermeidung unerwünschter Restriktionsenzymschnittstellen versehen werden können;
f) Herstellung der in Schritt e) definierten Sequenzen als synthetische DNA durch Gensynthese und Klonierung der genetischen Einheiten, die einzelne Funktionsmodule oder eine Serie mehrere gleicher oder verschiedener Funktionsmodule kodieren, in geeignete Vektoren, Klonierung des kompletten Modubodies durch Zusammenfügen der einzelnen Funktionsmodule oder Funktionsmodulserien
   beabsichtigen Funktionsmodulabfolge und Anzahl, wobei optional auch direkt eine den kompletten Modubody kodierende genetische Einheit kloniert werden kann.

Diese Konstruktionsweise, z.B. die Auswahl räumlich abgegrenzter Domänen, die Vermeidung von Disulfidbrücken, welche ein Dimer stabilisieren, und die Verknüpfung der einzelnen Module durch flexible Glycin-Serin Linker mit einer bevorzugten Länge von 30-40 Aminosäuren zielt darauf ab, dass sich die einzelnen Module voneinander unabhängig verhalten können.

Die Erfindung betrifft außerdem eine Nukleinsäure, z.B. eine DNA oder RNA, die für ein Fusionspolypeptid wie zuvor beschrieben kodiert. Diese Nukleinsäure kann gegebenenfalls in operativer Verknüpfung mit einer Expressionskontrollsequenz, z.B. einem Promotor, vorliegen. Die Erfindung betrifft somit auch Expressionsvektoren, die eine für ein erfindungsgemäßes Fusionspolypeptid kodierende Nukleinsäuresequenz enthalten und zur Expression dieser Nukleinsäure in einer Wirtszelle geeignet sind. Die Wirtszelle kann eine prokaryontische Wirtszelle, wie ein gram-negatives Bakterium wie etwa *E. coli* oder ein gram-positives Bakterium wie etwa *B. subtilis* aber auch eine eukaryontische Wirtszelle, z.B. eine Hefezelle, eine Pilzzelle, eine Insektenzelle oder eine Säugerzelle, sein. Um die Expression der Nukleinsäure in der ausgewählten Wirtszelle zu verbessern, kann sie bezüglich der Codonnutzung in der jeweiligen Wirtszelle optimiert werden. Entsprechende Verfahren sind dem Fachmann bekannt.

Ein weiterer Gegenstand der Erfindung ist eine Wirtszelle wie oben angegeben, die eine erfindungsgemäße Nukleinsäure enthält. Die Nukleinsäure kann mit bekannten Techniken, z.B. Transformation oder Transfektion, in die entsprechende Wirtszelle eingeführt werden. Zur Herstellung des erfindungsgemäßen Fusionspolypeptids kann die Wirtszelle kultiviert und das Fusionspolypeptid nach grundsätzlich bekannten Methoden aus der Zelle oder dem Kulturüberstand gewonnen werden.

Die erfindungsgemäßen Fusionspolypeptide können beispielsweise als Reagenz in einem diagnostischen oder biochemischen Test, insbesondere in einem auf immunologischen Methoden basierenden Test, eingesetzt werden, beispielsweise als Kontroll- oder Kalibratorreagenz oder aber auch als Testreagenz zur Bestimmung eines Analyten. Der Nachweis der erfindungsgemäßen Fusionspolypeptide kann dabei über die CHX-Domäne (n) z.B. unter Verwendung von isotyp- oder speziesspezifischen Erkennungsreagenzien, z.B. Sekundärantikörpern, und/oder über Nicht-Immunglobulin-Erkennungsdomänen unter Verwendung spezifischer Bindepartner für diese Domänen erfolgen. Entsprechende Testformate sind dem Fachmann bekannt.

Weiterhin können das Fusionspolypeptid, die Nukleinsäure oder die Wirtszelle gemäß vorliegender Erfindung auch für medizinische Zwecke, z.B. in der Human- oder Veterinärmedizin, eingesetzt werden. Fusionspolypeptide können beispielsweise als Immuntherapeutika, gegebenenfalls gekoppelt mit Nicht-Immunglobulin-Effektordomänen, z.B. Radionukleiden oder Toxinen, eingesetzt werden. Die für das Fusionspolypeptid kodierenden Nukleinsäuren können beispielsweise als Nukleinsäurevakzine verwendet werden.

Noch ein weiterer Gegenstand der Erfindung ist somit eine pharmazeutische Zusammensetzung, die das Fusionspolypeptid, die Nukleinsäure oder die Wirtszelle zusammen mit pharmazeutisch geeigneten Trägersubstanzen enthält. Die Verabreichung der pharmazeutischen Zusammensetzung kann nach bekannten Methoden, wie sie beispielsweise für die Therapie mit Antikörpern oder die DNA-Vakzinierung verwendet werden, einem Subjekt, z.B. einem menschlichen Patienten, verabreicht werden, welches eine entsprechende therapeutische Behandlung benötigt.

Weiterhin soll die vorliegende Erfindung durch die nachfolgenden Beispiele erläutert werden.

### Beispiele

### Beispiel 1: Konstruktion, Klonierung und Charakterisierung eines β2-Glycoprotein-Bindemoduls (scFv-CAD)

### 1.1 Konstruktion des scFv-CAD

Das folgende Beispiel beschreibt die Konstruktion, eines β2-Glycoprotein-spezifischen Bindemoduls (scFv-CAD) in Form eines synthetischen Konstrukts.

Anteile der variablen Region der leichten Kette VL (SEQ ID NO:1) und der variablen Region der schweren Kette VH (SEQ ID NO:2) des monoklonalen Antikörper WBCAL-1, der aus einem Mausmodell des Antiphospholipidsyndroms stammt, der F1 Maus aus New Zealand white X BXSB (Ichikawa et al. (1999), Arthritis and Rheumatism 42:2461) wurden als Bausteine für ein β2-Glycoproteinerkennungsmodul ausgewählt, mit einem flexiblen Linker (SEQ ID NO: 3) und flankierenden Sequenzen zu einer künstlichen Proteinsequenz scFv-RP-CAD-P (SEQ ID NO: 4) zusammengefügt. Diese Proteinsequenz wurde unter Berücksichtigung einer Codonhäufigkeitstabelle in eine Nukleinsäuresequenz, welche für die Expression in *E. coli* optimiert ist, übersetzt, mit flankierenden Klonierungssequenzen versehen und durch Gensynthese als künstliche DNA-Sequenz scFv-RP-CAD-N, die für ein β2-Glycoprotein-Erkennungsmodul kodiert (SEQ ID NO: 5), hergestellt. Die VL-Domäne erstreckt sich von Aminosäure 1-118 in SEQ ID NO: 4, die VH-Domäne erstreckt sich von Aminosäure 159-272 in SEQ ID NO: 4.

### 1.2 Klonierung des scFv-CAD

Zur Konstruktion wurde die unter Beispiel 1.1 beschriebene synthetische DNA-Sequenz scFv-RP-CAD-N (SEQ ID NO: 5), die für eine β2-Glycoprotein-Detektionsdomäne kodiert, mit den Primern RP-CAD01 (SEQ ID NO: 6), RP-CAD02 (SEQ ID NO: 7) durch Polymerasekettenreaktion (PCR) amplifiziert. Das 856 Basenpaar (bp)-Amplifikat wurde nach Agarosegelelektrophorese mit dem QiaExII-Kit (Qiagen, Hilden) isoliert. Das isolierte Fragment wurde zunächst mit dem Restriktionsenzym Hind III verdaut und anschließend einem BgIII-Partialverdau unterworfen. Die Restriktionsfragmente wurden mit dem Plasmid pQE-80L (Qiagen, Hilden), welches mit den kompatiblen Enzymen BamHI und HindIII verdaut wurde, ligiert und in den *E. coli*-Stamm NovaBlue (Merck, Nottingham) transformiert. Der Transformationsansatz wurde auf LB-Agarplatten, welche mit 50 µg/ml Carbenicillin supplementiert waren, ausplattiert und über Nacht (ü.N.), (16-20 h) bei 36 °C inkubiert. Einzelkolonien des resultierenden *E. coli-* Stammes CAD-pQE80-NovaBlue wurden auf dem Schüttler bei 180 Umdrehungen pro Minute (upm) in LB-Medium, welches mit 50 µg/ml Carbenicillin supplementiert war (LB-Carb), vermehrt (36 °C, ü.N.). Von den Einzelklonkulturen wurden Gefrierstammkulturen angelegt. Je 1 ml der Einzelklonkulturen wurde zur Plasmidpräparation verwendet. Die isolierten Plasmide wurden durch EcoRI/HindIII-Verdau analysiert. Klone mit einem zu erwartenden 901 bp-Fragment wurden durch Induktionsanalyse weiter untersucht. Hierzu wurden die ausgewählten Einzelklone in LB-Carb angezogen und bei einer O.D.500 von 0,5 bis 1,0 durch Zugabe von einem Kulturvolumen LB-Carb, 1 mM Isopropylthiogalactosid (IPTG) zur Expression des durch das klonierte DNA-Fragment kodierten Proteins induziert und 16 Stunden (h), bei 36 °C, 180 upm kultiviert. Die Induktionszellen wurden in Natriumdodecylsulfat (SDS)-haltigem Probenpuffer lysiert, Proteine wurden durch SDS-Polyacrylamidgelelektrophorese (PAGE) getrennt. Im Western-Blot wurde durch Detektion mit einem Anti-RGS-6xHis-Peroxidase gekoppelten Antikörper (Qiagen, Hilden) die Expression eines zu erwartenden 30 kDa scFv-CAD Proteins nachgewiesen. Durch Sequenzierung wurde die korrekte Klonierung bestätigt.

### 1.3 Expression des scFv-CAD

Der unter 1.2 mit dem Expressionskonstrukt transformierte *E*. *coli*-Stamm CAD-pQE80-NovaBlue wurde in LB-Carb Medium bei 36 °C bis zu einer O.D.500 von 0,5 bis 1,0 angezogen und durch Zugabe von IPTG zur Synthese des scFv-CAD induziert. Die induzierte Kultur wurde bei 36 °C für 4 Stunden bis über Nacht kultiviert. Die Induktionszellen wurden geerntet und nach Lysozymbehandlung in einem 8 M Harnstoff-haltigen Tris-HCl-Natriumchlorid-Puffer (TBS) lysiert. Exprimiertes scFv-CAD wurde durch eine Kombination aus Affinitäts- und Ionenaustauscherchromatographie zur Homogenität gereinigt. Im Coomassieblau gefärbten SDS-Polyacrylamidgel ist das scFv-CAD Protein in Form einer 30 kDa-Bande sichtbar. Die Anreicherung der β2-Glycoprotein-Bindeaktivität geht mit der Reinigung der 30 kDa-Bande einher.

### 1.4 Charakterisierung des scFv-CAD

Die Bindungsspezifität des scFv-CAD wurde in Enzyme linked Immunosorbent-Assay (ELISA) mit β2-Glycoprotein bzw. Rinderserumalbumin (BSA) beschichteten Mikrotiterplatten überprüft. Die Stabilität des scFv-CAD gegen Hitzestress wurde an geeigneten Verdünnungen des scFv-CAD untersucht.

### 1.4.1 Bindungsspezifität des scFv-CAD

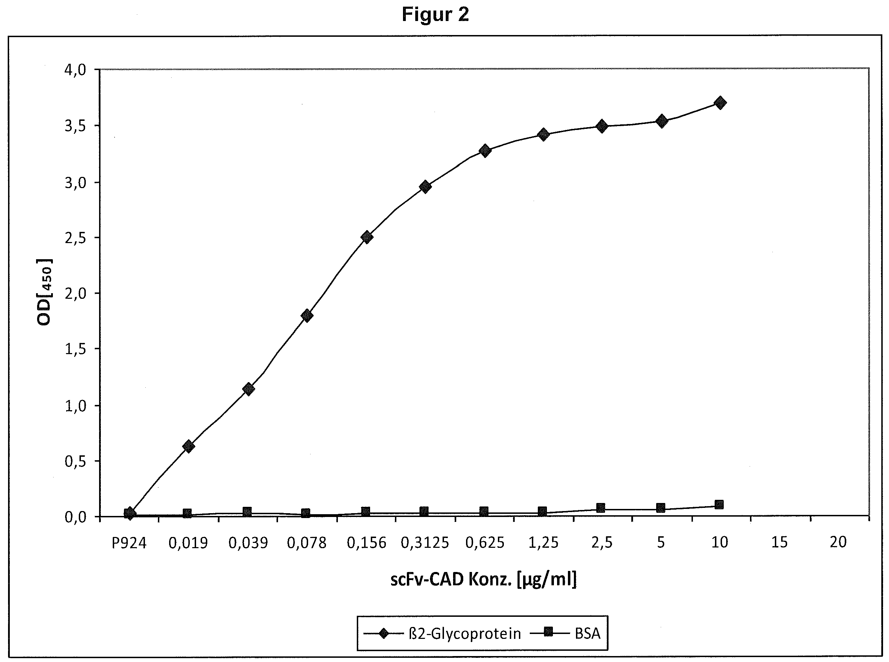

Um zu bestimmen, ob scFv-CAD eine spezifische β2-Glycoproteinbindeaktivtät besitzt, wurde das gereinigte Proteinmodul mit Immunoassays getestet. Hierzu wurden Verdünnungsserien von scFv-CAD auf Mikrotiterplatten des mit β2-Glycoprotein beschichteten Anti-β2-Glycoprotein-Assays (ORG 521, ORGENTEC Diagnostika GmbH, Mainz) und zur Kontrolle der Bindungsspezifität auf BSA-beschichteten Platten aufgetragen und 30 min bei 20-25 °C inkubiert. Die Mikrotiterplatten wurden gewaschen und die Bindung von scFv-CAD wurde mit einem Peroxidase-markierten RGS-6X-His-Antikörper (Qiagen, Hilden) und einer Tetramethylbenzidin (TMB)-Farbreaktion durch Messung der O.D. 450 bestimmt. Hierbei wurde wie in **Figur 2** dargestellt, bereits bei einer Konzentration des scFv-CAD von 19 ng/ml eine spezifische Bindung an β2-Glycoprotein nachgewiesen. Der Vergleich der Reaktionsniveaus der scFv-CAD-Verdünnungsstufen bei der Bindung an β2-Glycoprotein bzw. BSA beschichtete Mikrotiterplatten zeigt, dass im Konzentrationsbereich von 0,019-10 µg/ml scFv-CAD keine unspezifische Bindung auftritt.

### 1.4.2 Temperaturstabilität des scFv-CAD

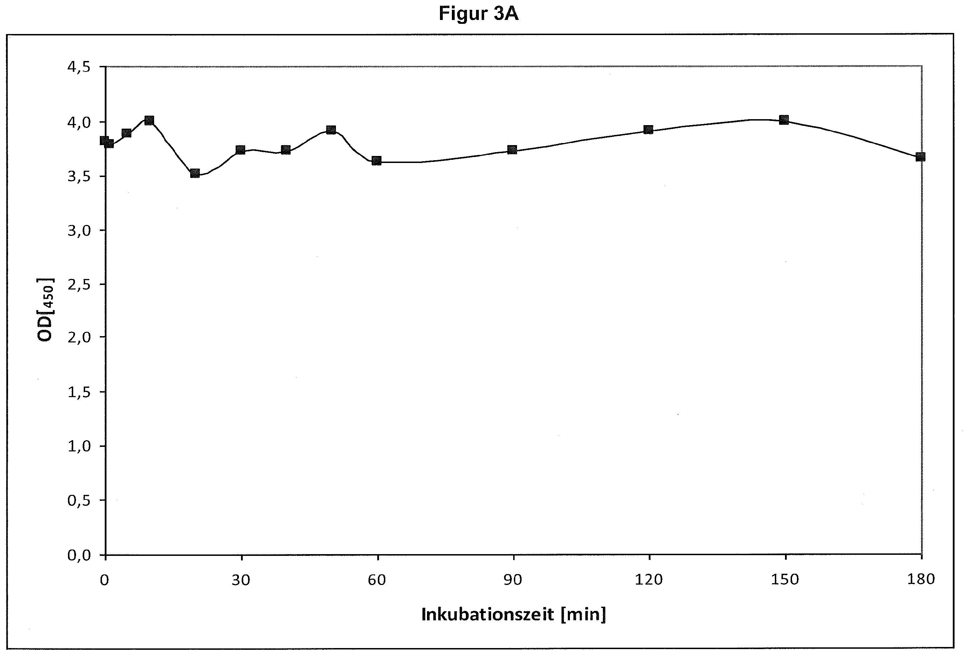

Um den Einfluss von Temperaturstress auf die spezifische β2-Glycoprotein-Bindeaktivtät des scFv-CAD zu untersuchen, wurde das gereinigte Proteinmodul in einer Konzentration von 5 µg/ml über einen Zeitraum von 0-180 Minuten einer Temperatur von 50 °C ausgesetzt. Die Bindung des scFv-CAD an β2-Glycoprotein wurde im Immunoassay wie unter 1.4.1 beschrieben bestimmt. Hierbei erwies sich, wie in **Figur 3A** dargestellt, das in Kalibratorverdünnungsmedium verdünnte scFv-CAD Modul gegenüber einer Temperatur von 50 °C über 3 Stunden als resistent.

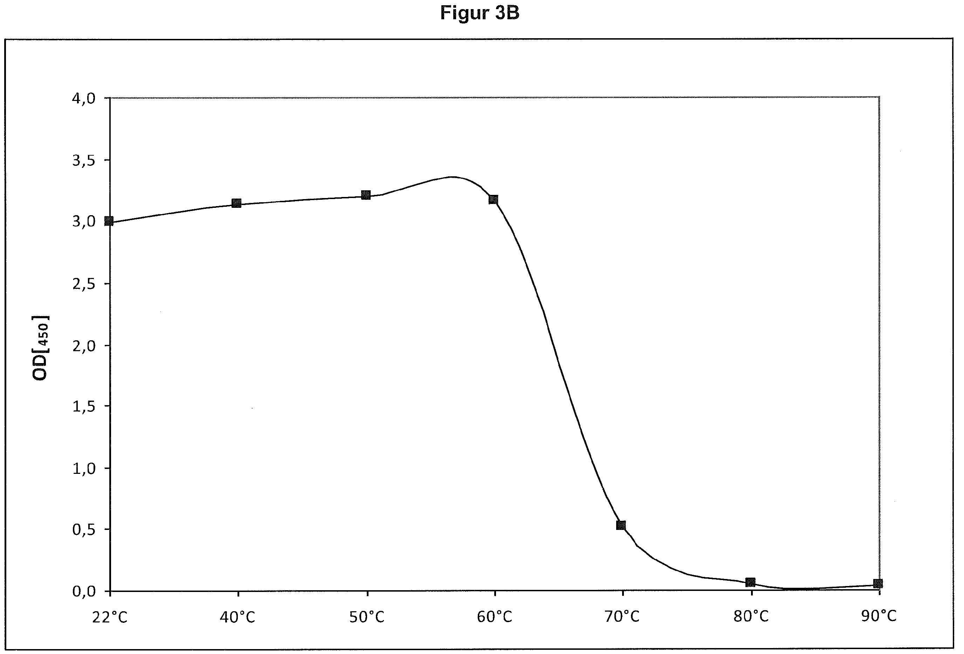

Um die Inaktivierungstemperatur des scFv-CAD zu bestimmen, wurde das gereinigte Proteinmodul in einer Konzentration von 5 µg/ml in Kalibratorverdünnungsmedium für 10 min einer Serie von Temperaturen von 20 °C bis 90 °C ausgesetzt. Die Bindung der hitzebehandelten scFv-CAD-Verdünnungen an β2-Glycoprotein wurde im Immunoassay wie unter 1.4.1 beschrieben bestimmt. Hierbei erwies sich, wie in **Figur 3B** dargestellt, das scFv-CAD Modul bis zu einer Temperatur von 50 °C als stabil, ab einer Temperatur von 60 °C trat eine deutliche Inaktivierung auf.

### Beispiel 2: Konstruktion einer Serie von Reaktionsmodulen für Modubodies

Das folgende Beispiel beschreibt die Konstruktion einer Serie von Reaktionsmodulen, die aus Domänen der konstanten Region der schweren Kette von humanen Antikörpern abgeleitet sind. Hierzu wurden von Proteinsequenzen der schweren Kette humaner Antikörper der Klassen IgG, IgA, IgM mit bekannter Raumstruktur strukturell und funktionell abgegrenzte Domänen abgeleitet. Die ausgewählten Proteinsequenzen wurden in Bezug auf potenzielle Interdomänen-Disulfidbrücken überprüft. Cysteinpositionen, welche außerhalb der gewählten Domäne mit Regionen des intakten Immunglobulins Disulfidbrücken ausbilden, wurden von Cystein zu Serin editiert. An einzelnen Glykosilierungspositionen wurde ebenfalls eine Editierung von Asparagin zu Serin vorgenommen. Die ausgewählten Immunglobulindomänensequenzen wurden als Bausteine für Reaktionsmodule mit einem flexiblen Linker (SEQ ID NO: 3) und flankierenden Sequenzen zu künstlichen Proteinsequenzen zusammengefügt. Diese Reaktionsmodulsequenzen wurden unter Zugrundelegen einer Codonhäufigkeitstabelle in eine Nukleinsäuresequenz, welche für die Expression in *E. coli* optimiert ist, übersetzt, mit flankierenden Klonierungssequenzen versehen und durch Gensynthese als künstliche DNA-Sequenzen hergestellt.

### 2.1 Konstruktion eines humanen IgG-CH1-Reaktionsmoduls

Aus der Worldwide Protein Data Bank (pdb), (Berman et al. (2003), Nature Structural Biology 10: 980) wurden aus der Kristallstruktur des Fab-Fragments des Anti-Factor Ix-Antikörpers 10c12 anhand des Proteinstrukturdatenbankeintrages pdb 3D69H die Sequenzpositionen 125-219 als repräsentative IgG-CH1-Domäne ausgewählt. Die ausgewählte IgG-CH1 Immunglobulindomänensequenz wurde als Baustein für ein IgG-CH1-Reaktionsmodul mit einem flexiblen Linker (SEQ ID NO: 3) zu einer künstlichen Proteinsequenz Sc-RP-CH1-G-P (SEQ ID NO: 8) zusammengefügt und unter Berücksichtigung einer Codonhäufigkeitstabelle in eine Nukleinsäuresequenz, welche für die Expression in *E. coli* optimiert ist, übersetzt, zusätzlich mit flankierenden Klonierungssequenzen (BamHI, HindIII) versehen und durch Gensynthese als künstliche DNA-Sequenz Sc-RP-CH1-G-N (SEQ ID NO: 9) hergestellt. Die IgG-CH1-Domäne erstreckt sich von Aminosäure 37-131 in SEQ ID NO: 8.

### 2.2 Konstruktion eines humanen IgG-CH2-Reaktionsmoduls

Aus der Kristallstruktur des mutierten Adcc-vertärkten Fc-Fragmentes wurden anhand des Proteinstrukturdatenbankeintrages pdb 2QL1A die Sequenzpositionen 15-116 als repräsentative IgG-CH2-Domäne ausgewählt. Die ausgewählte IgG-CH2-Immunglobulindomänensequenz wurde als Baustein für ein IgG-CH2-Reaktionsmodul mit einem flexiblen Linker (SEQ ID NO: 3) zu einer künstlichen Proteinsequenz Sc-RP-CH2-G-P (SEQ ID NO: 10) zusammengefügt und unter Berücksichtigung einer Codonhäufigkeitstabelle in eine Nukleinsäuresequenz, welche für die Expression in *E. coli* optimiert ist, übersetzt, zusätzlich mit flankierenden Klonierungssequenzen (BamHI/HindIII) versehen und durch Gensynthese als künstliche DNA-Sequenz Sc-RP-CH2-G-N (SEQ ID NO: 11) hergestellt. Die IgG-CH2-Domäne erstreckt sich von Aminosäure 37-138 in SEQ ID NO: 10.

### 2.3 Konstruktion eines humanen IgG-CH3-Reaktionsmoduls

Aus der Kristallstruktur der schweren Kette eines humanen Immunglobulins mit einer Hinge-Deletion wurden anhand des Proteinstrukturdatenbankeintrages pdb 1 MCO_H (GI: 494350) die Sequenzpositionen 330-428 als repräsentative IgG-CH3-Domäne ausgewählt. Die ausgewählte IgG-CH3-Immunglobulindomänensequenz wurde als Baustein für ein IgG-CH3-Reaktionsmodul mit einem flexiblen Linker (SEQ ID NO: 3) zu einer künstlichen Proteinsequenz Sc-RP-CH3-G-P (SEQ ID NO: 12) zusammengefügt und unter Berücksichtigung einer Codonhäufigkeitstabelle in eine Nukleinsäuresequenz, welche für die Expression in *E. coli* optimiert ist, übersetzt, zusätzlich mit flankierenden Klonierungssequenzen (BamHI/HindIII) versehen und durch Gensynthese als künstliche DNA-Sequenz Sc-RP-CH3-G-N (SEQ ID NO: 13) hergestellt. Die IgG-CH3-Domäne erstreckt sich von Aminosäure 37-135 in SEQ ID NO: 12.

### 2.4 Konstruktion eines humanen IgA-CH2-Reaktionsmoduls

Aus einem Strukturmodell von humanem IgA, welches auf Basis von Neutronenstreuung in Lösung und Homologiemodellierung abgeleitet wurde (Boehm et al. (1999) J.Mol.Biol. 286: 1421-1447), wurden aus dem Proteinstrukturdatenbankeintrag pdb 1IGA anhand der Raumstruktur der schweren Kette von humanem IgA1 die Sequenzpositionen 126-222 (entsprechend UNIPROT P01876) als repräsentative IgA-CH2-Domäne ausgewählt. An den Positionen 182, 192 wurde Cystein zu Serin editiert. Die ausgewählte und editierte 1gA-CH2-Immunglobulindomänensequenz wurde als Baustein für ein IgA-CH2-Reaktionsmodul mit einem flexiblen Linker (SEQ ID NO: 3) zu einer künstlichen Proteinsequenz Sc-RP-CH2-A-P (SEQ ID NO: 14) zusammengefügt und unter Berücksichtigung einer Codonhäufigkeitstabelle in eine Nukleinsäuresequenz, welche für die Expression in *E. coli* optimiert ist, übersetzt, zusätzlich mit flankierenden Klonierungssequenzen (BamHI/HindIII) versehen und durch Gensynthese als künstliche DNA-Sequenz Sc-RP-CH2-A-N (SEQ ID NO: 15) hergestellt. Die IgA-CH2-Domäne erstreckt sich von Aminosäure 37-133 in SEQ ID NO: 14.

### 2.5 Konstruktion eines humanen IgA-CH3-Reaktionsmoduls

Aus dem Proteinstrukturdatenbankeintrag PDB 1IGA wurden anhand der Raumstruktur der schweren Kette von humanem IgA1 die Sequenzpositionen 227-331 (entsprechend UNIPROT P01876) als repräsentative IgA-CH3-Domäne ausgewählt. Die ausgewählte IgA-CH3-Immunglobulindomänensequenz wurde als Baustein für ein IgA-CH3-Reaktionsmodul mit einem flexiblen Linker (SEQ ID NO: 3) zu einer künstlichen Proteinsequenz sc-RP-CH3-A-P (SEQ ID NO: 16) zusammengefügt und unter Berücksichtigung einer Codonhäufigkeitstabelle in eine Nukleinsäuresequenz, welche für die Expression in *E. coli* optimiert ist, übersetzt, zusätzlich mit flankierenden Klonierungssequenzen (BamHI/HindIII) versehen und durch Gensynthese als künstliche DNA-Sequenz sc-RP-CH3-A-N (SEQ ID NO: 17) hergestellt. Die IgA-CH3-Domäne erstreckt sich von Aminosäure 37-141 in SEQ ID NO: 16.

### 2.6 Konstruktion eines humanen IgM-CH2-Reaktionsmoduls

Aus einem Strukturmodell von humanem IgM, welches auf Basis von Röntgenstreuung in Lösung und Modellierung abgeleiteten wurde (Perkins et al. (1991) J.Mol.Biol. 221:1345-1366), wurden aus dem Proteinstrukturdatenbankeintrag pdb 2rcj anhand der Raumstruktur der schweren Kette von humanem IgM die Sequenzpositionen 106-217 (entsprechend UNIPROT P01871) als repräsentative IgM-CH2-Domäne ausgewählt und editiert. An der Position 214 wurde Cystein zu Serin editiert, an der Position 109 wurde Asparagin zu Serin editiert. Die ausgewählte und editierte IgM-CH2-Immunglobulindomänensequenz wurde als Baustein für ein IgM-CH2- Reaktionsmodul mit einem flexiblen Linker (SEQ ID NO: 3) zu einer künstlichen Proteinsequenz Sc-RP-CH2-M-P (SEQ ID NO: 18) zusammengefügt und unter Berücksichtigung einer Codonhäufigkeitstabelle in eine Nukleinsäuresequenz, welche für die Expression in *E. coli* optimiert ist, übersetzt, zusätzlich mit flankierenden Klonierungssequenzen (BamHI/HindIII) versehen und durch Gensynthese als künstliche DNA-Sequenz Sc-RP-CH2-M-N (SEQ ID NO: 19) hergestellt. Die IgM-CH2-Domäne erstreckt sich von Aminosäure 37-148 in SEQ ID NO: 18.

### 2.7 Konstruktion eines humanen IgM-CH3-Reaktionsmoduls

Aus dem Proteinstrukturdatenbankeintrag pdb 2rcj wurde anhand der Raumstruktur der schweren Kette von humanem IgM die Sequenzpositionen 218-323 (entsprechend UNIPROT P01871) als repräsentative IgM-CH3-Domäne ausgewählt und editiert. An der Position 291 wurde Cystein zu Serin editiert, an den Positionen 272 und 279 wurde Asparagin zu Serin editiert. Die ausgewählte und editierte IgM-CH3-Immunglobulindomänensequenz wurde als Baustein für ein IgM-CH3-Reaktionsmodul mit einem flexiblen Linker (SEQ ID NO: 3) zu einer künstlichen Proteinsequenz Sc-RP-CH3-M-P (SEQ ID NO: 20) zusammengefügt und unter Berücksichtigung einer Codonhäufigkeitstabelle in eine Nukleinsäuresequenz, welche für die Expression in *E. coli* optimiert ist, übersetzt, zusätzlich mit flankierenden Klonierungssequenzen (BamHI/HindIII) versehen und durch Gensynthese als künstliche DNA-Sequenz Sc-RP-CH3-M-N (SEQ ID NO: 21) hergestellt. Die IgM-CH3-Domäne erstreckt sich von Aminosäure 37-142 in SEQ ID NO: 20.

### 2.8 Konstruktion eines humanen IgM-CH4-Reaktionsmoduls

Aus dem Proteinstrukturdatenbankeintrag pdb 2rcj wurde anhand der Raumstruktur der schweren Kette von humanem IgM die Sequenzpositionen 324-452 (entsprechend UNIPROT P01871) als repräsentative IgM-CH3-Domäne ausgewählt und editiert. An der Position 451 wurde Cystein zu Serin editiert, an der Positionen 439 wurde Asparagin zu Serin editiert. Die ausgewählte und editierte IgM-CH3-Immunglobulindomänensequenz wurde als Baustein für ein IgM-CH3- Reaktionsmodul mit einem flexiblen Linker (SEQ ID NO: 3) zu einer künstlichen Proteinsequenz Sc-RP-CH4-M-P (SEQ ID NO: 22) zusammengefügt und unter Berücksichtigung einer Codonhäufigkeitstabelle in eine Nukleinsäuresequenz, welche für die Expression in *E. coli* optimiert ist, übersetzt, zusätzlich mit flankierenden Klonierungssequenzen (BamHI/HindIII) versehen und durch Gensynthese als künstliche DNA-Sequenz Sc-RP-CH4-M-N (SEQ ID NO: 23) hergestellt. Die IgM-CH4-Domäne erstreckt sich von Aminosäure 37-165 in SEQ ID NO: 22.

### 2.9 Konstruktion eines monovalenten humanen IgG-CH3-Knob02 -Reaktionsmoduls

Aus der Kristallstruktur des schweren Kette eines humanen Immunglobulins mit einer Hinge-Deletion wurden anhand des Proteinstrukturdatenbankeintrages pdb 1MCO_H (GI:494350) die Sequenzpositionen 330-428 als repräsentative IgG-CH3-Domäne ausgewählt und editiert. Hierbei wurden Positionen von Aminosäureresten, deren Seitenketten an der Grenzfläche von IgG₁-CH3-Dimeren (Ridgway J.B.B et al. (1996), Protein Engineering, 9: 617-621) Kontakte ausbilden, so modifiziert, dass keine Dimerisierung der IgG-CH3-Domäne erfolgen kann. An den Positionen 351 und 379 wurde Threonin zu Tyrosin editiert, an der Position 390 wurde Phenylalanin zu Tyrosin editiert. Die ausgewählte und editierte IgG-CH3 Immunglobulindomänen-sequenz wurde als Baustein für ein IgG-CH3-Knob02- Reaktionsmodul mit einem flexiblen Linker (SEQ ID NO: 3) zu einer künstlichen Proteinsequenz Sc-RP-CH3-G-Knob02-P (SEQ ID NO: 32) zusammengefügt und unter Berücksichtigung einer Codonhäufigkeitstabelle in eine Nukleinsäuresequenz, welche für die Expression in *E. coli* optimiert ist, übersetzt, zusätzlich mit flankierenden Klonierungssequenzen (BamHI/HindIII) versehen und durch Gensynthese als künstliche DNA-Sequenz Sc-RP-CH3-G-Knob02-N (SEQ ID NO: 33) hergestellt. Die IgG-CH3-Knob02-Domäne erstreckt sich von Aminosäure 37-135 in SEQ ID NO: 32.

### Beispiel 3: Der einzelkettige CAD-IgG-CH3 Modubody

### 3.1 Konstruktion und Klonierung des einzelkettigen CAD-IgG-CH3 Modubody

Das folgende Beispiel beschreibt die Konstruktion und Klonierung des β2-Glycoprotein-spezifischen und mit einer humanen IgG-CH3-Detektionsdomäne versehenen Modubodies CAD-IgG-CH3.

Zur Konstruktion des CAD-IgG-CH3-Modubodies wurden die Module scFv-CAD (Beispiel 1) und IgG-CH3 (Beispiel 2.3) durch Restriktionsverdau und Ligation in der Domänenreihenfolge VL-Linker-VH-Linker-IgG-CH3 zu einer CAD-IgG-CH3 kodierenden Sequenz (SEQ ID NO: 26) zusammengesetzt. Hierzu wurde das unter Beispiel 2.3 beschriebene synthetische IgG-CH3-Reaktionsmodul Sc-RP-CH3-G-N entsprechend der Sequenz SEQ ID NO: 13 mit den Primern CH05 (SEQ ID NO: 24) und CH04 (SEQ ID NO: 25) durch PCR amplifiziert. Das 443 b-Amplifikat wurde nach Agarosegelelektrophorese mit dem QiaExII-Kit (Qiagen, Hilden) isoliert. Das isolierte Fragment wurde zunächst mit den Restriktionsenzymen Bcll und HindIII verdaut. Die Restriktionsfragmente wurden mit dem unter Beispiel 1 beschriebenen CAD-scFv-pQE80-Vektorkonstrukt, welches mit den kompatiblen Enzymen BamHI und HindIII verdaut wurde, ligiert und in den *E. coli* Stamm NovaBlue (Merck, Nottingham) transformiert. Der Transformationsansatz wurde auf LB-Agarplatten, die mit Carbenicillin (50 µg/ml) supplementiert waren, ausplattiert und ü.N. bei 36 °C inkubiert. Einzelkolonien des resultierenden *E*. *coli*-Stammes CAD-IgG-CH3-pQE80-NovaBlue wurden in LB-Carb Medium vermehrt (36 °C, ü.N. 180 upm). Von den Einzelklonkulturen wurden Gefrierstammkulturen angelegt, je 1 ml der Einzelklonkulturen wurde zur Plasmidpräparation verwendet. Die isolierten Plasmide wurden durch EcoRI/HindIII-Verdau analysiert. Klone mit einem zu erwartenden 1312 bp-Fragment wurden durch Induktionsanalyse weiter untersucht. Hierzu wurden die ausgewählten Einzelklone in LB-Carb angezogen und bei einer O.D.500 von 0,5 bis 1,0 durch Zugabe von einem Kulturvolumen LB-Carb, welches mit 1 mM IPTG supplementiert war, zur Expression des rekombinanten Proteins induziert und 16 Stunden bei 36 °C, 180 upm kultiviert. Die Induktionszellen wurden in SDS-Probenpuffer lysiert, Proteine wurden durch SDS-PAGE getrennt. Im Western Blot wurde durch Detektion mit einem Anti-RGS-6xHis-Peroxidase gekoppelten Antikörper (Qiagen, Hilden) die Expression des zu erwartenden 44 kDa- CAD-IgG-CH3-Modubodies nachgewiesen. Durch Sequenzierung wurde die korrekte Klonierung bestätigt.

### 3.2 Expression und Reinigung des einzelkettigen CAD-IgG-CH3-Modubodies

Das folgende Beispiel beschreibt die Expression und Reinigung des β2-Glycoprotein-spezifischen und mit einer humanen IgG-CH3-Detektionsdomäne versehenen Modubodies CAD-IgG-CH3.

Der unter 3.1 mit dem Expressionskonstrukt transformierte *E. coli*-Stamm CAD-IgG-CH3-pQE80-NovaBlue wurde in LB-Carb Medium bei 36 °C bis zu einer O.D.500 von 0,5 bis 1.0 angezogen und durch Zugabe von IPTG zur Synthese des CAD-IgG-CH3 Modubodies induziert. Die induzierte Kultur wurde bei 36 °C für 4 Stunden bis über Nacht kultiviert. Die Induktionszellen wurden geerntet und nach Lysozymbehandlung in einem 8 M Harnstoff-haltigen TBS-Puffer lysiert. Exprimierte CAD-IgG-CH3-Modubodies wurden durch eine Kombination aus Affinitäts- und Ionenaustauscherchromatographie zur Homogenität gereinigt. Im Coomassieblau gefärbten SDS-Polyacrylamidgel ist eine 44 kDa-Bande sichtbar. Die Anreicherung der β2-Glycoprotein-Bindeaktivität und einer Reaktivität mit einem Peroxidase-gekoppelten Anti-human-IgG-Sekundärantikörper (Jackson Immunoresearch) geht mit der Reinigung der 44 kDa-Bande einher.

### 3.3 Charakteristika des einzelkettigen CAD-IgG-CH3-Modubodies

Das folgende Beispiel beschreibt die Charakterisierung des β2-Glycoprotein-spezifischen und mit einer humanen IgG-CH3-Detektionsdomäne versehenen Modubodies CAD-IgG-CH3 in Bezug auf spezifische Antigenerkennung, spezifischer Nachweisbarkeit über einen Anti-human-IgG-Sekundärantikörper und Stabilität gegen erhöhte Temperaturen, Eintrocknen und Gefriertauzyklen.

### 3.3.1 Kalibratorfunktion des CAD-IgG-CH3-Modubodies

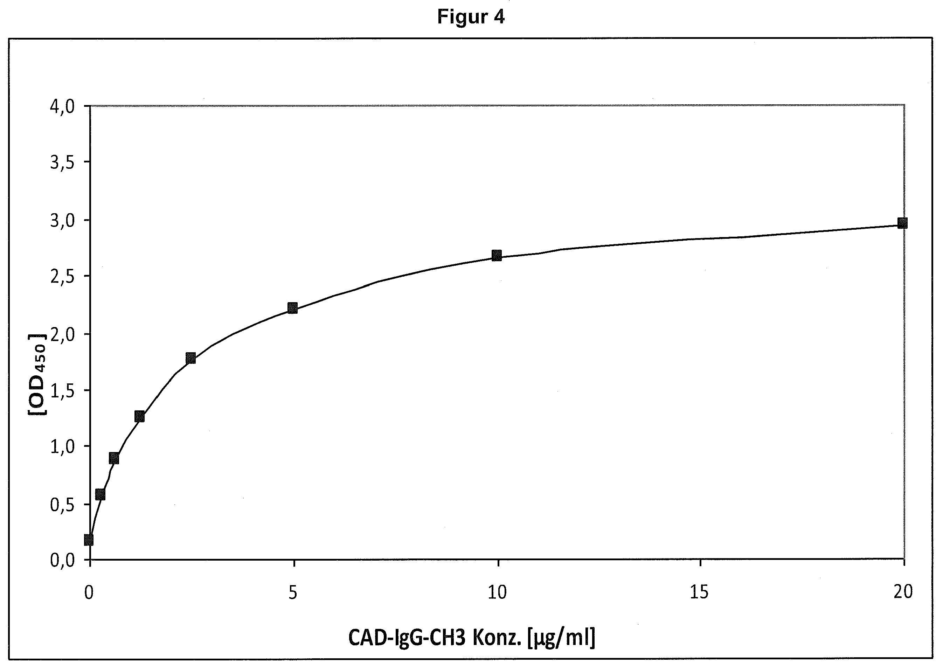

Um zu bestimmen, ob der CAD-IgG-CH3-Modubody die β2-Glycoprotein-Bindungs-Aktivität des scFv-CAD beibehalten hat und die Bindung an das Antigen über einen Anti-human-IgG-Sekundärantikörper spezifisch nachgewiesen werden kann, wurden gereinigte CAD-IgG-CH3-Präparationen im Anti-β2-Glycoproteinimmunoassay untersucht. Der gereinigte CAD-IgG-CH3-Modubody wurde in einer Verdünnungsserie mit den Konzentrationen 0 µg/ml, 0,31 µg/ml, 0,62 µg/µl, 1,25 µg/ml, 2,5 µg/ml, 5 µg/ml, 10 µg/ml, 20 µg/ml in Kalibratorverdünnungsmedium auf mit β2-Glycoprotein beschichteten Mikrotiterplatten aufgetragen. Die Antigen-Bindungsaktivität wurde unter den für den Anti-β2-Glycoprotein-Assay (ORG 521, ORGENTEC GmbH, Mainz) vorgesehenen Inkubationsbedingungen und einem Anti-human-IgG-Peroxidase markierten Sekundärantikörper in der Konzentration von 80 ng/ml bestimmt. In **Figur 4** ist der Verlauf der unter den gewählten Reaktionsbedingungen bestimmten OD 450 nm in Abhängigkeit von der Konzentration dargestellt. Unter den gewählten Reaktionsbedingungen wurde der CAD-IgG-CH3-Modubody bei einer Konzentration von 5 µg/ml mit einer O.D. 450 von 2,2 nachgewiesen.

### 3.3.2 Stabilität des CAD-IgG-CH3-Modubodies

Zur Charakterisierung der Robustheit des CAD-IgG-CH3-Modubodies wurden Verdünnungen in Kalibratorverdünnungsmedium den Stressfaktoren 50 °C-Hitzestress, erhöhte Lagertemperatur bei 36 °C, Trockenheit und wiederholte Gefrier-Auftauzyklen ausgesetzt. Im Vergleich zu unbehandelten Proben wurde die β2-Glycoprotein-Bindeaktivität und die Detektierbarkeit mit einem Anti-hu-IgG-Peroxidase markierten Sekundärantikörper (Jackson Immunoresearch) bestimmt.

### 3.3.2.1 Stabilität des CAD-IgG-CH3- Modubodies gegenüber Hitzestress

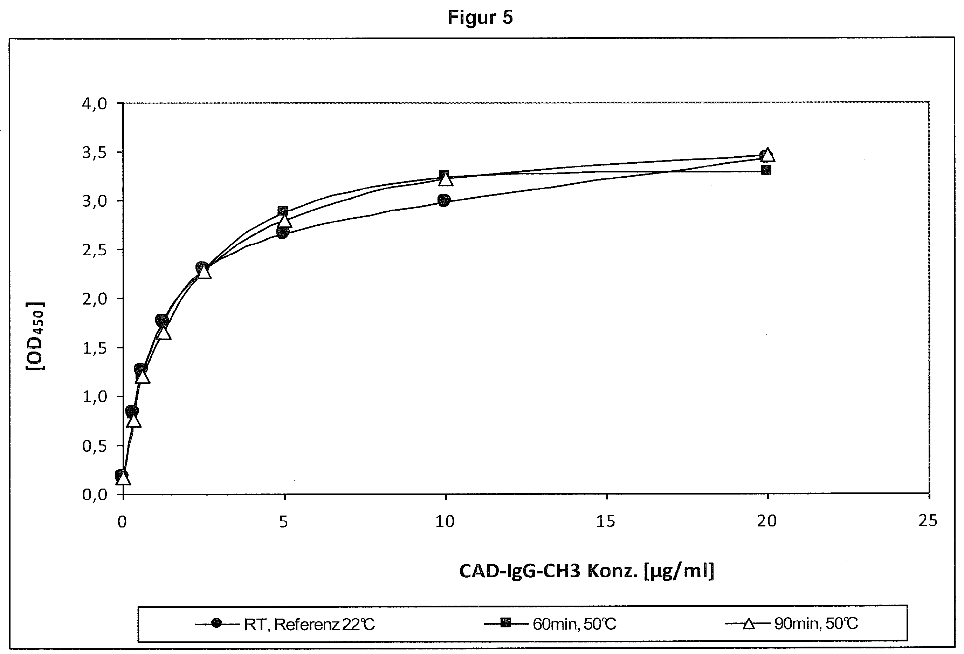

Die Stabilität gegenüber Hitzestress wurde untersucht, indem Verdünnungen des CAD-IgG-CH3-Modubodies in Kalibratorverdünnungsmedium in den Konzentrationen 0 µg/ml, 0,31 µg/ml, 0,62 µg/µl, 1,25 µg/ml, 2,5 pµ/ml, 5 µg/ml, 10 µg/ml, 20 µg/ml für 60 Minuten und 90 Minuten bei 50 °C inkubiert wurden und im Vergleich zu einer bei Raumtemperatur gelagerten Verdünnungsserie im Anti-β2-Glycoproteinimmunoassay untersucht wurden. Hierbei wurde die β2-Glycoprotein-Bindungsaktivität unter den für den Anti-β2-Glycoprotein-Assay (ORG 521, ORGENTEC GmbH, Mainz) vorgesehenen Inkubationsbedingungen und einem Anti-human-IgG-Peroxidase markierten Sekundärantikörper in der Konzentration von 80 ng/ml bestimmt. In **Figur 5** ist der Verlauf der unter den gewählten Reaktionsbedingungen bestimmten OD 450 nm für die Verdünnungsserien bei 60- und 90-minütiger Inkubation bei 50 °C im Vergleich zur bei Raumtemperatur gelagerten Verdünnungsserie dargestellt. Unter den gewählten Reaktionsbedingungen wurde die Funktion des CAD-IgG-CH3-Modubodies in Verdünnungen im Konzentrationsbereich von 0,31 µg/ml bis 20 µg/ml durch 50 °C Temperaturstress für 60 Minuten oder 90 Minuten nicht beeinträchtigt.

### 3.3.2.2 Stabilität des CAD-IgG-CH3-Modubodies gegenüber der Lagerung bei 36 °C

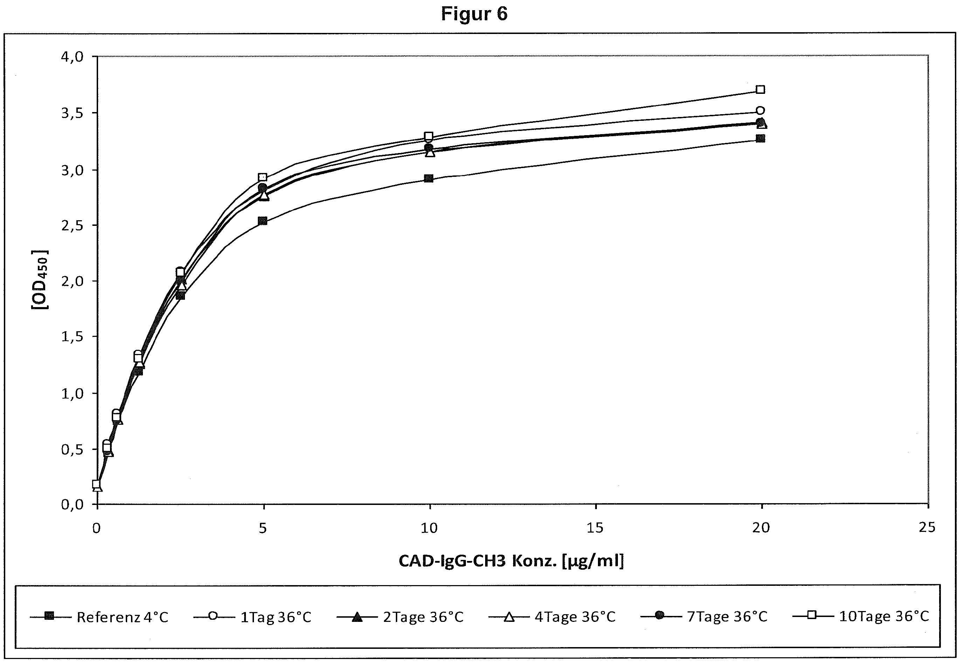

Die Stabilität gegenüber erhöhter Lagertemperatur wurde untersucht, indem Verdünnungen des CAD-IgG-CH3-Modubodies in den Konzentrationen 0 µg/ml, 0,31 µg/ml, 0,62 µg/µl, 1,25 µg/ml, 2,5 µg/ml, 5 µg/ml, 10 µg/ml, 20 µg/ml in Kalibratorverdünnungsmedium für 1, 2, 4, 7, 10 Tage bei 36 °C inkubiert wurden und im Vergleich zu einer bei 4 °C gelagerten Verdünnungsserie im Anti-β2-Glycoproteinimmunoassay untersucht wurden. Hierbei wurde die β2-Glycoprotein-Bindungsaktivität unter den für den Anti-β2-Glycoprotein-Assay (ORG 521, ORGENTEC GmbH, Mainz) vorgesehenen Inkubationsbedingungen und einem Anti-human-IgG-Peroxidase markierten Sekundärantikörper in der Konzentration von 80 ng/ml bestimmt. In **Figur 6** ist der Verlauf der unter den gewählten Reaktionsbedingungen bestimmten OD 450 nm für die Verdünnungsserien bei 1, 2, 4, 7, 10 tägiger Lagerung 36 °C im Vergleich zur bei 4 °C gelagerten Verdünnungsserie dargestellt. Unter den gewählten Reaktionsbedingungen wurde die Funktion des CAD-IgG-CH3-Modubodies in Verdünnungen im Konzentrationsbereich von 0,31 µg/ml bis 20 µg/ml durch eine Lagerung bei 36 °C über einen Zeitraum von zehn Tagen nicht beeinträchtigt.

### 3.3.2.3 Stabilität des CAD-IgG-CH3- Modubodies gegenüber Eintrocknung

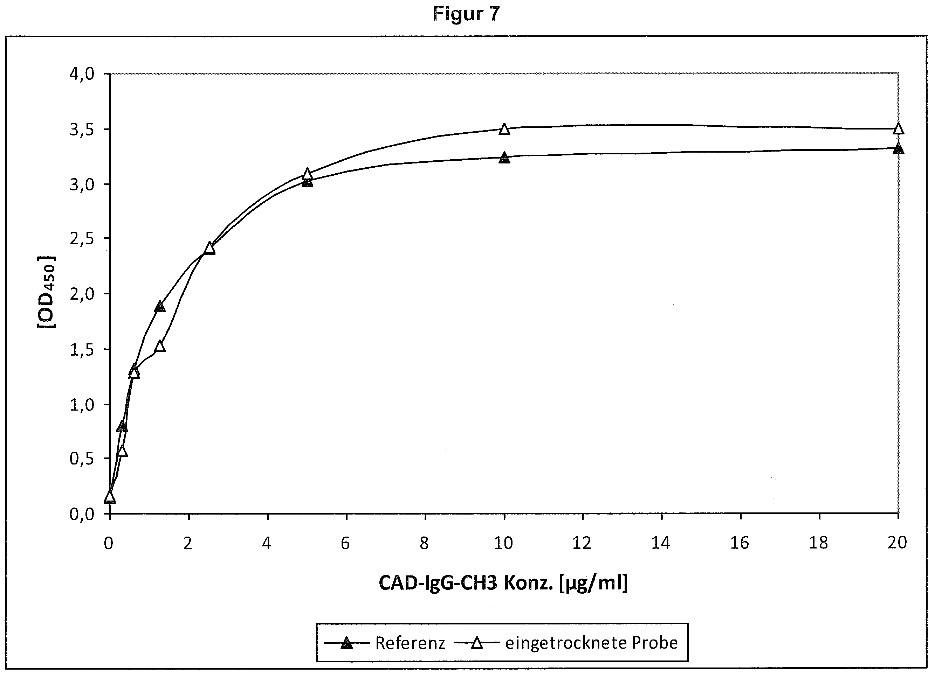

Die Stabilität gegenüber Eintrocknung wurde untersucht, indem Verdünnungen des CAD-IgG-CH3-Modubodies in Kalibratorverdünnungsmedium in 50 µl Portionen in den Konzentrationen 0 µg/ml, 3,1 µg/ml, 6,21 µg/ml, 12,5 µg/ml, 25 µg/ml, 50 µg/ml, 100 µg/ml, 200 µg/ml in einer Speedvak unter Vakuum bei 22 °C eingetrocknet wurden. Die getrockneten Proben wurden mit 450 µl Kalibratorverdünnungsmedium und 50 µl Wasser zu einer Verdünnungsserie mit den Konzentrationen 0 µg/ml, 0,31 µg/ml, 0,62 µg/µl, 1,25 µg/ml, 2,5 µg/ml, 5 µg/ml, 10 µg/ml, 20 µg/ml resolubilisiert und im Vergleich zu einer unbehandelten Verdünnungsserie im Anti-β2-Glycoprotein-Cardiolipinimmunoassay untersucht. Hierbei wurde die β2-Glycoprotein-Bindungsaktivität unter den für den Anti-Cardiolipin-β2-Glycoprotein-Assay (ORG 515, ORGENTEC GmbH, Mainz) vorgesehenen Inkubationsbedingungen und einem Anti-human-IgG-Peroxidase markierten Sekundärantikörper in der Konzentration von 80 ng/ml bestimmt. In **Figur 7** ist der Verlauf der unter den gewählten Reaktionsbedingungen bestimmten OD 450 nm für die eingetrockneten Proben im Vergleich zur unbehandelten Verdünnungsserie dargestellt. Unter den gewählten Reaktionsbedingungen wurde die Funktion des CAD-IgG-CH3-Modubodies in Verdünnungen im Konzentrationsbereich von 3,1 µg/ml bis 200 µg/ml durch Eintrocknen nicht beeinträchtigt.

### 3.3.2.4 Stabilität des CAD-IgG-CH3-Modubodies gegenüber Gefrier-Auftauzyklen

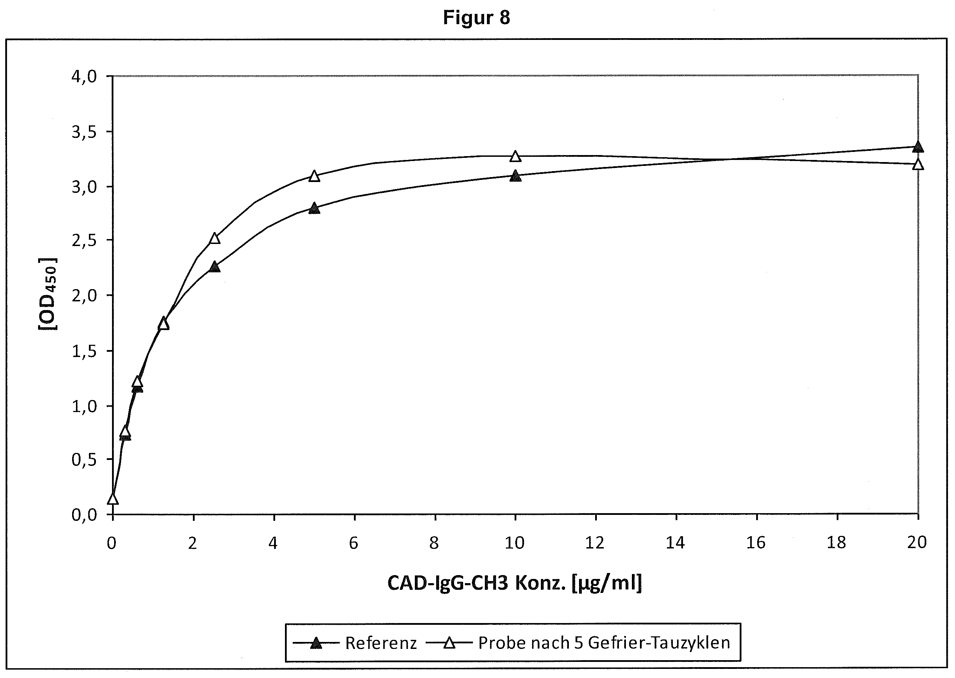

Die Stabilität gegenüber wiederholten Gefrier-Auftauzyklen wurde untersucht, indem Verdünnungen des CAD-IgG-CH3-Modubodies in Kalibratorverdünnungsmedium in 50 µl Portionen in den Konzentrationen 0 µg/ml, 3,1 µg/ml, 6,21 µg/ml, 12,5 µg/ml, 25 µg/ml, 50 µg/ml, 100 µg/ml, 200 µg/ml in fünf wiederholten Gefrier-Auftauzyklen bei -70 °C eingefroren und bei 37 °C wieder aufgetaut wurden. Anschließend wurden die Proben mit 450 µl Kalibratorverdünnungsmedium zu einer Verdünnungsserie mit den Konzentrationen 0 µg/ml, 0,31 µg/ml, 0,62 µg/µl, 1,25 µg/ml, 2,5 µg/ml, 5 µg/ml, 10 µg/ml, 20 µg/ml verdünnt und im Vergleich zu einer unbehandelten CAD-IgG-CH3-Verdünnungsserie im Anti-β2-Cardiolipin-Glycoprotein-immunoassay untersucht. Hierbei wurde die β2-Glycoprotein-Bindungsaktivität unter den für den Anti-Cardiolipin-β2-Glycoprotein-Assay (ORG 515, ORGENTEC GmbH, Mainz) vorgesehenen Inkubationsbedingungen und einem Anti-human-IgG-Peroxidase markierten Sekundärantikörper in der Konzentration von 80 ng/ml bestimmt. In **Figur 8** ist der Verlauf der unter den gewählten Reaktionsbedingungen bestimmten OD 450 nm für die wiederholt gefrorenen und aufgetauten Proben im Vergleich zur unbehandelten Verdünnungsserie dargestellt. Unter den gewählten Reaktionsbedingungen wurde die Funktion des CAD-IgG-CH3-Modubodies im Konzentrationsbereich von 3,1 µg/ml bis 200 µg/ml durch wiederholte Gefrier- Auftauzyklen nicht beeinträchtigt.

### Beispiel 4: Der einzelkettige CAD-IgG-CH2-Modubody

### 4.1 Konstruktion und Klonierung des einzelkettigen CAD-IgM-CH2-Modubodies

Das folgende Beispiel beschreibt die Konstruktion und Klonierung des β2-Glycoprotein-spezifischen und mit einer monomeren humanen IgG-CH2-Detektionsdomäne versehenen Modubodies CAD-IgG-CH2.

Zur Konstruktion des CAD-IgG-CH2-Modubodies wurden die Module scFv-CAD (Beispiel 1) und IgG-CH2 (Beispiel 2.2) durch Restriktionsverdau und Ligation in der Domänenreihenfolge VL-Linker-VH-Linker-IgG-CH2 zu einer CAD-IgG-CH2 kodierenden Sequenz (SEQ ID NO: 27) zusammengesetzt. Hierzu wurde das unter Beispiel 2.2 beschriebene synthetische IgG-CH2-Reaktionsmodul Sc-RP-CH2-G-N entsprechend der Sequenz SEQ ID NO: 11 mit den Primern CH03 (SEQ ID NO: 28) und CH04 (SEQ ID NO: 25) durch PCR amplifiziert. Das 443 bp-Amplifikat wurde nach Agarosegelelektrophorese mit dem QiaExII-Kit (Qiagen, Hilden) gelisoliert. Das gelisolierte Fragment wurde zunächst mit den Restriktionsenzymen BcII und HindIII verdaut. Die Restriktionsfragmente wurden mit dem unter Beispiel 1 beschriebenen scFv-CAD-pQE80 Vektorkonstrukt, welches mit den kompatiblen Enzymen BamHI und HindIII verdaut wurde, ligiert und in den *E*. *coli*-Stamm NovaBlue (Merck, Nottingham) transformiert. Der Transformationsansatz wurde auf LB-Agarplatten, die mit Carbenicillin (50 µg/ml) supplementiert waren, ausplattiert und über Nacht bei 36 °C inkubiert. Einzelkolonien des resultierenden *E*. *coli*-Stammes CAD-IgG-CH2-pQE80-NovaBlue wurden in LB-Medium, das mit Carbenicillin (50 µg/ml) supplementiert war (LB-Carb.), vermehrt (36 °C, über Nacht 180 upm). Von den Einzelklonkulturen wurden Gefrierstammkulturen angelegt, je 1 ml der Einzelklonkulturen wurde zur Plasmidpräparation verwendet. Die isolierten Plasmide wurden durch EcoRI/HindIII-Verdau analysiert. Klone mit einem zu erwartenden 1321 bp-Fragment wurden durch Induktionsanalyse weiter untersucht. Hierzu wurden die ausgewählten Einzelklone in LB-Carb. angezogen und bei einer O.D.500 von 0,5 bis 1,0 durch Zugabe von einem Kulturvolumen LB-Carb., welches mit 1 mM IPTG supplementiert war, zur Expression des rekombinanten Proteins induziert und 16 Stunden bei 36 °C, 180 upm kultiviert. Die Induktionszellen wurden in SDS-Probenpuffer lysiert, Proteine wurden durch SDS-PAGE getrennt. Im Western Blot wurde durch Detektion mit einem Anti-RGS-6xHis-Peroxidase gekoppelten Antikörper (Qiagen, Hilden) die Expression des zu erwartenden 44 kDa-CAD-IgG-CH2-Modubodies nachgewiesen. Durch Sequenzierung wurde die korrekte Klonierung bestätigt.

### 4.2 Expression und Reinigung des einzelkettigen CAD-IgG -CH2-Modubodies

Das folgende Beispiel beschreibt die Expression und Reinigung des β2-Glycoprotein-spezifischen und mit einer monomeren humanen IgG-CH2-Detektionsdomäne versehenen Modubodies CAD-IgG-CH2.

Der unter 4.1 mit dem Expressionskonstrukt transformierte *E*. *coli*-Stamm CAD-IgG-CH2-pQE80-NovaBlue wurde in LB-Carb.-Medium bei 36 °C bis zu einer O.D.500 von 0,5 bis 1,0 angezogen und durch Zugabe von IPTG zur Synthese des CAD-IgG-CH2-Modubodies induziert. Die induzierte Kultur wurde bei 36 °C für 4 Stunden bis über Nacht kultiviert. Die Induktionszellen wurden geerntet und nach Lysozymbehandlung in einem 8M Harnstoff-haltigen TBS-Puffer lysiert. Exprimierte CAD-IgG-CH2-Modubodies wurden durch Ni-NTA-Affinitätschromatographie gereinigt. Im Coomassieblau gefärbten SDS-Polyacrylamidgel ist eine 44 kDa-Bande sichtbar. Die Anreicherung der β2-Glycoprotein-Bindeaktivität und einer Reaktivität mit einem Peroxidase-gekoppelten Anti-human-IgG-Sekundärantikörper (Jackson Immunoresearch) geht mit der Reinigung der 44 kDa-Bande einher.

### 4.3 Charakteristika des einzelkettigen CAD-IgG-CH2 -Modubodies

Das folgende Beispiel beschreibt die Charakterisierung des ß2-Glycoprotein-spezifischen und mit einer monomeren humanen IgG-CH2-Detektionsdomäne versehenen Modubodies CAD-IgG-CH2 in Bezug auf spezifische Antigenerkennung, spezifischer Nachweisbarkeit über einen Anti-human-IgG-Sekundärantikörper und Stabilität gegen erhöhte Temperaturen, Eintrocknen und Gefrier-Auftauzyklen.

### 4.3.1 Kalibratorfunktion des CAD-IgG-CH2 -Modubodies

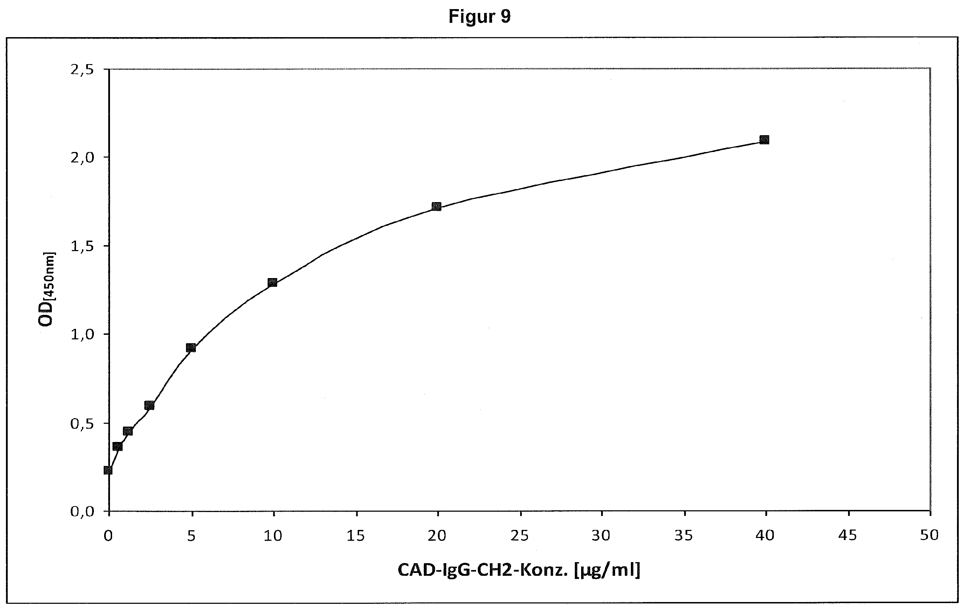

Um zu bestimmen, ob der CAD-IgG-CH2-Modubody die ß2-Glycoprotein-Bindungs-Aktivität des scFv-CAD beibehalten hat und die Bindung an das Antigen über einen Anti-human-IgG-Sekundärantikörper spezifisch nachgewiesen werden kann, wurden gereinigte CAD-IgG-CH2 Präparationen im Anti-Cardiolipin/β2-Glycoproteinimmunoassay untersucht. Der gereinigte CAD-IgG-CH2-Modubody wurde in einer Verdünnungsserie mit den Konzentrationen 0 µg/ml, 0,62 µg/µl, 1,25 µg/ml, 2,5 µg/ml, 5 µg/ml, 10 µg/ml, 20 µg/ml, 40 µg/ml in Kalibratorverdünnungsmedium auf Mikrotiterplatten, die mit ß2-Glycoprotein im Komplex mit Cardiolipin beschichtet waren, aufgetragen. Die Antigen-Bindungsaktivität wurde unter den für den Anti-Cardiolipin-Assay (ORG 515, ORGENTEC GmbH, Mainz) vorgesehenen Inkubationsbedingungen und einem Anti-human-IgG-Peroxidase markierten Sekundärantikörper (Jackson Immunoresearch) in der Konzentration von 200 ng/ml bestimmt. In **Figur 9** ist der Verlauf der unter den gewählten Reaktionsbedingungen bestimmten OD 450 nm in Abhängigkeit von der Konzentration dargestellt. Unter den gewählten Reaktionsbedingungen wurde der CAD-IgG-CH2-Modubody bei einer Konzentration von 20 µg/ml mit einer O.D. 450 nm von 1,7 nachgewiesen.

### 4.3.2 Stabilität des CAD-IgG-CH2-Modubodies gegenüber Eintrocknung

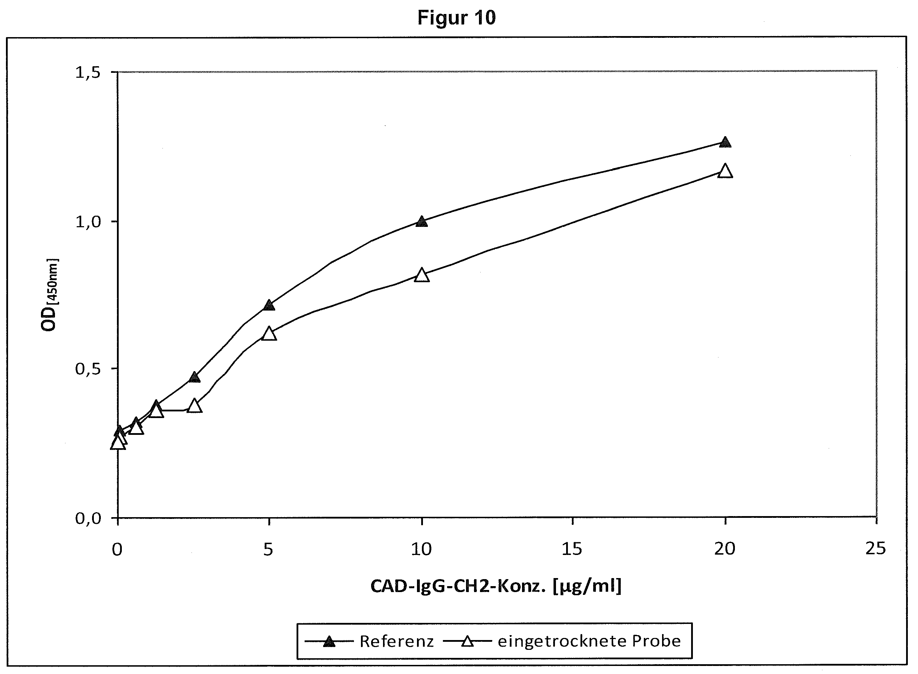

Die Stabilität gegenüber Eintrocknung wurde untersucht, indem Verdünnungen des CAD-IgG-CH2-Modubodies in Kalibratorverdünnungsmedium in 50 µl Portionen in den Konzentrationen 0 µg/ml, 3,1 µg/ml, 6,21 µg/ml, 12,5 µg/ml, 25 µg/ml, 50 µg/ml, 100 µg/ml, 200 µg/ml in in einer Speedvak-Vorrichtung unter Vakuum bei 22 °C eingetrocknet wurden. Die getrockneten Proben wurden mit 450 µl Kalibratorverdünnungsmedium und 50 µl Wasser zu einer Verdünnungsserie mit den Konzentrationen 0 µg/ml, 0,31 µg/ml, 0,62 µg/µl, 1,25 µg/ml, 2,5 µg/ml, 5 µg/ml, 10 µg/ml, 20 µg/ml resolubilisiert und im Vergleich zu einer unbehandelten Verdünnungsserie auf Mikrotiterplatten, die mit ß2-Glycoprotein im Komplex mit Cardiolipin beschichtet waren, aufgetragen. Die Antigen-Bindungsaktivität wurde unter den für den Anti-Cardiolipin-Assay (ORG 515, ORGENTEC GmbH, Mainz) vorgesehenen Inkubationsbedingungen und einem Anti-human-IgG-Peroxidase markierten Sekundärantikörper (Jackson Immunoresearch) in der Konzentration von 200 ng/ml bestimmt. In **Figur 10** **ist** der Verlauf der unter den gewählten Reaktionsbedingungen bestimmten OD 450 nm für die eingetrockneten Proben im Vergleich zur unbehandelten Verdünnungsserie dargestellt. Unter den gewählten Reaktionsbedingungen wurde die Funktion des CAD-IgG-CH2 Modubodies in Verdünnungen im Konzentrationsbereich von 3,1 µg/ml bis 200 µg/ml durch Eintrocknen nicht beeinträchtigt.

### 4.3.3 Stabilität des CAD-IgG-CH2-Modubodies gegenüber Gefrier-Auftauzyklen

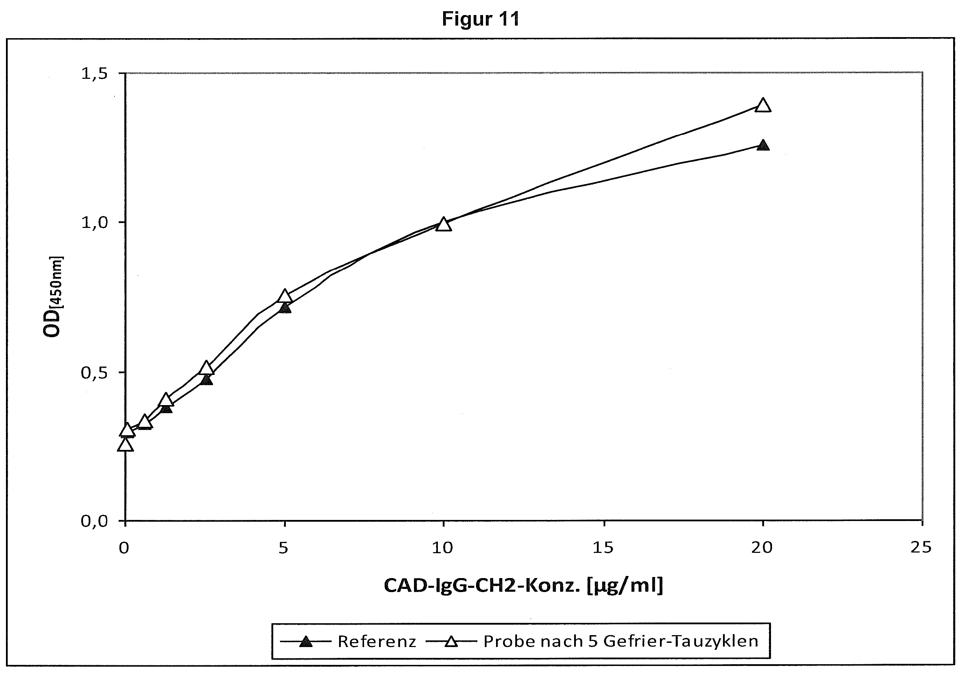

Die Stabilität gegenüber wiederholten Gefrier-Auftauzyklen wurde untersucht, indem Verdünnungen des CAD-IgG-CH2-Modubodies in Kalibratorverdünnungsmedium in 50 µl Portionen in den Konzentrationen 0 µg/ml, 3,1 µg/ml, 6,21 µg/ml, 12,5 µg/ml, 25 µg/ml, 50 µg/ml, 100 µg/ml, 200 µg/ml in fünf wiederholten Gefrier-Auftauzyklen bei -70 °C eingefroren und bei 37 °C wieder aufgetaut wurden. Anschließend wurden die Proben mit 450 µl Kalibratorverdünnungsmedium zu einer Verdünnungsserie mit den Konzentrationen 0 µg/ml, 0,31 µg/ml, 0,62 µg/µl, 1,25 µg/ml, 2,5 µg/ml, 5 µg/ml, 10 µg/ml, 20 µg/ml verdünnt und im Vergleich zu einer unbehandelten CAD-IgG-CH2-Verdünnungsserie auf Mikrotiterplatten, die mit ß2-Glycoprotein im Komplex mit Cardiolipin beschichtet waren , aufgetragen. Die Antigen-Bindungsaktivität wurde unter den für den Anti-Cardiolipin-Assay (ORG 515, ORGENTEC GmbH, Mainz) vorgesehenen Inkubationsbedingungen und einem Anti-human-IgG-Peroxidase markierten Sekundärantikörper (Jackson Immunoresearch) in der Konzentration von 200 ng/ml bestimmt. In **Figur 11** ist der Verlauf der unter den gewählten Reaktionsbedingungen bestimmten OD 450 nm für die wiederholt gefrorenen und aufgetauten Proben im Vergleich zur unbehandelten Verdünnungsserie dargestellt. Unter den gewählten Reaktionsbedingungen wurde die Funktion des CAD-IgG-CH2-Modubodies im Konzentrationsbereich von 3,1 µg/ml bis 200 µg/ml durch wiederholte Gefrier-Auftauzyklen nicht beeinträchtigt.

### Beispiel 5: Der einzelkettige multifunktionale CAD-IgM-IgA-IgG-Modubody

In dem einzelkettigen multifunktionalen Modubody CAD-IgM-IgA-IgG sind in linearer Abfolge vier Funktionsmodule enthalten. Eine β2-Glycoproteinerkennungsdomäne und mehrere Reaktionsmodule, die sich von CH3-Domänen der schweren Kette der humanen Immunglobuline IgM, IgA und IgG ableiten, sind über Peptidlinker verknüpft. Die Detektion des CAD-IgM-IgA-IgG-Modubodies kann so über unterschiedliche isotypspezifische Sekundärantikörper erfolgen.

### 5.1 Konstruktion und Klonierung des einzelkettigen CAD-IgM-IgA-IgG-Modubodies

Das folgende Beispiel beschreibt die Konstruktion und Klonierung des β2-Glycoprotein-spezifischen und mit humanen IgM-, IgG- und IgA-CH3-Detektionsdomänen versehenen Modubodies CAD-IgM-IgA-IgG.

Zur Konstruktion des CAD-IgM-IgA-IgG-Modubodies wurden die Module scFv-CAD (Beispiel 1), IgM-CH3 (Beispiel 2.7), IgA-CH3 (Beispiel 2.5) und IgG-CH3 (Beispiel 2.3) durch Restriktionsverdau und Ligation in der Domänenreihenfolge VL-Linker-VH-Linker-IgM-CH3-Linker-IgA-CH3-Linker-IgG-CH3 zu einer CAD-IgM-IgA-IgG kodierenden Sequenz (SEQ ID NO: 29) zusammengesetzt. Hierzu wurden in einem iterativen Prozess aus Restriktionen von Vektorkonstrukten und Insertligationen die Reaktionsmodule IgM-CH3, IgA-CH3 und IgG-CH3 in das unter Beispiel 1 beschriebenen CAD-scFv-pQE80-Vektorkonstrukt eingesetzt. Im ersten Konstruktionsschritt wurde das unter Beispiel 2.7 beschriebene synthetische IgM-CH3-Reaktionsmodul Sc-RP-CH3-M-N entsprechend der Sequenz SEQ ID NO: 21 mit den Primern CH09 (SEQ ID NO: 30) und CH04 (SEQ ID NO: 25) durch PCR amplifiziert. Das 455 bp-Amplifikat wurde nach Agarosegelelektrophorese mit dem QiaExII-Kit (Qiagen, Hilden) gelisoliert. Das gelisolierte Fragment wurde mit den Restriktionsenzymen BcII und HindIII verdaut. Die Restriktionsfragmente wurden mit dem unter Beispiel 1 beschriebenen scFv-CAD-pQE80-Vektorkonstrukt, welches mit den kompatiblen Enzymen BamHI und HindIII verdaut wurde, ligiert und in den *E. coli* Stamm NovaBlue (Merck, Nottingham) transformiert und auf LB-Agarplatten, die mit Carbenicillin (50 µg/ml) supplementiert waren, ausplattiert und über Nacht bei 36 °C inkubiert. Aus einem Einzelklon mit korrekter Insertligation wurde Plasmid-DNA isoliert. In einem zweiten Konstruktionsschritt wurde das unter Beispiel 2.5 beschriebene synthetische IgA-CH3-Reaktionsmodul Sc-RP-CH3-A-N entsprechend der Sequenz SEQ ID NO: 17 mit den Primern CH07 (SEQ ID NO: 31) und CH04 (SEQ ID NO: 25) durch PCR amplifiziert. Das 452 bp-Amplifikat wurde nach Agarosegelelektrophorese mit dem QiaExll-Kit (Qiagen, Hilden) gelisoliert. Das gelisolierte Fragment wurde mit den Restriktionsenzymen BcII und HindIII verdaut. Die Restriktionsfragmente wurden mit dem im ersten Konstruktionsschritt isolierten Vektorkonstrukt, welches mit den kompatiblen Enzymen BamHI und HindIII verdaut wurde, ligiert und in den *E*. *coli*-Stamm NovaBlue (Merck, Nottingham) transformiert und auf LB-Agarplatten, die mit Carbenicillin (50 µg/ml) supplementiert waren, ausplattiert und über Nacht bei 36 °C inkubiert. Aus einem Einzelklon mit korrekter Insertligation wurde Plasmid-DNA isoliert. In einem dritten Konstruktionsschritt wurde das unter Beispiel 2.3 beschriebene synthetische IgG-CH3-Reaktionsmodul Sc-RP-CH3-G-N entsprechend der Sequenz SEQ ID NO: 13 mit den Primern CH05 (SEQ ID NO: 24) und CH04 (SEQ ID NO: 25) durch PCR amplifiziert. Das 434 bp Amplifikat wurde nach Agarosegelelektrophorese mit dem QiaExll-Kit (Qiagen, Hilden) gelisoliert. Das gelisolierte Fragment wurde mit den Restriktionsenzymen BcII und HindIII verdaut. Die Restriktionsfragmente wurden mit dem im zweiten Konstruktionsschritt isolierten Vektorkonstrukt, welches mit den kompatiblen Enzymen BamHI und HindIII verdaut wurde, ligiert und in den *E. coli*-Stamm NovaBlue (Merck, Nottingham) transformiert. Der Transformationsansatz wurde auf LB-Agarplatten, die mit Carbenicillin (50 µg/ml) supplementiert waren, ausplattiert und über Nacht bei 36 °C inkubiert. Einzelkolonien des resultierenden *E*. *coli*-Stammes CAD-IgM-IgA-IgG-pQE80-NovaBlue wurden in LB-Medium, das mit Carbenicillin (50 µg/ml) supplementiert war (LB-Carb.), vermehrt (36 °C, über Nacht 180 upm). Von den Einzelklonkulturen wurden Gefrierstammkulturen angelegt, je 1 ml der Einzelklonkulturen wurde zur Plasmidpräparation verwendet. Die isolierten Plasmide wurden durch EcoRI/HindIII-Verdau analysiert. Klone mit einem zu erwartenden 2173 bp-Fragment wurden durch Induktionsanalyse weiter untersucht. Hierzu wurden die ausgewählten Einzelklone in LB-Carb. angezogen und bei einer O.D. 500 von 0,5 bis 1,0 durch Zugabe von einem Kulturvolumen LB-Carb., welches mit 1 mM IPTG supplementiert war, zur Expression des durch das klonierte DNA-Konstrukt kodierte Protein induziert und 16 Stunden bei 36 °C, 180 upm kultiviert. Die Induktionszellen wurden in SDS-Probenpuffer lysiert, Proteine wurden durch SDS-PAGE getrennt. Im Western Blot wurde durch Detektion mit einem Anti-RGS-6xHis-Peroxidase gekoppelten Antikörper (Qiagen, Hilden) die Expression des zu erwartenden 72 kDa CAD-IgM-IgA-IgG Modubodies nachgewiesen. Durch Sequenzierung wurde die korrekte Klonierung bestätigt.

### 5.2 Expression und Reinigung des einzelkettigen CAD-IgM-IgA-IgG-Modubodies

Das folgende Beispiel beschreibt die Expression und Reinigung des β2-Glycoprotein-spezifischen und mit humanen 1gM-CH3, IgA-CH3 und IgG-CH3-Detektionsdomänen versehenen Modubodies CAD-IgM-IgA-IgG.

Der unter 5.1 mit dem Expressionskonstrukt transformierte *E. coli*-Stamm CAD-IgM-IgA-IgG-pQE80-NovaBlue wurde in LB-Carb Medium bei 36 °C bis zu einer O.D.500 von 0,5 bis 1.0 angezogen und durch Zugabe von IPTG zur Synthese des CAD-IgM-IgA-IgG-Modubodies induziert. Die induzierte Kultur wurde bei 36 °C für 4 Stunden bis über Nacht kultiviert. Die Induktionszellen wurden geerntet und nach Lysozymbehandlung in einem 8M Harnstoff-haltigen TBS-Puffer lysiert. Exprimierte CAD-IgM-IgA-IgG-Modubodies wurden durch Affinitätschromatographie gereinigt. Im Coomassieblau gefärbten SDS-Polyacrylamidgel ist eine 72 kDa-Bande sichtbar. Die Anreicherung der ß2-Glycoprotein-Bindeaktivität und einer Reaktivität mit Peroxidase-gekoppelten Anti-human-IgM, -IgA- und -IgG-Sekundärantikörpern (Jackson Immunoresearch) geht mit der Reinigung der 72 kDa-Bande einher.

### 5.3 Charakteristika des einzelkettigen CAD-IgM-IgA-IgG-Modubodies

Das folgende Beispiel beschreibt die Charakterisierung des ß2-Glycoprotein-spezifischen und mit humanen IgM-CH3-, IgA-CH3- und IgG-CH3-Detektionsdomänen versehenen Modubodies CAD-IgM-IgA-IgG in Bezug auf spezifische Antigenerkennung, spezifischer Nachweisbarkeit über Anti-human-IgM-, -IgA- und -IgG-Sekundärantikörper und Stabilität gegen Eintrocknen und Gefrier-Auftauzyklen.

### 5.3.1 Kalibratorfunktion des CAD-IgM-IgA-IgG-Modubodies

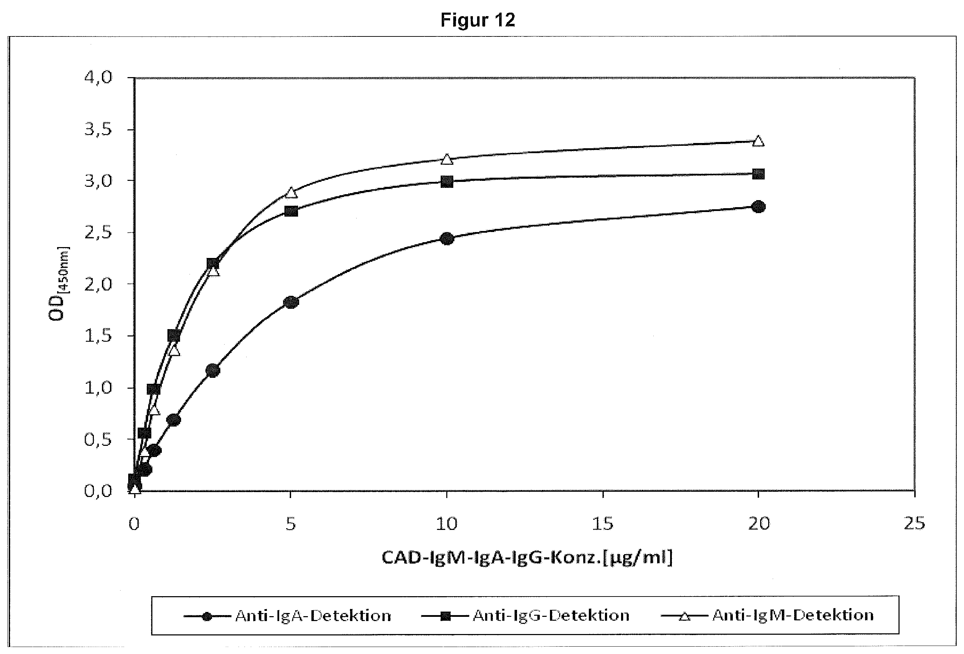

Um zu bestimmen, ob der CAD-IgM-IgA-IgG-Modubodies die ß2-Glycoprotein-Bindungs-Aktivität des scFv-CAD beibehalten hat und die Bindung an das Antigen über unterschiedliche isotypspezifische Anti-human-Sekundärantikörper nachgewiesen werden kann, wurden gereinigte CAD-IgM-IgA-IgG-Präparationen im Anti-ß2-Glycoprotein/Cardiolipin-Immunoassay untersucht. Der gereinigte CAD-IgM-IgA-IgG-Modubody wurde in einer Verdünnungsserie mit den Konzentrationen 0 µg/ml, 0,31 µg/ml, 0,62 µg/µl, 1,25 µg/ml, 2,5 µg/ml, 5 µg/ml, 10 µg/ml, 20 µg/ml in Kalibratorverdünnungsmedium auf ß2-Glycoprotein im Komplex mit Cardiolipin beschichtete Mikrotiterplatten aufgetragen. Die Antigen-Bindungsaktivität wurde unter den für den Anti-Cardiolipin-Assay (ORG 515, ORGENTEC GmbH, Mainz) vorgesehenen Inkubationsbedingungen in separaten Bestimmungen mit Anti-human-IgM-, Anti-human-IgA- und Anti-human-IgG-Peroxidase markierten Sekundärantikörpern in den Konzentrationen von 80 ng/ml bestimmt. In **Figur 12** ist der Verlauf der OD 450 nm Bestimmungen mit den gewählten Detektionsantikörpern in Abhängigkeit von der Konzentration des CAD-IgM-IgA-IgG-Modubodies dargestellt. Unter den gewählten Reaktionsbedingungen wurde der CAD-IgM-IgA-IgG-Modubody bei einer Konzentration von 5 µg/ml bei Detektion mit Anti-human-IgM-, Anti-human-IgA- und Anti-human-IgG-Sekundärantikörpern mit O.D. 450 nm Werten von 2,8, 1,8 und 2,7 nachgewiesen.

### 5.3.2 Stabilität des CAD-IgM-IgA-IgG-Modubodies gegenüber Eintrocknung

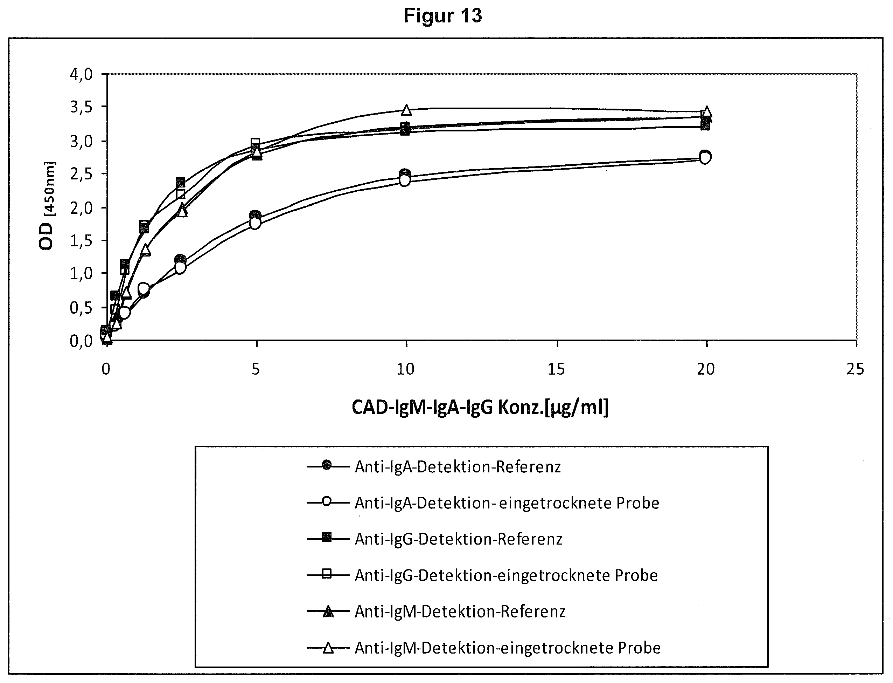

Die Stabilität gegenüber Eintrocknung wurde untersucht, indem Verdünnungen des CAD-IgM-IgA-IgG-Modubodies in Kalibratorverdünnungsmedium in 50 µl Portionen in den Konzentrationen 0 µg/ml, 3,1 µg/ml, 6,21 µg/ml, 12,5 µg/ml, 25 µg/ml, 50 µg/ml, 100 µg/ml, 200 µg/ml in in einer Speedvak-Vorrichtung unter Vakuum bei 22 °C eingetrocknet wurden. Die getrockneten Proben wurden mit 450 µl Kalibratorverdünnungsmedium und 50 µl Wasser zu einer Verdünnungsserie mit den Konzentrationen 0 µg/ml, 0,31 µg/ml, 0,62 µg/µl, 1,25 µg/ml, 2,5 µg/ml, 5 µg/ml, 10 µg/ml, 20 µg/ml resolubilisiert und im Vergleich zu einer unbehandelten Verdünnungsserie auf ß2-Glycoprotein im Komplex mit Cardiolipin beschichteten Mikrotiterplatten aufgetragen. Die Antigen-Bindungsaktivität wurde unter den für den Anti-Cardiolipin-Assay (ORG 515, ORGENTEC GmbH, Mainz) vorgesehenen Inkubationsbedingungen in separaten Bestimmungen mit Anti-human-IgM-, Anti-human-IgA- und Anti-human-IgG-Peroxidase markierten Sekundärantikörpern (Jackson Immunoresearch) in den Konzentrationen von 80 ng/ml bestimmt. In Figur 13 ist der Verlauf der OD 450 nm Bestimmungen mit den gewählten Detektionsantikörpern bei unbehandelten Probenverdünnungsstufen im Vergleich zu eingetrockneten Probenverdünnungsstufen dargestellt. Unter den gewählten Reaktionsbedingungen wurde die Funktion des CAD-IgM-IgA-IgG-Modubodies in Verdünnungen im Konzentrationsbereich von 3,1 µg/ml bis 200 µg/ml durch Eintrocknen nicht beeinträchtigt.

### 5.3.2.3 Stabilität des CAD-IgM-IgA-IgG-Modubodies gegenüber Gefrier-Auftauzyklen

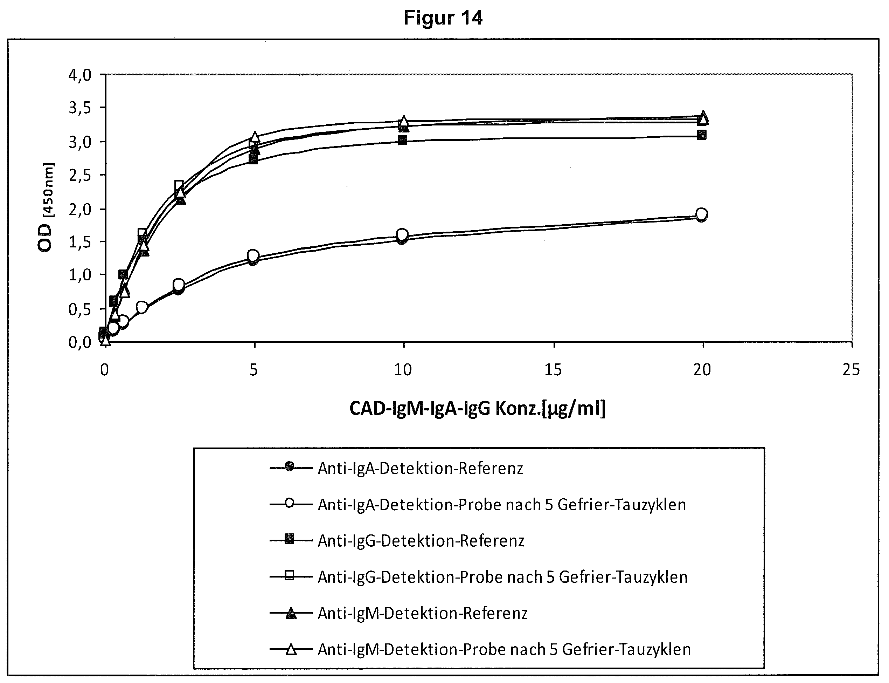

Die Stabilität gegenüber wiederholten Gefrier-Auftauzyklen wurde untersucht, indem Verdünnungen des CAD-IgM-IgA-IgG-Modubodies in Kalibratorverdünnungsmedium in 50 µl Portionen in den Konzentrationen 0 µg/ml, 3,1 µg/ml, 6,21 µg/ml, 12,5 µg/ml, 25 µg/ml, 50 µg/ml, 100 µg/ml, 200 µg/ml in fünf wiederholten Gefrier-Auftauzyklen bei -70 °C eingefroren und bei 37 °C wieder aufgetaut wurden. Anschließend wurden die Proben mit 450 µl Kalibratorverdünnungsmedium zu einer Verdünnungsserie mit den Konzentrationen 0 µg/ml, 0,31 µg/ml, 0,62 µg/µl, 1,25 µg/ml, 2,5 µg/ml, 5 µg/ml, 10 µg/ml, 20 µg/ml verdünnt und im Vergleich zu einer unbehandelten CAD-IgM-IgA-IgG-Verdünnungsserie auf ß2-Glycoprotein im Komplex mit Cardiolipin beschichteten Mikrotiterplatten aufgetragen. Die Antigen-Bindungsaktivität wurde unter den für den Anti-Cardiolipin-Assay (ORG 515, ORGENTEC GmbH, Mainz) vorgesehenen Inkubationsbedingungen in separaten Bestimmungen mit Anti-human-IgM-, Anti-human-IgA- und Anti-human-IgG-Peroxidase markierten Sekundärantikörpern (Jackson Immunoresearch) in den Konzentrationen von 80 ng/ml bestimmt. In **Figur 14** ist der Verlauf der OD 450 nm Bestimmungen mit den gewählten Detektionsantikörpern bei unbehandelten Probenverdünnungsstufen im Vergleich zu den wiederholt gefrorenen und aufgetauten Proben dargestellt. Unter den gewählten Reaktionsbedingungen wurde die Funktion des CAD-IgM-IgA-IgG-Modubodies in Verdünnungen im Konzentrationsbereich von 3,1 µg/ml bis 200 µg/ml durch wiederholte Gefrier-Auftauzyklen nicht beeinträchtigt.

### Beispiel 6: Der einzelkettige CAD-IgG-CH3-Knob02-Modubody

### 6.1 Konstruktion und Klonierung des einzelkettigen CAD-IgG-CH3-Knob02-Modubodies

Das folgende Beispiel beschreibt die Konstruktion und Klonierung des ß2-Glycoprotein-spezifischen und mit einer monomeren modifizierten humanen IgG-CH3-Knob02-Detektionsdomäne versehenen Modubodies CAD-IgG-CH3-Knob02.

Zur Konstruktion des CAD-IgG-CH3-Knob02-Modubodies wurden die Module scFv-CAD (Beispiel 1) und IgG-CH3-Knob02 (Beispiel 2.9) durch Restriktionsverdau und Ligation in der Domänenreihenfolge VL-Linker-VH-Linker-IgG-CH3-Knob02 zu einer CAD-IgG-CH3-Knob02 kodierenden Sequenz (SEQ ID NO: 34) zusammengesetzt. Hierzu wurde das unter Beispiel 2.9 beschriebene synthetische IgG-CH3-Knob02-Reaktionsmodul Sc-RP-CH3-Knob02-G-N entsprechend der Sequenz SEQ ID NO: 33 durch Verdau mit den Restriktionsenzymen BamHI und Hindll aus dem Vektorkonstrukt IgG-CH3-Knob02-pMA freigesetzt.

Das 417 bp Restriktionsfragment wurde nach Agarosegelelektrophorese mit dem QiaExII-Kit (Qiagen, Hilden) gelisoliert und mit dem unter Beispiel 1 beschriebenen CAD-scFv-pQE80-Vektorkonstrukt, welches mit den kompatiblen Enzymen BamHI und HindIII verdaut wurde, ligiert. Die Ligationsprodukte wurden in den *E. coli* Stamm NovaBlue (Merck, Nottingham) transformiert. Der Transformationsansatz wurde auf LB-Agarplatten, die mit Carbenicillin (50 µg/ml) supplementiert waren, ausplattiert und über Nacht bei 36 °C inkubiert. Einzelkolonien des resultierenden *E*. *coli*-Stammes CAD-IgG-CH3-Knob02-pQE80-NovaBlue wurden in LB-Medium, das mit Carbenicillin (50 µg/ml) supplementiert war (LB-Carb.), vermehrt (36 °C, über Nacht, 180 upm). Von den Einzelklonkulturen wurden Gefrierstammkulturen angelegt, je 1 ml der Einzelklonkulturen wurde zur Plasmidpräparation verwendet. Die isolierten Plasmide wurden durch EcoRI/HindIII-Verdau analysiert. Klone mit einem zu erwartenden 1308 bp-Fragment wurden durch Induktionsanalyse weiter untersucht. Hierzu wurden die ausgewählten Einzelklone in LB-Carb. angezogen und bei einer O.D.500 von 0,5 bis 1,0 durch Zugabe von einem Kulturvolumen LB-Carb., welches mit 1 mM IPTG supplementiert war, zur Expression des durch das klonierte DNA-Fragment kodierte Protein induziert und 16 Stunden bei 36 °C, 180 upm kultiviert. Die Induktionszellen wurden in SDS-Probenpuffer lysiert, Proteine wurden durch SDS-PAGE getrennt. Im Western Blot wurde durch Detektion mit einem Anti-RGS-6xHis-Peroxidase gekoppelten Antikörper (Qiagen, Hilden) die Expression des zu erwartenden 44 kDa-CAD-IgG-CH3-Knob02-Modubodies nachgewiesen. Durch Sequenzierung wurde die korrekte Klonierung bestätigt.

### 6.2 Expression und Reinigung des einzelkettigen CAD-IgG-CH3-Knob02-Modubodies

Das folgende Beispiel beschreibt die Expression und Reinigung des ß2-Glycoprotein-spezifischen und mit einer modifizierten humanen IgG-CH3-Knob02 Detektionsdomäne versehenen Modubodies CAD-IgG-CH3-Knob02.

Der unter 6.1 mit dem Expressionskonstrukt transformierte *E*. *coli*-Stamm CAD-IgG-CH3-Knob02-pQE80-NovaBlue wurde in LB-Carb.-Medium bei 36 °C bis zu einer O.D.500 von 0,5 bis 1.0 angezogen und durch Zugabe von IPTG zur Synthese des CAD-IgG-CH3-Knob02-Modubodies induziert. Die induzierte Kultur wurde bei 36 °C für 4 Stunden bis über Nacht kultiviert. Die Induktionszellen wurden geerntet und nach Lysozymbehandlung in einem 8M Harnstoff-haltigen TBS-Puffer lysiert. Exprimierte CAD-IgG-CH3-Knob02-Modubodies wurden durch Ni-NTA-Affinitätschromatographie gereinigt. Im Coomassieblau gefärbten SDS-Polyacrylamidgel ist eine 44 kDa-Bande sichtbar. Die Anreicherung der ß2-Glycoprotein-Bindeaktivität und einer Reaktivität mit einem Peroxidase-gekoppelten Anti-human-IgG-Sekundärantikörper (Jackson Immunoresearch) geht mit der Reinigung der 44 kDa-Bande einher.

### 6.3 Bindung des CAD-IgG-CH3-Knob02-Modubodies an β2-Glycoprotein

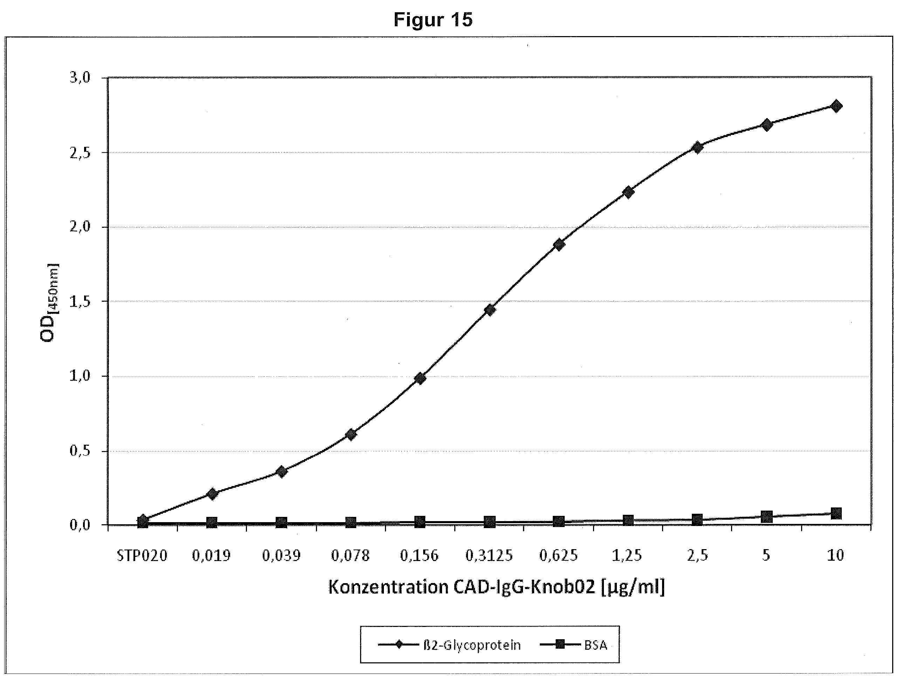

Um zu bestimmen, ob der CAD-IgG-CH3-Knob02-Modubody die ß2-Glycoprotein-Bindungs-Aktivität des scFv-CAD beibehalten hat, wurden gereinigte CAD-IgG-CH3-Knob02-Präparationen mit Immunoassays getestet. Hierzu wurden Verdünnungsserien des CAD-IgG-CH3-Knob02-Modubodies auf Mikrotiterplatten des mit ß2-Glycoprotein beschichteten Anti-ß2-Glycoprotein-Assays (ORG 521, ORGENTEC Diagnostika GmbH, Mainz) und zur Kontrolle der Bindungsspezifität auf BSA-beschichteten Platten aufgetragen und 30 min bei 20-25 °C inkubiert. Die Mikrotiterplatten wurden gewaschen und die Bindung des CAD-IgG-CH3-Knob02-Modubodies wurde mit einem Peroxidase-markierten RGS-6X-His-Antikörper (Qiagen, Hilden) und einer Tetramethylbenzidin (TMB)-Farbreaktion durch Messung der O.D. 450 nm bestimmt. In **Figur 15** ist der Verlauf der unter den gewählten Reaktionsbedingungen bestimmten OD 450 nm in Abhängigkeit von der Konzentration dargestellt. Bereits bei einer Konzentration des CAD-IgG-CH3-Knob02-Modubodies von 19 ng/ml wurde eine spezifische Bindung an ß2-Glycoprotein nachgewiesen. Der Vergleich der Reaktionsniveaus der CAD-IgG-CH3-Knob02-Verdünnungsstufen bei der Bindung an ß2-Glycoprotein- bzw. BSA-beschichtete Mikrotiterplatten zeigt, dass im Konzentrationsbereich von 0,019-10 µg/ml CAD-IgG-CH3-Knob02 keine unspezifische Bindung auftritt.

## Patentansprüche

1. Monovalentes Fusionspolypeptid, das als Monomer vorliegt, umfassend
(i) eine erste Domäne, umfassend die variable Schwerkettenregion eines Antikörpers (VH) oder zumindest einen die Antigenbindung vermittelnden Abschnitt davon,
(ii) eine zweite Domäne, umfassend die variable Leichtkettenregion eines Antikörpers (VL) oder zumindest einen die Antigenbindung vermittelnden Abschnitt davon und
(iii) eine dritte Domäne, umfassend einen Abschnitt einer konstanten Schwerkettenregion eines Antikörpers (CHX),
wobei die Domäne (iii) eine Länge von 80-130 Aminosäureresten aufweist und Aminosäurepositionen,
deren Seitenketten entsprechend dem Konzept der "knobs-into-holes"-Anordnung Interaktionen aufweisen,
modifiziert sind,
wobei die Domänen (i), (ii) und (iii) über Peptidlinker (L) miteinander verknüpft sind,
wobei die Peptidlinker (L) jeweils unabhängig eine Länge von 25-45 Aminosäureresten aufweisen und vollständig aus Glycin- und/oder Serinresten bestehen, und wobei das Fusionspolypeptid keine intermolekularen Disulfidbrücken ausbildet und frei von Hinge-Regionen aus Antikörpern ist.

2. Fusionspolypeptid nach Anspruch 1 umfassend die Struktur
VH - L - VL - L - CHX oder
VL - L - VH - L - CHX.

3. Fusionspolypeptid nach einem der Ansprüche 1 bis 2, wobei die Domänen (i), (ii) und (iii) jeweils eine Länge von 80-130 Aminosäureresten aufweisen.

4. Fusionspolypeptid nach einem der Ansprüche 1 bis 3, wobei die Domäne (iii) ausgewählt ist aus den Abschnitten CH1, CH2, CH3 und CH4 von Antikörpern, bevorzugt von humanen Antikörpern der Klassen IgG, IgM, IgE und IgA oder Kombinationen dieser Abschnitte.

5. Fusionspolypeptid nach einem der Ansprüche 1 bis 4, wobei die Peptidlinker jeweils unabhängig eine Länge von 30-40 Aminosäureresten aufweisen.

6. Fusionspolypeptid nach einem der Ansprüche 1 bis 5, welches mindestens eine zusätzliche Domäne, z.B. ein Signalpeptid und/oder ein Peptid-Tag aufweist.

7. Fusionspolypeptid nach einem der Ansprüche 1 bis 6, welches eine oder mehrere Domänen VH, VL und/oder CHX, mit einer Identität auf Aminosäureebene von mindestens 90 %, vorzugsweise von mindestens 95 % zu den entsprechenden Domänen gemäß SEQ ID NO: 1 (VL), SEQ ID NO: 2 (VH), SEQ ID NO: 4 (VH, VL), SEQ ID NO: 8 (IgG-CH1), SEQ ID NO: 10 (IgG-CH2), SEQ ID NO: 12 (IgG-CH3), SEQ ID NO: 14 (IgA-CH2), SEQ ID NO: 16 (IgA-CH3), SEQ ID NO: 18 (IgM-CH2), SEQ ID NO: 20 (IgM-CH3) oder SEQ ID NO: 22 (IgM-CH4) aufweist.

8. Fusionspolypeptid nach einem der Ansprüche 1 bis 7, wobei mindestens ein Asparaginrest aus einer Glykosilierungsposition durch einen anderen Aminosäurerest, vorzugsweise Serin, Alanin oder Glycin, ersetzt ist, und/oder wobei mindestens ein Cysteinrest durch einen anderen Aminosäurerest, vorzugsweise Serin, Alanin oder Glycin, ersetzt ist.

9. Nukleinsäure, kodierend für ein Fusionspolypeptid nach einem der Ansprüche 1 bis 8, gegebenenfalls in operativer Verknüpfung mit einer Expressionskontrollsequenz.

10. Nukleinsäure nach Anspruch 9, die bezüglich der Expression in einer ausgewählten Wirtszelle, z.B. einem Bakterium wie etwa *E. coli,* codonoptimiert ist.

11. Wirtszelle, die eine Nukleinsäure nach einem der Ansprüche 9 bis 10 enthält.

12. Verfahren zur Herstellung eines Fusionspolypeptids nach einem der Ansprüche 1 bis 8, umfassend die Kultivierung einer Wirtszelle nach Anspruch 11 und die Gewinnung des Fusionspolypeptids aus der Zelle oder dem Kulturüberstand.

13. Verwendung eines Fusionspolypeptids nach einem der Ansprüche 1 bis 8 als Reagenz in einem diagnostischen oder biochemischen Test, bevorzugt
(i) als Kontroll- oder Kalibratorreagenz oder
(ii) als Testreagenz zur Bestimmung eines Analyten.

14. Fusionspolypeptid nach einem der Ansprüche 1 bis 8, Nukleinsäure nach Anspruch 9 oder 10 oder Wirtszelle nach Anspruch 11 zur Verwendung in der Medizin, z.B. in der Human- oder Veterinärmedizin.

15. Pharmazeutische Zusammensetzung, die ein Fusionspolypeptid nach einem der Ansprüche 1 bis 8, eine Nukleinsäure nach Anspruch 9 oder 10 oder eine Wirtszelle nach Anspruch 11 zusammen mit pharmazeutisch geeigneten Trägersubstanzen enthält.

## Claims

1. Monovalent fusion polypeptide, which is present in the form of a monomer, comprising
(i) a first domain comprising the heavy chain variable region of an antibody (VH) or at least a portion thereof that mediates antigen binding,
(ii) a second domain comprising the light chain variable region of an antibody (VL) or at least a portion thereof that mediates antigen binding, and
(iii) a third domain comprising a portion of a heavy chain constant region of an antibody (CHX),
wherein domain (iii) has a length of from 80-130 amino acid residues, and amino acid positions are modified, the side chains of which interact in a manner corresponding to the concept of the "knobs-into-holes" arrangement,
wherein domains (i), (ii) and (iii) are linked together via peptide linkers (L),
wherein the peptide linkers (L) each independently have a length of from 25-45 amino acid residues and consist completely of glycine- and/or serine residues, and wherein the fusion polypeptide does not form intermolecular disulphide bridges and is free of hinge regions of antibodies.

2. Fusion polypeptide according to claim 1, comprising the structure
VH - L - VL - L - CHX or
VL - L - VH - L - CHX.

3. Fusion polypeptide according to either of claims 1 to 2, wherein domains (i), (ii) and (iii) each have a length of from 80-130 amino acid residues.

4. Fusion polypeptide according to any of claims 1 to 3, wherein domain (iii) is selected from portions CH1, CH2, CH3 and CH4 of antibodies, preferably of human antibodies of classes IgG, IgM, IgE and IgA, or combinations of these portions.

5. Fusion polypeptide according to any of claims 1 to 4, wherein the peptide linkers each independently have a length of from 30-40 amino acid residues.

6. Fusion polypeptide according to any of claims 1 to 5, which comprises at least one additional domain, for example a signal peptide and/or a peptide tag.

7. Fusion polypeptide according to any of claims 1 to 6, which comprises one or more VH, VL and/or CHX domains which have at least 90 % identity, preferably at least 95 % identity, at amino acid level with the corresponding domains according to SEQ ID NO: 1 (VL), SEQ ID NO: 2 (VH), SEQ ID NO: 4 (VH, VL), SEQ ID NO: 8 (IgG-CH1), SEQ ID NO: 10 (IgG-CH2), SEQ ID NO: 12 (IgG-CH3), SEQ ID NO: 14 (IgA-CH2), SEQ ID NO: 16 (IgA-CH3), SEQ ID NO: 18 (IgM-CH2), SEQ ID NO: 20 (IgM-CH3) or SEQ ID NO: 22 (IgM-CH4).

8. Fusion polypeptide according to any of claims 1 to 7, wherein at least one asparagine residue from a glycosylation position is replaced by a different amino acid residue, preferably serine, alanine or glycine, and/or wherein at least one cysteine residue is replaced by a different amino acid residue, preferably serine, alanine or glycine.

9. Nucleic acid, coding for a fusion polypeptide according to any of claims 1 to 8, optionally in operative linkage with an expression control sequence.

10. Nucleic acid according to claim 9, which is codon-optimised in respect of expression in a selected host cell, for example a bacterium such as *E. coli.*

11. Host cell containing a nucleic acid according to either of claims 9 to 10.

12. Method for the preparation of a fusion polypeptide according to any of claims 1 to 8, comprising cultivating a host cell according to claim 11 and obtaining the fusion polypeptide from the cell or from the culture supernatant.

13. Use of a fusion polypeptide according to any of claims 1 to 8 as a reagent in a diagnostic or biochemical test, preferably:
(i) as a control or calibrator reagent or
(ii) as a test reagent for determining an analyte.

14. Fusion polypeptide according to any of claims 1 to 8, nucleic acid according to either claim 9 or claim 10, or host cell according to claim 11, for use in medicine, for example in human or veterinary medicine.

15. Pharmaceutical composition comprising a fusion polypeptide according to any of claims 1 to 8, a nucleic acid according to either claim 9 or claim 10, or a host cell according to claim 11, together with pharmaceutically suitable carrier substances.

## Revendications

1. Polypeptide de fusion monovalent, qui est présent en tant que monomère, comprenant
(i) un premier domaine, comprenant la région de chaîne lourde variable d'un anticorps (VH) ou au moins un segment de celle-ci permettant la liaison aux antigènes,
(ii) un deuxième domaine, comprenant la région de chaîne légère variable d'un anticorps (VL) ou au moins un segment de celle-ci permettant la liaison aux antigènes, et
(iii) un troisième domaine, comprenant un segment d'une région de chaîne lourde constante d'un anticorps (CHX),
dans lequel le domaine (iii) présente une longueur de 80-130 résidus d'acides aminés, et des positions d'acides aminés,
dont les chaînes latérales présentent des interactions correspondant au concept de l'agencement « knobs-into-holes »,
sont modifiées,
dans lequel les domaines (i), (ii) et (iii) sont reliés entre eux par le biais de lieurs peptidiques (L),
dans lequel les lieurs peptidiques (L) présentent respectivement de façon indépendante une longueur de 25-45 résidus d'acides aminés et se composent entièrement de résidus glycine et/ou sérine, et dans lequel le polypeptide de fusion ne constitue aucun pont disulfure intermoléculaire et est exempt de régions charnières d'anticorps.

2. Polypeptide de fusion selon la revendication 1, comprenant la structure
VH - L - VL - L - CHX ou
VL - L - VH - L - CHX.

3. Polypeptide de fusion selon l'une des revendications 1 à 2, dans lequel les domaines (i), (ii) et (iii) présentent respectivement une longueur de 80-130 résidus d'acides aminés.

4. Polypeptide de fusion selon l'une des revendications 1 à 3, dans lequel le domaine (iii) est sélectionné parmi les segments CH1, CH2, CH3 et CH4 d'anticorps, de préférence d'anticorps humains des classes IgG, IgM, IgE et IgA ou de combinaisons de ces segments.

5. Polypeptide de fusion selon l'une des revendications 1 à 4, dans lequel les lieurs peptidiques présentent respectivement de façon indépendante une longueur de 30-40 résidus d'acides aminés.

6. Polypeptide de fusion selon l'une des revendications 1 à 5, qui présente au moins un domaine supplémentaire, par exemple un peptide signal et/ou un marqueur peptidique.

7. Polypeptide de fusion selon l'une des revendications 1 à 6, qui contient un ou plusieurs domaines VH, VL et/ou CHX qui présente(nt) une identité sur le plan des acides aminés d'au moins 90 %, de préférence d'au moins 95 % par rapport aux domaines correspondants selon SEQ ID NO : 1 (VL), SEQ ID NO : 2 (VH), SEQ ID NO : 4 (VH, VL), SEQ ID NO : 8 (IgG-CH1), SEQ ID NO : 10 (IgG-CH2), SEQ ID NO : 12 (IgG-CH3), SEQ ID NO : 14 (IgA-CH2), SEQ ID NO : 16 (IgA-CH3), SEQ ID NO : 18 (IgM-CH2), SEQ ID NO : 20 (IgM-CH3) ou SEQ ID NO : 22 (IgM-CH4).

8. Polypeptide de fusion selon l'une des revendications 1 à 7, dans lequel au moins un résidu asparagine à partir d'une position de glycosylation est remplacé par un autre résidu d'acides aminés, de préférence sérine, alanine ou glycine, et/ou dans lequel au moins un résidu cystéine est remplacé par un autre résidu d'acides aminés, de préférence sérine, alanine ou glycine.

9. Acide nucléique, codant pour un polypeptide de fusion selon l'une des revendications 1 à 8, éventuellement en liaison opérationnelle avec une séquence de contrôle de l'expression.

10. Acide nucléique selon la revendication 9, qui, en ce qui concerne l'expression, est optimisé au plan du codon dans une cellule hôte sélectionnée, par ex. dans une bactérie telle qu'E. *coli.*

11. Cellule hôte, qui contient un acide nucléique selon l'une des revendications 9 à 10.

12. Procédé de production d'un polypeptide de fusion selon l'une des revendications 1 à 8, comprenant la culture d'une cellule hôte selon la revendication 11 et l'extraction du polypeptide de fusion à partir de la cellule ou du surnageant de culture.

13. Utilisation d'un polypeptide de fusion selon l'une des revendications 1 à 8 en tant que réactif dans un test de diagnostic ou biochimique, de préférence
(i) en tant que réactif de contrôle ou d'agent d'étalonnage ou
(ii) en tant que réactif de test pour la détermination d'un analyte.

14. Polypeptide de fusion selon l'une des revendications 1 à 8, acide nucléique selon la revendication 9 ou 10 ou cellule hôte selon la revendication 11 en vue d'une utilisation en médecine, par ex. en médecine humaine ou vétérinaire.

15. Composition pharmaceutique, qui contient un polypeptide de fusion selon l'une des revendications 1 à 8, un acide nucléique selon la revendication 9 ou 10 ou une cellule hôte selon la revendication 11 conjointement avec des excipients appropriés au plan pharmaceutique.


### SEQUENCE LISTING

<110> ORGENTEC Diagnostika GmbH
<120> Monospezifische Polypeptidreagenzien
<130> 46816P WO
<160> 34
<170> PatentIn version 3.3
<210> 1
   <211> 112
   <212> PRT
   <213> Artificial
<220>
   <223> WBCAL-1 VL
<400> 1
   Asp Val Leu Met Thr Gln Thr Pro Leu Ser Leu Pro Val Ser Leu Gly
   1               5                   10                  15
   Asp Gln Ala Ser Ile Ser Cys Arg Ser Ser Gln Ser Ile Val His Ser
               20                  25                  30
   Asn Gly Asn Thr Tyr Leu Glu Trp Tyr Leu Gln Lys Pro Gly Gln Ser
           35                  40                  45
   Pro Lys Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Leu Gly Val Tyr Tyr Cys Phe Gln Gly
                   85                  90                  95
   Ser His Val Pro Tyr Thr Phe Gly Gly Gly Thr Lys Leu Glu Ile Lys
               100                 105                 110
<210> 2
   <211> 114
   <212> PRT
   <213> Artificial
<220>
   <223> WBCAL-1 VH
<400> 2
   Glu Val Gln Leu Gln Gln Ser Gly Pro Glu Leu Val Lys Pro Gly Ala
   1               5                   10                  15
   Ser Val Lys Ile Ser Cys Lys Ala Ser Gly Tyr Thr Phe Thr Asp Tyr
                   20                  25                  30
   Tyr Met His Trp Val Arg Gln Arg Pro Gly Gln Gly Leu Glu Trp Ile
           35                  40                  45
   Gly Glu Ile Tyr Pro Gly Ser Gly Asn Thr Ser Tyr Asn Glu Lys Phe
       50                  55                  60
   Arg Gly Lys Ala Thr Leu Thr Ala Asp Lys Ser Ser Ser Thr Ala Tyr
   65                  70                  75                  80
   Met Gln Leu Ser Ser Leu Thr Ser Glu Asp Ser Ala Val Tyr Phe Cys
                   85                  90                  95
   Ala Arg Gly Thr Leu Asp Tyr Thr Met Asp Tyr Trp Gly Gln Gly Thr
               100                 105                 110
   Ser Val
<210> 3
   <211> 36
   <212> PRT
   <213> Artificial
<220>
   <223> peptide linker
<400> 3
   Gly Ser Gly Gly Gly Gly Ser Gly Gly Gly Gly Ser Gly Gly Gly Gly
   1               5                   10                  15
   Ser Ser Gly Gly Gly Gly Ser Gly Gly Gly Gly Ser Gly Gly Gly Gly
               20                  25                  30
   Ser Gly Ser Gly
           35
<210> 4
   <211> 276
   <212> PRT
   <213> Artificial
<220>
   <223> scFv-RP-CAD-P
<220>
   <221> DOMAIN
   <222> (1)..(118)
   <223> VL domain
<220>
   <221> DOMAIN
   <222> (159)..(272)
   <223> VH domain
<400> 4
   Leu Val Pro Arg Gly Ser Asp Val Leu Met Thr Gln Thr Pro Leu Ser
   1               5                   10                  15
   Leu Pro Val Ser Leu Gly Asp Gln Ala Ser Ile Ser Cys Arg Ser Ser
               20                  25                  30
   Gln Ser Ile Val His Ser Asn Gly Asn Thr Tyr Leu Glu Trp Tyr Leu
           35                  40                  45
   Gln Lys Pro Gly Gln Ser Pro Lys Leu Leu Ile Tyr Lys Val Ser Asn
       50                  55                  60
   Arg Phe Ser Gly Val Pro Asp Arg Phe Ser Gly Ser Gly Ser Gly Thr
   65                  70                  75                  80
   Asp Phe Thr Leu Lys Ile Ser Arg Val Glu Ala Glu Asp Leu Gly Val
                   85                  90                  95
   Tyr Tyr Cys Phe Gln Gly Ser His Val Pro Tyr Thr Phe Gly Gly Gly
               100                 105                 110
   Thr Lys Leu Glu Ile Lys Arg Thr Gly Ser Gly Ser Gly Gly Gly Gly
           115                 120                 125
   Ser Gly Gly Gly Gly Ser Gly Gly Gly Gly Ser Ser Gly Gly Gly Gly
       130                 135                 140
   Ser Gly Gly Gly Gly Ser Gly Gly Gly Gly Ser Gly Ser Gly Glu Val
   145                 150                 155                 160
   Gln Leu Gln Gln Ser Gly Pro Glu Leu Val Lys Pro Gly Ala Ser Val
                   165                 170                 175
   Lys Ile Ser Cys Lys Ala Ser Gly Tyr Thr Phe Thr Asp Tyr Tyr Met
               180                 185                 190
   His Trp Val Arg Gln Arg Pro Gly Gln Gly Leu Glu Trp Ile Gly Glu
           195                 200                 205
   Ile Tyr Pro Gly Ser Gly Asn Thr Ser Tyr Asn Glu Lys Phe Arg Gly
       210                 215                 220
   Lys Ala Thr Leu Thr Ala Asp Lys Ser Ser Ser Thr Ala Tyr Met Gln
   225                 230                 235                 240
   Leu Ser Ser Leu Thr Ser Glu Asp Ser Ala Val Tyr Phe Cys Ala Arg
                   245                 250                 255
   Gly Thr Leu Asp Tyr Thr Met Asp Tyr Trp Gly Gln Gly Thr Ser Val
               260                 265                 270
   Thr Val Ser Ser
           275
<210> 5
   <211> 862
   <212> DNA
   <213> Artificial
<220>
   <223> scFv-RP-CAD-N
<400> 5
   ggtaccggat ccctggttcc gcgtggttct gatgttctga tgacccagac accgctgtct     60
   ctgccggtta gcctgggtga tcaggcaagc attagctgtc gtagcagcca gagcattgtt    120
   catagcaatg gcaataccta tctggaatgg tatctgcaga aaccgggtca gagcccgaaa    180
   ctgctgattt ataaagtgag caatcgcttt agcggtgttc cggatcgttt tagcggttca    240
   ggttctggca ccgattttac cctgaaaatt agccgtgttg aagcagaaga tctgggcgtt    300
   tattattgtt ttcagggtag ccatgttccg tatacctttg gtggtggcac caaactggaa    360
   attaaacgta ccggtagcgg tagtggaggt ggtggaagcg gtggtggcgg ttctggcggt    420
   ggaggttctt ctggtggcgg tggatcaggt ggaggtggct caggcggtgg cggtagcggc    480
   agcggtgaag ttcagctgca gcagagcggt ccggaactgg ttaaaccggg tgcaagcgtt    540
   aaaattagct gtaaagccag cggctatacc tttaccgatt attatatgca ttgggttcgt    600
   cagcgtccgg gtcagggtct ggaatggatt ggtgaaattt atccgggtag cggtaatacc    660
   agctataatg aaaaatttcg tggcaaagca accctgaccg cagataaaag cagcagcacc    720
   gcatatatgc agctgtctag cctgaccagc gaagatagcg cagtttactt ttgtgcacgt    780
   ggcaccctgg attataccat ggattattgg ggacagggca ccagcgttac cgttagcagc    840
   ggatcctaat aagcttgagc tc                                              862
<210> 6
   <211> 27
   <212> DNA
   <213> Artificial
<220>
   <223> primer RP-CAD01
<400> 6
   agtagatctc tggttccgcg tggttct 27
<210> 7
   <211> 18
   <212> DNA
   <213> Artificial
<220>
   <223> primer RP-CAD02
<400> 7
   ctcaagctta ttaggatc 18
<210> 8
   <211> 131
   <212> PRT
   <213> Artificial
<220>
   <223> Sc-RP-CH1-G-P
<220>
   <221> DOMAIN
   <222> (37)..(131)
   <223> IgG-CH1 domain
<400> 8
   Gly Ser Gly Gly Gly Gly Ser Gly Gly Gly Gly Ser Gly Gly Gly Gly
   1               5                   10                  15
   Ser Ser Gly Gly Gly Gly Ser Gly Gly Gly Gly Ser Gly Gly Gly Gly
               20                  25                  30
   Ser Gly Ser Gly Gly Pro Ser Val Phe Pro Leu Ala Pro Ser Ser Lys
           35                  40                  45
   Ser Thr Ser Gly Gly Thr Ala Ala Leu Gly Cys Leu Val Lys Asp Tyr
       50                  55                  60
   Phe Pro Glu Pro Val Thr Val Ser Trp Asn Ser Gly Ala Leu Thr Ser
   65                  70                  75                  80
   Gly Val His Thr Phe Pro Ala Val Leu Gln Ser Ser Gly Leu Tyr Ser
                   85                  90                  95
   Leu Ser Ser Val Val Thr Val Pro Ser Ser Ser Leu Gly Thr Gln Thr
               100                 105                 110
   Tyr Ile Cys Asn Val Asn His Lys Pro Ser Asn Thr Lys Val Asp Lys
           115                 120                 125
   Lys Val Glu
       130
<210> 9
   <211> 427
   <212> DNA
   <213> Artificial
<220>
   <223> Sc-RP-CH1-G-N
<400> 9
   gagctcggat ccggcagtgg tggaggtggc tcaggcggag gtggaagcgg tggaggcggt      60
   tcttcaggtg gcggtggaag tggcggtgga ggtagtggtg gcggaggctc tggatcaggt     120
   ggtccgagcg tttttccgct ggcaccgagc agcaaaagca ccagcggtgg cacagcagca     180
   ctgggctgtc tggtgaaaga ttattttccg gaaccggtta ccgttagctg gaatagcggt     240
   gcactgacca gcggtgttca tacctttccg gcagttctgc agagcagcgg tctgtatagc     300
   ctgagcagcg ttgttaccgt tccgtctagc agcctgggca cccagaccta tatttgcaat     360
   gtgaatcata aaccgagcaa tacgaaagtg gataaaaaag tggaaggatc ctgataagct     420
   tggtacc                                                               427
<210> 10
   <211> 138
   <212> PRT
   <213> Artificial
<220>
   <223> Sc-RP-CH2-G-P
<220>
   <221> DOMAIN
   <222> (37)..(138)
   <223> IgG-CH2 domain
<400> 10
   Gly Ser Gly Gly Gly Gly Ser Gly Gly Gly Gly Ser Gly Gly Gly Gly
   1               5                   10                  15
   Ser Ser Gly Gly Gly Gly Ser Gly Gly Gly Gly Ser Gly Gly Gly Gly
               20                  25                  30
   Ser Gly Ser Gly Gly Pro Asp Val Phe Leu Phe Pro Pro Lys Pro Lys
           35                  40                  45
   Asp Thr Leu Met Ile Ser Arg Thr Pro Glu Val Thr Cys Val Val Val
       50                  55                  60
   Asp Val Ser His Glu Asp Pro Glu Val Lys Phe Asn Trp Tyr Val Asp
   65                  70                  75                  80
   Gly Val Glu Val His Asn Ala Lys Thr Lys Pro Arg Glu Glu Gln Tyr
                   85                  90                  95
   Asn Ser Thr Tyr Arg Val Val Ser Val Leu Thr Val Leu His Gln Asp
               100                 105                 110
   Trp Leu Asn Gly Lys Glu Tyr Lys Cys Lys Val Ser Asn Lys Ala Leu
           115                 120                 125
   Pro Leu Pro Glu Glu Lys Thr Ile Ser Lys
       130                 135
<210> 11
   <211> 448
   <212> DNA
   <213> Artificial
<220>
   <223> Sc-RP-CH2-G-N
<400> 11
   gagctcggat ccggttctgg tgggggaggg tctggcggag gtggctctgg cggaggcggt      60
   tcaagcggtg gcggaggtag tggcggtggt ggtagtggag gcggaggctc tggttcaggt     120
   ggtccggatg tttttctgtt tccgcctaaa ccgaaagata ccctgatgat tagccgtaca     180
   ccggaagtta cctgtgttgt tgttgatgtg agccatgaag atccggaagt gaaatttaat     240
   tggtatgtgg atggtgtgga agttcataat gccaaaacca aaccgcgtga agaacagtat     300
   aatagcacct atcgtgttgt ttctgttctg accgttctgc atcaggattg gctgaatggc     360
   aaagaatata aatgcaaagt gtctaataaa gcactgccgc tgccggaaga aaaaaccatt     420
   agcaaaggat cctaataagc ttggtacc                                        448
<210> 12
   <211> 135
   <212> PRT
   <213> Artificial
<220>
   <223> Sc-RP-CH3-G-P
<220>
   <221> DOMAIN
   <222> (37)..(135)
   <223> IgG-CH3 domain
<400> 12
   Gly Ser Gly Gly Gly Gly Ser Gly Gly Gly Gly Ser Gly Gly Gly Gly
   1               5                   10                  15
   Ser Ser Gly Gly Gly Gly Ser Gly Gly Gly Gly Ser Gly Gly Gly Gly
               20                  25                  30
   Ser Gly Ser Gly Glu Pro Gln Val Tyr Thr Leu Pro Pro Ser Arg Glu
           35                  40                  45
   Glu Met Thr Lys Asn Gln Val Ser Leu Thr Cys Leu Val Lys Gly Phe
       50                  55                  60
   Tyr Pro Ser Asp Ile Ala Val Glu Trp Glu Ser Asn Gly Gln Pro Glu
   65                  70                  75                  80
   Asn Asn Tyr Lys Thr Thr Pro Pro Val Leu Asp Ser Asp Gly Ser Phe
                   85                  90                  95
   Phe Leu Tyr Ser Lys Leu Thr Val Asp Lys Ser Arg Trp Gln Gln Gly
               100                 105                 110
   Asn Val Phe Ser Cys Ser Val Met His Glu Ala Leu His Asn His Tyr
           115                 120                 125
   Thr Gln Lys Ser Leu Ser Leu
       130                 135
<210> 13
   <211> 439
   <212> DNA
   <213> Artificial
<220>
   <223> Sc-RP-CH3-G-N
<400> 13
   gagctcggat ccggtagcgg tggtggtggt tctggcggtg gtggcagcgg aggtggcggt      60
   agctcaggtg gcggaggtag tggcggtgga ggcagtggtg gcggtggctc tggatctggt     120
   gaaccgcagg tttataccct gcctccgagc cgtgaagaaa tgaccaaaaa tcaggttagc     180
   ctgacctgtc tggtgaaagg tttttatccg agcgatattg cagttgaatg ggaaagcaat     240
   ggtcagccgg aaaataatta taaaaccaca cctccggttc tggattctga tggtagcttt     300
   tttctgtata gcaaactgac cgttgataaa agccgttggc agcagggtaa tgtttttagc     360
   tgtagcgtta tgcatgaagc cctgcataat cattataccc agaaaagcct gagcctggga     420
   tcctaataag cttggtacc                                                  439
<210> 14
   <211> 133
   <212> PRT
   <213> Artificial
<220>
   <223> Sc-RP-CH2-A-P
<220>
   <221> DOMAIN
   <222> (37)..(133)
   <223> IgA-CH2 domain
<400> 14
   Gly Ser Gly Gly Gly Gly Ser Gly Gly Gly Gly Ser Gly Gly Gly Gly
   1 5 10 15
   Ser Ser Gly Gly Gly Gly Ser Gly Gly Gly Gly Ser Gly Gly Gly Gly
   20 25 30
   Ser Gly Ser Gly Arg Leu Ser Leu His Arg Pro Ala Leu Glu Asp Leu
   35 40 45
   Leu Leu Gly Ser Glu Ala Asn Leu Thr Cys Thr Leu Thr Gly Leu Arg
   50 55 60
   Asp Ala Ser Gly Val Thr Phe Thr Trp Thr Pro Ser Ser Gly Lys Ser
   65 70 75 80
   Ala Val Gln Gly Pro Pro Glu Arg Asp Leu Cys Gly Ser Tyr Ser Val
   85 90 95
   Ser Ser Val Leu Pro Gly Ser Ala Glu Pro Trp Asn His Gly Lys Thr
   100 105 110
   Phe Thr Cys Thr Ala Ala Tyr Pro Glu Ser Lys Thr Pro Leu Thr Ala
   115 120 125
   Thr Leu Ser Lys Ser
   130
<210> 15
   <211> 433
   <212> DNA
   <213> Artificial
<220>
   <223> Sc-RP-CH2-A-N
<400> 15
   gagctcggat ccggaagtgg cggaggcgga tcaggtggag gtggcagcgg aggtggcggt      60
   agctctggtg gcggtggtag tggcggtgga ggtagtggtg gcggaggctc tggttctggt     120
   cgtctgagcc tgcatcgtcc ggcactggaa gatctgctgc tgggtagcga agcaaatctg     180
   acctgtaccc tgaccggtct gcgtgatgca agcggtgtta catttacctg gaccccgagc     240
   agcggtaaaa gcgcagttca gggtccgcct gaacgtgatc tgtgtggtag ctatagcgtt     300
   agcagcgttc tgcctggtag cgcagaaccg tggaatcatg gtaaaacctt tacctgtacc     360
   gcagcatatc cggaaagcaa aacaccgctg accgcaaccc tgagcaaaag cggatcctaa     420
   taagcttggt acc                                                        433
<210> 16
   <211> 141
   <212> PRT
   <213> Artificial
<220>
   <223> sc-RP-CH3-A-P
<220>
   <221> DOMAIN
   <222> (37)..(141)
   <223> IgA-CH3 domain
<400> 16
   Gly Ser Gly Gly Gly Gly Ser Gly Gly Gly Gly Ser Gly Gly Gly Gly
   1               5                   10                  15
   Ser Ser Gly Gly Gly Gly Ser Gly Gly Gly Gly Ser Gly Gly Gly Gly
               20                  25                  30
   Ser Gly Ser Gly Arg Pro Glu Val His Leu Leu Pro Pro Pro Ser Glu
           35                  40                  45
   Glu Leu Ala Leu Asn Glu Leu Val Thr Leu Thr Cys Leu Ala Arg Gly
       50                  55                  60
   Phe Ser Pro Lys Asp Val Leu Val Arg Trp Leu Gln Gly Ser Gln Glu
   65                  70                  75                  80
   Leu Pro Arg Glu Lys Tyr Leu Thr Trp Ala Ser Arg Gln Glu Pro Ser
                   85                  90                  95
   Gln Gly Thr Thr Thr Phe Ala Val Thr Ser Ile Leu Arg Val Ala Ala
               100                 105                 110
   Glu Asp Trp Lys Lys Gly Asp Thr Phe Ser Cys Met Val Gly His Glu
           115                 120                 125
   Ala Leu Pro Leu Ala Phe Thr Gln Lys Thr Ile Asp Arg
       130                 135                 140
<210> 17
   <211> 457
   <212> DNA
   <213> Artificial
<220>
   <223> sc-RP-CH3-A-N
<400> 17
   gagctcggat ccggtagcgg aggtggcgga agcggaggtg gaggcagtgg tggaggcggt     60
   agctcaggtg gcggtggaag tggtggaggt ggctcagggg gtggaggttc tggttctggt    120
   cgtccggaag ttcatctgct gcctccgcct agcgaagaac tggcactgaa tgaactggtt    180
   accctgacct gtctggcacg tggttttagc ccgaaagatg ttctggttcg ttggctgcag    240
   ggtagccagg aactgcctcg cgaaaaatat ctgacctggg catctcgcca ggaaccgagc    300
   cagggcacca ccacctttgc agttaccagc attctgcgtg ttgcagcaga agattggaaa    360
   aaaggcgata cctttagctg tatggttggt catgaagcac tgccgctggc atttacccag    420
   aaaaccatcg atcgcggatc ctaataagct tggtacc                             457
<210> 18
   <211> 148
   <212> PRT
   <213> Artificial
<220>
   <223> Sc-RP-CH2-M-P
<220>
   <221> DOMAIN
   <222> (37)..(148)
   <223> IgM-CH2 domain
<400> 18
   Gly Ser Gly Gly Gly Gly Ser Gly Gly Gly Gly Ser Gly Gly Gly Gly
   1               5                   10                  15
   Ser Ser Gly Gly Gly Gly Ser Gly Gly Gly Gly Ser Gly Gly Gly Gly
               20                  25                  30
   Ser Gly Ser Gly Ile Ala Glu Leu Pro Pro Lys Val Ser Val Phe Val
           35                  40                  45
   Pro Pro Arg Asp Gly Phe Phe Gly Asn Pro Arg Lys Ser Lys Leu Ile
       50                  55                  60
   Cys Gln Ala Thr Gly Phe Ser Pro Arg Gln Ile Gln Val Ser Trp Leu
   65                  70                  75                  80
   Arg Glu Gly Lys Gln Val Gly Ser Gly Val Thr Thr Asp Gln Val Gln
                   85                  90                  95
   Ala Glu Ala Lys Glu Ser Gly Pro Thr Thr Tyr Lys Val Thr Ser Thr
               100                 105                 110
   Leu Thr Ile Lys Glu Ser Asp Trp Leu Gly Gln Ser Met Phe Thr Cys
           115                 120                 125
   Arg Val Asp His Arg Gly Leu Thr Phe Gln Gln Ser Ala Ser Ser Met
       130                 135                 140
   Ser Val Pro Asp
   145
<210> 19
   <211> 478
   <212> DNA
   <213> Artificial
<220>
   <223> Sc-RP-CH2-M-N
<400> 19
   gagctcggat ccggttcagg tggtggtggt tctggtggtg gaggcagtgg aggtggcggt     60
   tctagcggtg gcggtggaag tggcggaggt ggcagtggtg gcggtggctc tggttctggt    120
   attgcagaac tgcctccgaa agttagcgtt tttgttcctc cgcgtgatgg tttttttggt    180
   aatccgcgta aaagcaaact gatttgtcag gcaaccggtt tttctccgcg tcagattcag    240
   gttagctggc tgcgtgaagg taaacaggtt ggtagcggtg ttaccaccga tcaggttcag    300
   gcagaagcaa aagaaagcgg tccgaccacc tataaagtta ccagcaccct gaccattaaa    360
   gaaagcgatt ggctgggtca gagcatgttt acctgtcgtg ttgatcatcg tggtctgacc    420
   tttcagcaga gcgcaagcag catgagcgtt ccggatggat cctaataagc ttggtacc      478
<210> 20
   <211> 142
   <212> PRT
   <213> Artificial
<220>
   <223> Sc-RP-CH3-M-P
<220>
   <221> DOMAIN
   <222> (37)..(142)
   <223> IgM-CH3 domain
<400> 20
   Gly Ser Gly Gly Gly Gly Ser Gly Gly Gly Gly Ser Gly Gly Gly Gly
   1               5                   10                  15
   Ser Ser Gly Gly Gly Gly Ser Gly Gly Gly Gly Ser Gly Gly Gly Gly
               20                  25                  30
   Ser Gly Ser Gly Gln Asp Thr Ala Ile Arg Val Phe Ala Ile Pro Pro
           35                  40                  45
   Ser Phe Ala Ser Ile Phe Leu Thr Lys Ser Thr Lys Leu Thr Cys Leu
       50                  55                  60
   Val Thr Asp Leu Thr Thr Tyr Asp Ser Val Thr Ile Ser Trp Thr Arg
   65                  70                  75                  80
   Gln Asn Gly Glu Ala Val Lys Thr His Thr Ser Ile Ser Glu Ser His
                   85                  90                  95
   Pro Ser Ala Thr Phe Ser Ala Val Gly Glu Ala Ser Ile Ser Glu Asp
               100                 105                 110
   Asp Trp Asn Ser Gly Glu Arg Phe Thr Cys Thr Val Thr His Thr Asp
           115                 120                 125
   Leu Pro Ser Pro Leu Lys Gln Thr Ile Ser Arg Pro Lys Gly
       130                 135                 140
<210> 21
   <211> 460
   <212> DNA
   <213> Artificial
<220>
   <223> Sc-RP-CH3-M-N
<400> 21
   gagctcggat ccggaagtgg tggaggtggc agcggtggtg gtggctctgg tggaggcggt      60
   agctcaggtg gcggtggtag tggcggtgga ggtagtggtg gcggaggttc tggatctggt     120
   caggataccg caattcgtgt ttttgcaatt cctccgagct ttgcaagcat ttttctgacc     180
   aaaagcacca aactgacctg tctggttacc gatctgacca cctatgatag cgttaccatt     240
   agctggaccc gtcagaatgg tgaagcagtt aaaacccata ccagcattag cgaaagccat     300
   ccgagcgcaa cctttagcgc agttggtgaa gcaagcattt ctgaagatga ttggaatagc     360
   ggtgaacgtt ttacctgtac cgttacccat accgatctgc cgtcaccgct gaaacagacc     420
   attagccgtc cgaaaggtgg atcctaataa gcttggtacc                          460
<210> 22
   <211> 165
   <212> PRT
   <213> Artificial
<220>
   <223> Sc-RP-CH4-M-P
<220>
   <221> DOMAIN
   <222> (37)..(165)
   <223> IgM-CH4 domain
<400> 22
   Gly Ser Gly Gly Gly Gly Ser Gly Gly Gly Gly Ser Gly Gly Gly Gly
   1               5                   10                  15
   Ser Ser Gly Gly Gly Gly Ser Gly Gly Gly Gly Ser Gly Gly Gly Gly
               20                  25                  30
   Ser Gly Ser Gly Val Ala Leu His Arg Pro Asp Val Tyr Leu Leu Pro
           35                  40                  45
   Pro Ala Arg Glu Gln Leu Asn Leu Arg Glu Ser Ala Thr Ile Thr Cys
       50                  55                  60
   Leu Val Thr Gly Phe Ser Pro Ala Asp Val Phe Val Gln Trp Met Gln
   65                  70                  75                  80
   Arg Gly Gln Pro Leu Ser Pro Glu Lys Tyr Val Thr Ser Ala Pro Met
                   85                  90                  95
   Pro Glu Pro Gln Ala Pro Gly Arg Tyr Phe Ala His Ser Ile Leu Thr
               100                 105                 110
   Val Ser Glu Glu Glu Trp Asn Thr Gly Glu Thr Tyr Thr Cys Val Ala
           115                 120                 125
   His Glu Ala Leu Pro Asn Arg Val Thr Glu Arg Thr Val Asp Lys Ser
       130                 135                 140
   Thr Gly Lys Pro Thr Leu Tyr Ser Val Ser Leu Val Met Ser Asp Thr
   145                 150                 155                 160
   Ala Gly Thr Ser Tyr
                   165
<210> 23
   <211> 529
   <212> DNA
   <213> Artificial
<220>
   <223> Sc-RP-CH4-M-N
<400> 23
   gagctcggat ccggatctgg tggaggcgga tctggcggtg gaggcagcgg aggtggcgga     60
   agctcaggtg gcggtggaag tggcggtggt ggtagtggtg gcggtggctc tggttctggt    120
   gttgcactgc atcgtccgga tgtttatctg ctgcctccgg cacgtgaaca gctgaatctg    180
   cgtgaaagcg caaccattac ctgtctggtt accggttttt ctccggcaga tgtttttgtt    240
   cagtggatgc agcgtggtca gccgctgtct ccggaaaaat atgttaccag cgcaccgatg    300
   ccggaaccgc aggcaccggg tcgttatttt gcacatagca ttctgaccgt tagcgaagaa    360
   gaatggaata caggcgaaac ctatacctgt gttgcacatg aagcactgcc gaatcgtgtt    420
   accgaacgta ccgttgataa aagcaccggt aaaccgaccc tgtatagcgt tagcctggtt    480
   atgagcgata cagcaggcac aagctatgga tcctaataag cttggtacc                529
<210> 24
   <211> 29
   <212> DNA
   <213> Artificial
<220>
   <223> primer CH05
<400> 24
   agttgatcag gtagcggtgg tggtggttc 29
<210> 25
   <211> 19
   <212> DNA
   <213> Artificial
<220>
   <223> primer CH04
<400> 25
   taccaagctt attaggatc 19
<210> 26
   <211> 1284
   <212> DNA
   <213> Artificial
<220>
   <223> CAD-IgG-CH3
<400> 26
   atgagaggat cgcatcacca tcaccatcac ggatctctgg ttccgcgtgg ttctgatgtt     60
   ctgatgaccc agacaccgct gtctctgccg gttagcctgg gtgatcaggc aagcattagc    120
   tgtcgtagca gccagagcat tgttcatagc aatggcaata cctatctgga atggtatctg    180
   cagaaaccgg gtcagagccc gaaactgctg atttataaag tgagcaatcg ctttagcggt    240
   gttccggatc gttttagcgg ttcaggttct ggcaccgatt ttaccctgaa aattagccgt    300
   gttgaagcag aagatctggg cgtttattat tgttttcagg gtagccatgt tccgtatacc    360
   tttggtggtg gcaccaaact ggaaattaaa cgtaccggta gcggtagtgg aggtggtgga    420
   agcggtggtg gcggttctgg cggtggaggt tcttctggtg gcggtggatc aggtggaggt    480
   ggctcaggcg gtggcggtag cggcagcggt gaagttcagc tgcagcagag cggtccggaa    540
   ctggttaaac cgggtgcaag cgttaaaatt agctgtaaag ccagcggcta tacctttacc    600
   gattattata tgcattgggt tcgtcagcgt ccgggtcagg gtctggaatg gattggtgaa    660
   atttatccgg gtagcggtaa taccagctat aatgaaaaat ttcgtggcaa agcaaccctg    720
   accgcagata aaagcagcag caccgcatat atgcagctgt ctagcctgac cagcgaagat    780
   agcgcagttt acttttgtgc acgtggcacc ctggattata ccatggatta ttggggacag    840
   ggcaccagcg ttaccgttag cagcggatca ggtagcggtg gtggtggttc tggcggtggt    900
   ggcagcggag gtggcggtag ctcaggtggc ggaggtagtg gcggtggagg cagtggtggc    960
   ggtggctctg gatctggtga accgcaggtt tataccctgc ctccgagccg tgaagaaatg   1020
   accaaaaatc aggttagcct gacctgtctg gtgaaaggtt tttatccgag cgatattgca   1080
   gttgaatggg aaagcaatgg tcagccggaa aataattata aaaccacacc tccggttctg   1140
   gattctgatg gtagcttttt tctgtatagc aaactgaccg ttgataaaag ccgttggcag   1200
   cagggtaatg tttttagctg tagcgttatg catgaagccc tgcataatca ttatacccag   1260
   aaaagcctga gcctgggatc ctaa                                          1284
<210> 27
   <211> 1293
   <212> DNA
   <213> Artificial
<220>
   <223> CAD-IgG-CH2
<400> 27
   atgagaggat cgcatcacca tcaccatcac ggatctctgg ttccgcgtgg ttctgatgtt      60
   ctgatgaccc agacaccgct gtctctgccg gttagcctgg gtgatcaggc aagcattagc     120
   tgtcgtagca gccagagcat tgttcatagc aatggcaata cctatctgga atggtatctg     180
   cagaaaccgg gtcagagccc gaaactgctg atttataaag tgagcaatcg ctttagcggt     240
   gttccggatc gttttagcgg ttcaggttct ggcaccgatt ttaccctgaa aattagccgt     300
   gttgaagcag aagatctggg cgtttattat tgttttcagg gtagccatgt tccgtatacc     360
   tttggtggtg gcaccaaact ggaaattaaa cgtaccggta gcggtagtgg aggtggtgga     420
   agcggtggtg gcggttctgg cggtggaggt tcttctggtg gcggtggatc aggtggaggt     480
   ggctcaggcg gtggcggtag cggcagcggt gaagttcagc tgcagcagag cggtccggaa     540
   ctggttaaac cgggtgcaag cgttaaaatt agctgtaaag ccagcggcta tacctttacc     600
   gattattata tgcattgggt tcgtcagcgt ccgggtcagg gtctggaatg gattggtgaa     660
   atttatccgg gtagcggtaa taccagctat aatgaaaaat ttcgtggcaa agcaaccctg     720
   accgcagata aaagcagcag caccgcatat atgcagctgt ctagcctgac cagcgaagat     780
   agcgcagttt acttttgtgc acgtggcacc ctggattata ccatggatta ttggggacag     840
   ggcaccagcg ttaccgttag cagcggatca ggttctggtg ggggagggtc tggcggaggt     900
   ggctctggcg gaggcggttc aagcggtggc ggaggtagtg gcggtggtgg tagtggaggc     960
   ggaggctctg gttcaggtgg tccggatgtt tttctgtttc cgcctaaacc gaaagatacc    1020
   ctgatgatta gccgtacacc ggaagttacc tgtgttgttg ttgatgtgag ccatgaagat    1080
   ccggaagtga aatttaattg gtatgtggat ggtgtggaag ttcataatgc caaaaccaaa    1140
   ccgcgtgaag aacagtataa tagcacctat cgtgttgttt ctgttctgac cgttctgcat    1200
   caggattggc tgaatggcaa agaatataaa tgcaaagtgt ctaataaagc actgccgctg    1260
   ccggaagaaa aaaccattag caaaggatcc taa                                 1293
<210> 28
   <211> 30
   <212> DNA
   <213> Artificial
<220>
   <223> primer CH03
<400> 28
   agttgatcag gttctggtgg gggagggtct 30
<210> 29
   <211> 2145
   <212> DNA
   <213> Artificial
<220>
   <223> CAD-IgM-IgA-IgG
<400> 29
   atgagaggat cgcatcacca tcaccatcac ggatctctgg ttccgcgtgg ttctgatgtt     60
   ctgatgaccc agacaccgct gtctctgccg gttagcctgg gtgatcaggc aagcattagc    120
   tgtcgtagca gccagagcat tgttcatagc aatggcaata cctatctgga atggtatctg    180
   cagaaaccgg gtcagagccc gaaactgctg atttataaag tgagcaatcg ctttagcggt    240
   gttccggatc gttttagcgg ttcaggttct ggcaccgatt ttaccctgaa aattagccgt    300
   gttgaagcag aagatctggg cgtttattat tgttttcagg gtagccatgt tccgtatacc    360
   tttggtggtg gcaccaaact ggaaattaaa cgtaccggta gcggtagtgg aggtggtgga    420
   agcggtggtg gcggttctgg cggtggaggt tcttctggtg gcggtggatc aggtggaggt    480
   ggctcaggcg gtggcggtag cggcagcggt gaagttcagc tgcagcagag cggtccggaa    540
   ctggttaaac cgggtgcaag cgttaaaatt agctgtaaag ccagcggcta tacctttacc    600
   gattattata tgcattgggt tcgtcagcgt ccgggtcagg gtctggaatg gattggtgaa    660
   atttatccgg gtagcggtaa taccagctat aatgaaaaat ttcgtggcaa agcaaccctg    720
   accgcagata aaagcagcag caccgcatat atgcagctgt ctagcctgac cagcgaagat    780
   agcgcagttt acttttgtgc acgtggcacc ctggattata ccatggatta ttggggacag    840
   ggcaccagcg ttaccgttag cagcggatca ggaagtggtg gaggtggcag cggtggtggt    900
   ggctctggtg gaggcggtag ctcaggtggc ggtggtagtg gcggtggagg tagtggtggc    960
   ggaggttctg gatctggtca ggataccgca attcgtgttt ttgcaattcc tccgagcttt   1020
   gcaagcattt ttctgaccaa aagcaccaaa ctgacctgtc tggttaccga tctgaccacc   1080
   tatgatagcg ttaccattag ctggacccgt cagaatggtg aagcagttaa aacccatacc   1140
   agcattagcg aaagccatcc gagcgcaacc tttagcgcag ttggtgaagc aagcatttct   1200
   gaagatgatt ggaatagcgg tgaacgtttt acctgtaccg ttacccatac cgatctgccg   1260
   tcaccgctga aacagaccat tagccgtccg aaaggtggat caggtagcgg aggtggcgga   1320
   agcggaggtg gaggcagtgg tggaggcggt agctcaggtg gcggtggaag tggtggaggt 1380
   ggctcagggg gtggaggttc tggttctggt cgtccggaag ttcatctgct gcctccgcct 1440
   agcgaagaac tggcactgaa tgaactggtt accctgacct gtctggcacg tggttttagc 1500
   ccgaaagatg ttctggttcg ttggctgcag ggtagccagg aactgcctcg cgaaaaatat 1560
   ctgacctggg catctcgcca ggaaccgagc cagggcacca ccacctttgc agttaccagc 1620
   attctgcgtg ttgcagcaga agattggaaa aaaggcgata cctttagctg tatggttggt 1680
   catgaagcac tgccgctggc atttacccag aaaaccatcg atcgcggatc aggtagcggt 1740
   ggtggtggtt ctggcggtgg tggcagcgga ggtggcggta gctcaggtgg cggaggtagt 1800
   ggcggtggag gcagtggtgg cggtggctct ggatctggtg aaccgcaggt ttataccctg 1860
   cctccgagcc gtgaagaaat gaccaaaaat caggttagcc tgacctgtct ggtgaaaggt 1920
   ttttatccga gcgatattgc agttgaatgg gaaagcaatg gtcagccgga aaataattat 1980
   aaaaccacac ctccggttct ggattctgat ggtagctttt ttctgtatag caaactgacc 2040
   gttgataaaa gccgttggca gcagggtaat gtttttagct gtagcgttat gcatgaagcc 2100
   ctgcataatc attataccca gaaaagcctg agcctgggat cctaa 2145
<210> 30
   <211> 28
   <212> DNA
   <213> Artificial
<220>
   <223> primer CH09
<400> 30
   agttgatcag gaagtggtgg aggtggca 28
<210> 31
   <211> 28
   <212> DNA
   <213> Artificial
<220>
   <223> primer CH07
<400> 31
   agttgatcag gtagcggagg tggcggaa 28
<210> 32
   <211> 135
   <212> PRT
   <213> Artificial
<220>
   <223> Sc-RP-CH3-Knob02-P
<220>
   <221> DOMAIN
   <222> (37)..(135)
   <223> IgG-CH3-Knob02 domain
<400> 32
   Gly Ser Gly Gly Gly Gly Ser Gly Gly Gly Gly Ser Gly Gly Gly Gly
   1               5                   10                  15
   Ser Ser Gly Gly Gly Gly Ser Gly Gly Gly Gly Ser Gly Gly Gly Gly
               20                  25                  30
   Ser Gly Ser Gly Glu Pro Gln Val Tyr Thr Leu Pro Pro Ser Arg Glu
           35                  40                  45
   Glu Met Thr Lys Asn Gln Val Ser Leu Tyr Cys Leu Val Lys Gly Phe
       50                  55                  60
   Tyr Pro Ser Asp Ile Ala Val Glu Trp Glu Ser Asn Gly Gln Pro Glu
   65                  70                  75                  80
   Asn Asn Tyr Lys Thr Tyr Pro Pro Val Leu Asp Ser Asp Gly Ser Phe
                   85                  90                  95
   Tyr Leu Tyr Ser Lys Leu Thr Val Asp Lys Ser Arg Trp Gln Gln Gly
               100                 105                 110
   Asn Val Phe Ser Cys Ser Val Met His Glu Ala Leu His Asn His Tyr
           115                 120                 125
   Thr Gln Lys Ser Leu Ser Leu
       130                 135
<210> 33
   <211> 423
   <212> DNA
   <213> Artificial
<220>
   <223> Sc-RP-CH3-Knob02-N
<400> 33
   ggatccggta gcggtggtgg tggtagtggt ggcggtggtt caggtggtgg tggcagcagt      60
   ggtggtggcg gttctggcgg tggcggttca ggtggcggag gtagcggtag cggtgaaccg     120
   caggtttata ccctgcctcc gagccgtgaa gaaatgacca aaaatcaggt tagcctgtat     180
   tgcctggtga aaggttttta tccgagcgat attgcagttg aatgggaaag caatggtcag     240
   ccggaaaata actataaaac ctatccgcct gtgctggata gtgatggtag cttttatctg     300
   tatagcaaac tgaccgtgga taaaagccgt tggcagcagg gtaatgtttt tagctgtagc     360
   gttatgcatg aagccctgca taatcactac acccagaaaa gcctgagcct gtaataaaag     420
   ctt                                                                   423
<210> 34
   <211> 1281
   <212> DNA
   <213> Artificial
<220>
   <223> CAD-IgG-CH3-Knob02
<400> 34
   atgagaggat cgcatcacca tcaccatcac ggatctctgg ttccgcgtgg ttctgatgtt     60
   ctgatgaccc agacaccgct gtctctgccg gttagcctgg gtgatcaggc aagcattagc    120
   tgtcgtagca gccagagcat tgttcatagc aatggcaata cctatctgga atggtatctg    180
   cagaaaccgg gtcagagccc gaaactgctg atttataaag tgagcaatcg ctttagcggt    240
   gttccggatc gttttagcgg ttcaggttct ggcaccgatt ttaccctgaa aattagccgt    300
   gttgaagcag aagatctggg cgtttattat tgttttcagg gtagccatgt tccgtatacc    360
   tttggtggtg gcaccaaact ggaaattaaa cgtaccggta gcggtagtgg aggtggtgga    420
   agcggtggtg gcggttctgg cggtggaggt tcttctggtg gcggtggatc aggtggaggt    480
   ggctcaggcg gtggcggtag cggcagcggt gaagttcagc tgcagcagag cggtccggaa    540
   ctggttaaac cgggtgcaag cgttaaaatt agctgtaaag ccagcggcta tacctttacc    600
   gattattata tgcattgggt tcgtcagcgt ccgggtcagg gtctggaatg gattggtgaa    660
   atttatccgg gtagcggtaa taccagctat aatgaaaaat ttcgtggcaa agcaaccctg    720
   accgcagata aaagcagcag caccgcatat atgcagctgt ctagcctgac cagcgaagat    780
   agcgcagttt acttttgtgc acgtggcacc ctggattata ccatggatta ttggggacag    840
   ggcaccagcg ttaccgttag cagcggatcc ggtagcggtg gtggtggtag tggtggcggt    900
   ggttcaggtg gtggtggcag cagtggtggt ggcggttctg gcggtggcgg ttcaggtggc    960
   ggaggtagcg gtagcggtga accgcaggtt tataccctgc ctccgagccg tgaagaaatg   1020
   accaaaaatc aggttagcct gtattgcctg gtgaaaggtt tttatccgag cgatattgca   1080
   gttgaatggg aaagcaatgg tcagccggaa aataactata aaacctatcc gcctgtgctg   1140
   gatagtgatg gtagctttta tctgtatagc aaactgaccg tggataaaag ccgttggcag   1200
   cagggtaatg tttttagctg tagcgttatg catgaagccc tgcataatca ctacacccag   1260
   aaaagcctga gcctgtaata a                                             1281